# EUROPEAN PATENT APPLICATION

(11) **EP 3 747 409 A1**
(43) Date of publication of application: **09.12.2020**
(21) Application number: 19020359.6
(22) Date of filing: 03.06.2019
(51) Int. Cl.: A61F 7/00, A61K 9/00, A61K 9/127, A61K 41/00, A61K 49/18, A61N 5/06, A61P 31/00, A61P 35/00

(54) **NANOPARTICLE FOR USE IN A METHOD OF TREATING A BODY PART OF AN INDIVIDUAL BY NON-PREDOMINANT ICE-BALL CRYOTHERAPY**

(71) Applicant: Nanobacterie, 75116 Paris (FR)
(72) Inventor: ALPHANDERY, Edouard, 75116 Paris (FR)

(57) **Abstract**

The invention relates to nanoparticles for use in a treatment of a body part of an individual by cryotherapy by administering said nanoparticles to the body part, cooling down the body part, in such a way that ice-balls are not formed

## Description

### FIELD OF THE INVENTION

The field of the invention is that of nanoparticles for use in a treatment of a body part of an individual by cryotherapy by administering said nanoparticles to the body part, cooling down the body part, in such a way that ice-balls are not formed.

### STATE OF THE ART

The use of cryotherapy has been proposed for a number of medical applications (Yiu et al, Int. J. Angiol., 16, 1, 2008). It usually works by cooling the body part of an organism at temperatures that are below 0 °C (degree Celsius), producing so called ice-balls that can induce the destruction of the body part. Due to the low temperatures reached during conventional cryotherapy, side effects can occur. Here, we propose a new type of cryotherapy in which nanoparticles are introduced to the body part and cooled down at a temperature that is above the ice-balls formation temperature in order to treat the body part without forming iceballs and thus drastically limiting side effects.

### DESCRIPTION OF THE INVENTION

In one aspect of the invention, the invention relates to nanoparticle for use in a method of treating a body part of an individual by cryotherapy comprising the following steps:
a) administering nanoparticles to a body part of an individual;
b) cooling the body part comprising the nanoparticles by decreasing the temperature of said body part from an initial temperature to a cooling temperature of said body part, which is lower than the initial temperature;
c) optionally not maintaining the body part comprising the nanoparticles at the cooling temperature for a duration of time of more than 100 seconds; and
d) warming the body part comprising the nanoparticles by increasing the temperature of the body part from the cooling temperature of said body part to a final temperature of said body part that is above the cooling temperature,
characterized in that said cooling temperature of the body part is:
- above the freezing temperature of the body part and/or above 5, 0 or -5°C;
   and/or
- above a threshold temperature, where the threshold temperature has at least one property selected from the group consisting of:
   i) above the threshold temperature, the body part comprises a quantity of ice-balls smaller than the quantity of nanoparticles;
   ii) above the threshold temperature, the volume occupied by ice-balls in the body part is smaller than the volume occupied by the nanoparticles in the body part;
   iii) below the threshold temperature, the body part comprises a quantity of ice-balls larger than the quantity of nanoparticles;
   iv) below the threshold temperature, the volume occupied by ice-balls in the body part is larger than the volume occupied by the nanoparticles in the body part;
      and
   v) below the threshold temperature, the size or diameter of at least one ice-ball is larger than the size or diameter of at least one nanoparticle.

### NANOPARTICLES

As used herein, the term *"nanoparticle"* is meant to include any nano sized material with at least one dimension such as length, width, surface, volume, or thickness, within the size range of 0.1-1000 nm, preferentially within the size range of 1-100 nm.

As used herein, the term *"magnetic nanoparticle"* is meant to include any nanoparticle which gives rise to a response when it is subjected to a magnetic field, where the response can be: i), a non-zero magnetization or coercivity, ii) a coercivity or magnetization that increases in strength with increasing magnetic field strength, iii) a nanoparticle magnetic moment that gets coupled with the magnetic field, and/or iv) a nanoparticle movement, preferentially induced when the magnetic field is non-uniform spatially. This term is meant to also include ferromagnetic, ferrimagnetic, paramagnetic, superparamagnetic and diamagnetic materials. Non-limiting suitable examples can include : i) Fe₂0₃, Fe₃0₄, Fe₂0₄, FeₓPt_{y}, CoₓPt_{y}, MnFeₓO_{y}, CoFeₓO_{y}, NiFeₓO_{y}, CuFeₓO_{y}, ZaFeₓO_{y}, and CdFeₓO_{y}, wherein x and y are preferentially between 1 and 6, depending on the method of synthesis known in the art, and/or ii) nanoparticles comprising a magnetic material, preferentially predominantly, such as Fe, Pt, Au, Ag, Mg, Zn, Ni, or Si. A distinction can be made between nanoparticles that are magnetic in the absence of application of an external magnetic field or in the presence of a magnetic field of strength lower than 1 mT, such as those composed of iron or iron oxide, and nanoparticles that are magnetic in the presence of an external strength of strength preferentially higher than 10⁻⁵, 10⁻³, 1 or 10 mT, such as those composed of gold or silver.

As used herein, the term *"metallic nanoparticle"* is meant to include any nano sized metal with at least one dimension such as length, width, surface, volume, or thickness, within the size range of 0.1-1000 nm, preferentially within the size range of 1-100 nm. In some embodiment, the term *"metallic nanoparticle"* excludes some metallic nanoparticles such as gold or silver nanoparticles. In some embodiment, the term *"metallic nanoparticle"* only includes iron or iron oxide nanoparticles.

In one aspect, the invention relates to nanoparticle for use according to the invention or to the method according to the invention, wherein the nanoparticles have at least one property selected from the group consisting of: i) a size in the range from 1 to 10³ nm, ii) a metallic, magnetic, and/or crystallized structure, iii) a thermal conductivity in the range from 10⁻⁵ to 10⁵ W/mK, and iv) a concentration in the range from 10⁻⁵ to 10⁵ mg of nanoparticles per cm³ of suspension or mg of nanoparticles per cm³ of body part.

In one embodiment of this invention, the nanoparticle is selected from: a nanosphere, a nanocapsule, a dendrimer, a carbon nanotube, a lipid/solid nanoparticle, a lipid or protein or DNA or RNA based nanoparticle, a nanoparticle with an inner aqueous environment surrounded by a layer, preferentially a stabilizing layer, most preferentially a phospholipid layer, a multilayer nanoparticle, a polymeric nanoparticle, a quantum dot, a metallic nanoparticle, a polymeric micelle or nanoparticle, a carbon based nano-structure, a nanobubble, a nanosome, a pharmacyte, a niosome, a nanopore, a microbivore, a liposome, a virus, preferentially recombinant, a herbal nanoparticle, an antibody, and a vesicle.

In some embodiment, the nanoparticle can be comprised in a liquid, gaseous, or solid environment, preferentially before, during or after its presence or administration in the body part.

In still some other cases, the nanoparticles can be comprised in a ferrofluid, a chemical or biological ferrofluid, wherein chemical and biological ferrofluids are fluids containing iron, preferentially forming nanoparticles, which are fabricated through a chemical or biological synthesis, respectively.

In still some other cases, the ferrofluid or nanoparticle assembly can comprise the nanoparticles and an excipient, a solvent, a matrix, a gel, which preferentially enables the administration of the nanoparticles to the individual or body part.

In still some other cases, the nanoparticle can comprise synthetic material and/or biological material and/or inorganic material and/or organic material.

In one embodiment of the invention, (the) nanoparticle(s) can have at least one property in common with: i) a suspension of nanoparticles, ii) a composition comprising nanoparticles, iii) an assembly of nanoparticles, iv) the mineral part of the nanoparticle, vi) the organic part of the nanoparticle, vii) the inorganic part of the nanoparticle, viii) the coating of the nanoparticle, or ix) the compound.

In one embodiment of the invention, nanoparticles are characterized by an assembly of more than 1, 10, 10², 10³, 10⁵, 10¹⁰ or 10⁵⁰ nanoparticle(s) or nanoparticle(s) per cm³ of body part.

In another embodiment of the invention, nanoparticles are characterized by an assembly of less than 10⁵⁰, 10¹⁰, 10⁵, 10³, 10², 10, 5 or 2 nanoparticle(s) or nanoparticle(s) per cm³ of body part.

In one embodiment of the invention, nanoparticle(s) represent(s) or is or are an assembly or suspension or composition of more or comprising more than 10⁻¹⁰⁰, 10⁻⁵⁰, 10⁻¹⁰, 10⁻⁵, 10⁻¹, 1, 10, 10², 10³, 10⁵, 10¹⁰, 10²⁰ or 10⁵⁰ nanoparticle(s) or mg of nanoparticle(s) or mg of iron comprised in nanoparticle(s) or mg of nanoparticle(s) per cm³ or mg of nanoparticle(s) per cm³ of body part or mg of iron comprised in nanoparticle(s) per cm³ or mg of iron comprised in nanoparticle(s) per cm³ of body part. In some embodiment, an assembly or suspension or composition comprising a large number of nanoparticles can be used to induce or produce a temperature increase, radical or reactive species, or the dissociation of a compound from the nanoparticles.

In another embodiment of the invention, nanoparticle(s) represent(s) or is or are an assembly or suspension or composition of less or comprising less than 10¹⁰⁰, 10⁵⁰, 10²⁰, 10¹⁰, 10⁵, 10², 10, 1, 5, 2, 1, 10⁻¹, 10⁻⁵, 10⁻¹⁰ or 10⁻⁵⁰ nanoparticle(s) or mg of nanoparticle(s) or mg of iron comprised in nanoparticle(s) or mg of nanoparticle(s) per cm³ or mg of nanoparticle(s) per cm³ of body part or mg of iron comprised in nanoparticle(s) per cm³ or mg of iron comprised in nanoparticle(s) per cm³ of body part. In some embodiment, an assembly or suspension or composition of nanoparticles comprising a low number of nanoparticle(s) can be used to prevent toxicity.

The invention also relates to nanoparticles for use according to the invention, wherein the nanoparticles are crystallized, metallic, or magnetic.

In an embodiment of the invention, the nanoparticles are crystallized. In this case, they preferentially possess more than or at least 1, 2, 10, 10², 10³, 10⁶ or 10⁹ crystallographic plane(s) or regular atomic arrangement(s), preferentially observable by electron microscopy.

In one embodiment of the invention, the nanoparticles are metallic. In this case, they contain at least 1, 10, 10³, 10⁵ or 10⁹ metallic atom(s) or contain at least 1, 10, 50, 75 or 90% of metallic atoms, where this percentage can be the ratio between the number or mass of metallic atoms in the nanoparticle divided by the total number or mass of all atoms in the nanoparticle. The nanoparticles, preferentially metal oxide nanoparticles, can also contain at least 1, 10, 10³, 10⁵ or 10⁹ oxygen atom(s), or contain at least 1, 10, 50, 75 or 90% of oxygen atoms, where this percentage can be the ratio between the number or mass of oxygen atoms in the nanoparticles divided by the total number or mass of all atoms in the nanoparticles.

In another embodiment of the invention, the metal or metal atom is selected in the list consisting of: Lithium, Beryllium, Sodium, Magnesium, Aluminum, Potassium, Calcium, Scandium, Titanium, Vanadium, Chromium, Manganese, Iron, Cobalt, Nickel, Copper, Zinc, Gallium, Rubidium, Strontium, Yttrium, Zirconium, Niobium, Molybdenum, Technetium, Ruthenium, Rhodium, Palladium, Silver, Cadmium, Indium, Tin, Cesium, Barium, Lanthanum, Cerium, Praseodymium, Neodymium, Promethium, Samarium, Europium, Gadolinium, Terbium, Dysprosium, Holmium, Erbium, Thulium, Ytterbium, Lutetium, Hafnium, Tantalum, Tungsten, Rhenium, Osmium, Iridium, Platinum, Gold, Mercury, Thallium, Lead, Bismuth, Polonium, Francium, Radium, Actinium, Thorium, Protactinium, Uranium, Neptunium, Plutonium, Americium, Curium, Berkelium, Californium, Einsteinium, Fermium, Mendelevium, Nobelium, Lawrencium, Rutherfordium, Dubnium, Seaborgium, Bohrium, Hassium, Meitnerium, Darmstadtium, Roentgenium, Copernicium, Nihonium, Flerovium, Moscovium, and Livermorium or Livermorium atom.

In another embodiment of the invention, the nanoparticle contains less than 1, 10, 10³, 10⁵ or 10⁹ metallic atom(s) or contains less than 1, 10, 50, 75 or 90% of metallic atoms, where this percentage can be the ratio between the number or mass of metallic atoms in the nanoparticle divided by the total number or mass of all atoms in the nanoparticle. It can also contain less than 1, 10, 10³, 10⁵ or 10⁹ oxygen atom(s), or contain less than 1, 10, 50, 75 or 90% of oxygen atoms, where this percentage can be the ratio between the number or mass of oxygen atoms in the nanoparticle divided by the total number or mass of all atoms in the nanoparticle.

In one embodiment of the invention, the nanoparticle is magnetic when it has a magnetic behavior or property, where the magnetic behavior or property is preferentially selected from the group consisting of a diamagnetic, superparamagnetic, paramagnetic, ferromagnetic, and ferrimagnetic behavior or property.

In some embodiment, the magnetic behavior or property exists at a temperature, which is lower than: i) 10⁵, 10³, 500, 350, 200, 100, 50, 20, 10, 1, 0.5 or 1 K (Kelvin), ii) the Curie temperature, or iii) the blocking temperature.

In some other embodiment, the magnetic behavior or property exists at a temperature, which is larger than: i) 0.5, 1, 10, 20, 50, 100, 200, 350, 500, 10³ or 10⁵ K, ii) the Curie temperature, or iii) the blocking temperature.

In still some other embodiment, the magnetic behavior or property exists at a temperature, which is between 10⁻²⁰ and 10²⁰ K, or between 0.1 and 1000 K.

In one embodiment of the invention, the nanoparticles have or are characterized by at least one of the following properties: i) the presence of a core, preferentially magnetic, preferentially mineral, preferentially composed of a metallic oxide such as iron oxide, most preferentially maghemite or magnetite, or an intermediate composition between maghemite and magnetite, ii) the presence of a coating that surrounds the core and preferentially prevents nanoparticle aggregation, preferentially enabling nanoparticle administration in an organism or in the body part or stabilizing the nanoparticle core, where coating thickness may preferably lie between 0.1 nm and 10 µm, between 0.1 nm and 1 µm, between 0.1 nm and 100 nm, between 0.1 nm and 10 nm, or between 1 nm and 5 nm, iii) magnetic properties leading to diamagnetic, paramagnetic, superparamagnetic, ferromagnetic, or ferrimagnetic behavior, iv) a coercivity larger than 0.01, 0.1, 1, 10, 100, 10³, 10⁴, 10⁵, 10⁹ or 10²⁰ Oe, v) a ratio between remnant and saturating magnetization larger than 0.01, 0.1, 0.2, 0.3, 0.4, 0.5, 0.75, 0.9 or 0.99, vi) a saturating magnetization larger than 0.1, 1, 5, 10 or 50 emu/g, vii) magnetic properties such as coercivity, remnant and saturating magnetization, preferentially measured or observed at a temperature larger than 0.1 K, 1 K, 10 K, 20 K, 50 K, 100 K, 200 K, 300 K, 350 K or 3000 K, viii) a crystallinity, *i.e.* nanoparticles preferentially possessing at least 1, 2, 5, 10 or 100 crystalline plane(s), preferentially observable or measured by electron microscopy, ix) the presence of a single domain, x) a size that is larger than 0.1, 0.5, 1.5, 10, 15, 20, 25, 30, 50, 60, 70, 80, 100, 120, 150 or 200 nm, xi) a size lying between 0.1 nm and 10 µm, between 0.1 nm and 1 µm, between 0.1 nm and 100 nm, between 1 nm and 100 nm, or between 5 nm and 80 nm, xii) a non-pyrogenicity or apyrogenicity, which preferentially means that nanoparticles possess an endotoxin concentration lower than 10²⁰, 10000, 1000, 100, 50, 10, 5, 2 or 1 EU (endotoxin unit) per mg of nanoparticle or per mg of iron comprised in nanoparticles, or which means that nanoparticles do not trigger fever or an increase in whole body temperature larger than 100, 50, 6.6, 5, 3, 2 or 1 °C following their administration to a living organism or body part, xiii) a synthesis by a synthetizing living organism, preferentially by bacteria, xiv) a chemical synthesis, xv) the presence of less than 50, 25, 15, 10, 5, 2 or 1% of organic or carbon material originating from the synthetizing living organism, xv), the presence of more than 99, 95, 80, 70, 60, 50 or 25% of mineral material originating from the synthetizing living organism, or xvi) a specific absorption rate (SAR) that is larger than 1, 10, 1000 or 10⁴ Watt per gram of nanoparticle, preferentially measured under the application of an alternating magnetic field of strength preferentially larger than 0.1, 1, 10 or 100 mT, and/or frequency larger than 1, 10, 100 or 1000 KHz, alternatively preferentially measured under the application of the acoustic wave, alternatively under the application of a radiation such as an electromagnetic acoustic, or light radiation.

In another embodiment of the invention, the nanoparticles have or are characterized by at least one of the following properties: i) a coercivity lower than 0.01, 0.1, 1, 10, 100, 10³, 10⁴, 10⁵, 10⁹ or 10²⁰ Oe, ii) a ratio between remnant and saturating magnetization lower than 0.01, 0.1, 0.2, 0.3, 0.4, 0.5, 0.75, 0.9 or 0.99, iii) a saturating magnetization lower than 0.1, 1, 5, 10, 50, 200, 1000 or 5000 emu/g, iv) magnetic properties preferentially measured or observed at a temperature lower than 0.1 K, 1 K, 10 K, 20 K, 50 K, 100 K, 200 K, 300 K, 350 K or 3000 K, v) a size that is lower than 0.1, 0.5, 1.5, 10, 15, 20, 25, 30, 50, 60, 70, 80, 100, 120, 150 or 200 nm, vi) the presence of more than 50, 25, 15, 10, 5, 2 or 1% of organic or carbon material originating from the synthetizing living organism, vii) the presence of less than 99, 95, 80, 70, 60, 50 or 25% of mineral material originating from the synthetizing living organism, or xi), a specific absorption rate (SAR) that is lower than 1, 10, 1000 or 10⁴ Watt per gram of nanoparticle, preferentially measured under the application of an alternating magnetic field of strength preferentially lower than 0.1, 1, 10, or 100, 200, 500, 10³ or 10⁵ mT, and/or of frequency preferentially lower than 1, 10, 100, 10³, 10⁵ or 10⁹ KHz, alternatively preferentially measured under the application of the acoustic wave, alternatively under the application of a radiation such as an electromagnetic acoustic, or light radiation.

In some embodiment, the mineral can be the part of the nanoparticle or magnetosome that does not comprise organic material or comprises a low percentage in mass of organic material, preferentially less than 100, 99, 50, 20, 10, 5, 1, 10⁻¹ or 10⁻² percent or percent in mass of organic material. The mineral is preferentially the core of the nanoparticle.

In some other embodiment, the mineral can comprise a percentage in mass of organic material larger than 0, 10⁻⁵⁰, 10⁻¹⁰, 10⁻², 10⁻¹ or 1 percent or percent in in mass of organic material. This can be the case when the purification step unsuccessfully removes the organic material or when organic material is added to the mineral after the purification step.

In some embodiment, the nanoparticles can be surrounded by a coating. The coating can be made of a synthetic, organic, or inorganic material or of a substance comprising a function selected in the group consisting of carboxylic acids, phosphoric acids, sulfonic acids, esters, amides, ketones, alcohols, phenols, thiols, amines, ether, sulfides, acid anhydrides, acyl halides, amidines, amides, nitriles, hydroperoxides, imines, aldehydes, and peroxides. In some cases, the coating can be made of carboxymethyl-dextran, citric acid, phosphatidylcholine (DOPC), or oleic acid. In some cases, the coating can enable the dispersion of the nanoparticles in a matrix or solvent such as water, preferentially without aggregation or sedimentation of the nanoparticles. In some cases, the coating can enable internalization of the nanoparticles in cells. In some other cases, the coating can enable: i) to bind two or more nanoparticle(s) together preferentially in a chain, ii) to prevent nanoparticle aggregation and/or, iii) to obtain uniform nanoparticle distribution.

In one embodiment of the invention, the nanoparticles are non-pyrogenic. Non-pyrogenic nanoparticles preferentially: i) comprise less than 10¹⁰⁰, 10⁵⁰, 10²⁰, 10⁸, 10⁵, 10³, or 10 EU (endotoxin unit) or EU per cm³ of body part or EU per mg of nanoparticle or EU per cm³ of body part per mg of nanoparticle, or ii) induce a temperature increase of the individual or body part of less than 10⁵, 10³, 10², 50, 10, 5, 4, 3, 2 or 1 °C, preferentially above physiological temperature, preferentially before, after or without the application of the acoustic wave or radiation on the nanoparticle.

In one embodiment of this invention, the nanoparticle is composed of or comprises a chemical element of the families selected from the group consisting of: metals (alkali metal, alkaline earth metal, transition metals), semimetal, non-metal (halogens element, noble gas), chalcogen elements, lanthanide, and actinide.

In another embodiment of the invention, the nanoparticle is composed of or comprises a chemical element selected from the group consisting of: hydrogen, lithium, sodium, potassium, rubidium, caesium, francium, beryllium, magnesium, calcium, strontium, barium, radium, scandium, yttrium, lanthanide, actinide, titanium, zirconium, hafnium, rutherfordium, vanadium, niobium, tantalum, dubnium, chromium, molybdenum, tungsten, seaborgium, manganese, technetium, rhenium, bohrium, iron, ruthenium, osmium, hessium, cobalt, rhodium, iridium, meitherium, nickel, palladium, platinum, darmstadtium, copper, silver, gold, roentgenium, zinc, cadmium, mercury, copernicum, boron, aluminium, gallium, indium, thallium, ununtrium, carbon, silicon, germanium, tin, lead, fleovium, nitrogen, phosphorus, arsenic, antimony, bismuth, ununpentium, oxygen, sulphur, selenium, tellurium, polonium, livermorium, fluorine, chlorine, bromine, iodine, astatine, ununseptium, helium, neon, argon, krypton, xenon, radon, ununoctium, lanthanum, cerium, praseodymium, neodymium, promethium, samarium, europium, gadolinium, terbium, dysprosium, holmium, erbium, thulium, ytterbium, lutetium, actinium, thorium, proctactinium, uranium, neptunium, plutonium, americium, curium, berkelium, californium, einsteinium, fermium, mendelevium, nobelium, and lawrencium.

In some embodiment, the nanoparticle can also be composed of or comprise an alloy, a mixture, or an oxide of this(these) chemical element(s).

In some embodiment, the nanoparticle can be composed of more than 10⁻⁵⁰, 10⁻²⁰, 10⁻¹⁰, 10⁻⁵, 10⁻², 1, 5, 10, 50, 75, 80, 90, 95 or 99% of one or several of this(these) element(s), where this percentage can represent the mass or number of this(these) chemical elements comprised in the nanoparticle divided by the total number or total mass of all chemical elements comprised in the nanoparticle or by the total mass of the nanoparticle or compound.

In some other cases, the nanoparticle can be composed of or comprise less than 10⁻⁵⁰, 10⁻²⁰, 10⁻¹⁰, 10⁻⁵, 10⁻², 1, 5, 10, 50, 75, 80, 90, 95 or 99% of one or several of this(these) chemical element(s).

In still some other embodiment, this(these) chemical element(s) is(are) comprised inside the nanoparticle or compound, or at the surface of the nanoparticle or compound, or in the mineral or central part of the nanoparticle or compound, or in the coating of the nanoparticle or compound.

In one embodiment of the invention, the nanoparticle is characterized by a size in one dimension, which is larger than 10⁻¹, 1, 2, 5, 10, 20, 50, 70, 100, 200 or 500 nm. A nanoparticle with a large size can have a larger coercivity and/or a larger remnant magnetization and/or can more strongly or more efficiently absorb the energy or power of the acoustic wave than a nanoparticle with a small size. In some cases, the amount of energy or power absorbed by a nanoparticle is increased by a factor of more than 1.001, 1.01, 1.1, 1.2, 1.5, 2, 5, 10, 10³, 10⁵ or 10⁷ by increasing the size of the nanoparticle by a factor of more than 1.001, 1.01, 1.1, 1.2, 1.5, 2, 5, 10, 10³, 10⁵ or 10⁷.

In another embodiment of the invention, the nanoparticle is defined as a particle with a size in one dimension, which is lower than 10⁴, 10³, 10², 10, 1 or 10⁻¹ nm. A nanoparticle with a small size can more easily be administered, for example intravenously, or can enable the avoidance of some toxicity effects, such as embolism.

In still another embodiment of the invention, the nanoparticle size is between 10⁻² and 10²⁰ nm, 10⁻² and 10⁴ nm, between 10⁻¹ and 10³ nm, or between 1 and 10² nm. This can be the case when the nanoparticle or nanoparticle assembly possesses a well-defined, preferentially narrow, distribution in sizes.

In still another embodiment of the invention, the nanoparticle size distribution is lower than 1000, 100, 75, 50, 25, 10, 5, 2 or 1 nm. A narrow nanoparticle size distribution may be desired to prevent aggregation, or to favor an organization in chains of the nanoparticles.

In still another embodiment of the invention, the nanoparticle size distribution is larger than 1000, 100, 75, 50, 25, 10, 5, 2 or 1 nm. A large nanoparticle size distribution may in some cases enable nanoparticles to be eliminated more rapidly.

In another embodiment of the invention, the nanoparticle has a surface charge, which is higher than -200, -100, -50, -10, -5, 0.1, 1, 2, 5, 10, 50 or 100 mV, preferentially at a pH lower than 0.1, 1, 2, 3, 4, 5, 6, 7, 8, 9, 10, 11, 12, 13 or 14. Preferentially, a nanoparticle can have a high surface charge at low pH when it is surrounded by a coating that enables to reach such charge without being destroyed.

In another embodiment of the invention, the nanoparticle has a surface charge, which is lower than -200, -100, -50, -10, -5, 0.1, 1, 2, 5, 10, 50 or 100 mV, preferentially at a pH larger than 0.1, 1, 2, 3, 4, 5, 6, 7, 8, 9, 10, 11, 12, 13 or 14. A nanoparticle can have a low surface charge at high pH when it is surrounded by a coating that enables to reach such charge without being destroyed.

In still another embodiment of the invention, the nanoparticle has a surface charge comprised between +200 and -200 mV, +100 and -100 mV, +50 and -50 mV, +40 et-40mV, +20 and -20, +10 and -10 mV, or between +5 and -5 mV, preferentially at a pH lower than 0.1, 1, 2, 3, 4, 5, 6, 7, 8, 9, 10, 11, 12, 13 or 14.

In still another embodiment of the invention, the nanoparticle has a surface charge comprised between +200 and -200 mV, +100 and -100 mV, +50 and -50 mV, +40 et-40mV, +20 and -20, +10 and -10 mV, or between +5 and -5 mV, preferentially at a pH larger than 0.1, 1, 2, 3, 4, 5, 6, 7, 8, 9, 10, 11, 12, 13 or 14.

In another embodiment of the invention, the nanoparticle has a weight or a mass, preferentially expressed in unit such as gram (g), kilogram (kg), or milligram (mg). A gram of nanoparticle can be a gram of metal such as iron comprised in the nanoparticle. The mass or weight of the nanoparticle can correspond to the mass or weight of one nanoparticle or to the mass or weight of an assembly of nanoparticles.

In an embodiment, the mass of the nanoparticle is larger than 10⁻²⁰, 10⁻¹⁰, 10⁻⁵, 10⁻², 1, 10, 10³, 10⁹ or 10²⁰ gram. In some cases, a large nanoparticle mass may be desired to increase the quantity of acoustic wave energy absorbed by the nanoparticle.

In an embodiment, the mass of the nanoparticle is lower than 10⁻²⁰, 10⁻¹⁰, 10⁻⁵, 10⁻², 1, 10, 10³, 10⁹ or 10²⁰ gram. In some cases, a low nanoparticle mass may be desired to prevent or minimize nanoparticle toxicity.

In one embodiment of the invention, the nanoparticle, the suspension, composition, or assembly of nanoparticle is stable, preferentially during a lapse of time, preferentially being its stability duration, which is larger than 10⁻¹⁰, 5, 10, 10⁵⁰ or 10¹⁰⁰ minute(s). In some cases, the nanoparticle, the suspension, composition, or assembly of nanoparticle can be stable at a concentration of nanoparticles larger than 1, 5, 10, 50, 100, 200, 500 or 1000 mg of nanoparticles per mL of solvent, matrix, or body part surrounding or comprising the or nanoparticle. In some cases, the nanoparticle, the suspension, composition, or assembly of nanoparticle can be stable when: i) the nanoparticle is not degraded or does not lose partly or fully its coating or can be administered to the body part, or ii) the optical density of the nanoparticle, the suspension, composition, or assembly of nanoparticle, preferentially measured at 480 nm or at another fixed wavelength, does not decrease by more than 1, 5, 10, 50, 75 or 90 % or by more than 10⁻¹⁰, 10⁻³, 10⁻¹, 0.5 or 0.7, within 1, 5, 10, 10³, 10⁷ or 10²⁰ seconds following homogenization or mixing or optical density measurement or absorption measurement of this suspension or composition. This percentage can be equal to (OD_{B}-OD_{A})/OD_{B} or OD_{A}/OD_{B}, where OD_{B} is the optical density of the nanoparticle, the suspension, composition, or assembly of nanoparticle measured before the homogenization or mixing or optical density measurement or absorption measurement of the nanoparticle, the suspension, composition, or assembly of nanoparticle and OD_{A} is the optical density of the nanoparticle, the suspension, composition, or assembly of nanoparticle measured after the homogenization or mixing or optical density measurement or absorption measurement of the nanoparticle, the suspension, composition, or assembly of nanoparticle.

In some cases, the nanoparticle can be suspended in a liquid or dispersed in a matrix or body part to yield a homogenous nanoparticle dispersion or a highly stable nanoparticle composition or suspension.

In one embodiment of the invention, the nanoparticles are arranged in chains comprising more than 1, 2, 3, 4, 5, 6, 7, 8, 9, 10, 15, 20, 25, 30, 35 or 40 nanoparticles.

In another embodiment of the invention, the nanoparticles are arranged in chains, which have: i) a length smaller than 2.10¹⁰, 2.10⁵, 2.10³ or 2.10² nm, or ii) a number of nanoparticles in each chain smaller than 2, 5, 10, 10² or 10³. In some cases, short chains of nanoparticles may be desired or obtained, for example to favor nanoparticle internalization in cells or after partial or total destruction of long chains.

In another embodiment of the invention, the nanoparticles are arranged in chains, which have: i) a length longer than 10⁻¹, 1, 5, 10, 2.10², 2.10³ or 2.10⁵, or ii) a number of nanoparticles in each chain larger than 2, 5, 10, 10² or 10³. In some cases, long chains of nanoparticles may be desired or obtained to increase the quantity of heat or compounds dissociated from the nanoparticles under the application of an acoustic wave or radiation or to prevent nanoparticle aggregation or enable uniform nanoparticle distribution.

In still another embodiment of the invention, the nanoparticles are arranged in chains, which have: i) a length between 10⁻¹ and 10¹⁰ nm, or between 1 and 10⁵ nm, or ii) a number of nanoparticles in each chain between 2 and 10⁵, 2 and 10³, 2 and 10², or between 2 and 50.

In still another embodiment of the invention, the nanoparticles are arranged in chains when they are bound or linked to each other or when the crystallographic directions of two adjacent nanoparticles in the chain are aligned, wherein such alignment is preferentially characterized by an angle between two crystallographic directions belonging to two adjacent nanoparticles in the chains of less than 90, 80, 70, 60, 50, 20, 10, 3, or 2 °C (degree).

Preferentially when the nanoparticles are biologically synthesized, the nanoparticles can be arranged in chains: i) inside the organism that synthesizes them, also designated as synthetizing living organism, or ii) outside this organism. Preferentially, nanoparticles are arranged in chains after or before their extraction or isolation from this organism.

In one embodiment of the invention, the nanoparticles are not arranged in chains.

In another embodiment of the invention, the nanoparticles are synthesized chemically or are not synthesized by a living organism when less than 1, 2, 5, 10 or 100 step(s) of their production, such as crystallization of iron oxide, stabilization of the iron oxide mineral, organization of the nanoparticles, involves or is due to a living organism. In some cases, a chemical synthesis can be defined as a synthesis involving a majority of steps, or more than 1, 2, 5 or 10 steps, or more than 1, 2, 5, 25, 50, 75 or 90% of steps, which involve chemical reactions occurring without the involvement of living organisms, or parts of living organisms such as DNA, RNA, proteins, enzymes, lipids.

In another embodiment of the invention, a chemical synthesis can be used to produce a chemical substance that mimics, copies, or reproduces the compartment, organelle, or other biological material, wherein this chemical synthesis or chemical substance can be used or can result in the production of the nanoparticles. In some cases, the compartment, organelle, or other biological material, can be a lysosome, an endosome, a vesicle, preferentially biological material that has the capacity or the function either to dissolve or transform crystallized iron into free iron or to transform free iron into crystalized iron. In some cases, this transformation is partial and preferentially results in the destruction or formation of partly crystallized assembly of iron atoms or ions, or preferentially results in a mixture of crystallized iron and non-crystallized iron. In some cases, crystallized iron can be defined as an assembly of iron atoms or ions that leads to the presence of crystallographic planes, preferentially observable using a technique such as transmission or scanning electron microscopy as a characterization method, and free iron can preferentially be defined as one of several iron atoms or ions that do not lead to the presence of crystallographic planes, preferentially highlighted by the absence of diffraction patterns, using for example transmission or scanning electron microscopy as a characterization method.

In one embodiment of the invention, the nanoparticles are synthesized biologically or by a living organism, designated as synthetizing living organism, which preferentially consists or comprises at least 1, 2, 5, 10, 10³, 10⁶ or 10⁹ eukaryotic cell(s), prokaryotic cell(s), or part of these cells. In some cases, part of eukaryotic or prokaryotic cell(s) can be biological material originating or produced by these cells such as RNA, DNA, organelle, nucleolus, nucleus, ribosome, vesicle, rough endoplasmic reticulum, Golgi apparatus, cytoskeleton, smooth endoplasmic reticulum, mitochondrion, vacuole, cytosol, lysosome, centrosome, cell membrane. In some cases, a biological synthesis can be defined as a synthesis involving a majority of steps, or more than 1, 2, 5 or 10 steps, or more than 1, 2, 5, 25, 50, 75 or 90% of steps, which involve chemical reactions occurring with the involvement of at least 1, 2, 10, 10³, 10⁶ or 10⁹ living organisms, or parts of living organisms such as DNA, RNA, proteins, enzymes, lipids.

In some embodiment, the synthetizing living organism can be magnetotactic bacteria, other types bacteria than magnetotactic bacteria or enzymes of certain bacteria, preferentially synthetizing nanoparticles extra-cellularly, such as Mycobacterium paratuberculosis, Shewanella oneidensi, Geothrix fermentans, ants, fungi, or various plants.

In still another embodiment of the invention, the nanoparticles are synthesized or produced or crystallized or assembled or transformed into a nanoparticle by a compartment, organelle, or other biological material, such as protein, lipid, enzyme, DNA, or RNA, which is preferentially produced by or originates from an eukaryotic or prokaryotic cell.

In another embodiment of the invention, the nanoparticles are synthesized by or in at least one eukaryotic cell, prokaryotic cell, or part of this cell.

In another embodiment of the invention, the nanoparticles are synthesized by or in: i) the matrix or medium or environment located outside of at least one eukaryotic cell, prokaryotic cell, or part of this cell, or ii) the extracellular matrix.

In one embodiment of the invention, the nanoparticles are synthesized by a living organism when at least 1, 2, 5, 10 or 100 step(s) of their production, such as crystallization of iron oxide, stabilization of the iron oxide mineral, organization of the nanoparticles, for example in chains or aggregates, involves or is due to a living organism.

### BODY PART

Preferably, the individual is a living organism.

In one embodiment of the invention, the individual is a mammal, a bird, a fish, a human, a plant, a fungus, or an archaea.

In an embodiment of the invention, the body part comprises more than or is an assembly of more than 1, 2, 5, 10, 10³, 10⁵, 10¹⁰, 10⁵⁰ or 10¹⁰⁰ cell(s), apparatus, tissue(s), organ(s), biomolecule(s), molecule(s), atom(s), entities(s), or biological material(s), preferentially as measured per cm³ of body part.

In another embodiment of the invention, the body part comprises less than or is an assembly of less than 10¹⁰⁰, 10⁵⁰, 10¹⁰, 10⁵, 10³, 10, 5, 2 or 1 cell(s), apparatus, tissue(s), organ(s), biomolecule(s), molecule(s), atom(s), entities(s), or biological material(s), preferentially as measured per cm³ of body part.

In another embodiment of the invention, the body part comprises between or is an assembly of between 1 and 10¹⁰⁰, 1 and 10¹⁰, or 1 and 10³ cell(s), apparatus, tissue(s), organ(s), biomolecule(s), molecule(s), atom(s), entities(s), or biological material(s), preferentially as measured per cm³ of body part.

In some embodiment, the apparatus, the tissue(s), organ(s), biomolecule(s), molecule(s), atom(s), entities(s), and/or biological material(s) is/are the same or belong to an assembly comprising the same tissue(s), organ(s), biomolecule(s), molecule(s), atom(s), entities(s), or biological material(s).

In some other embodiment, the apparatus, the tissue(s), organ(s), biomolecule(s), molecule(s), atom(s), entities(s), or biological material(s) is different or belongs to an assembly comprising different tissue(s), organ(s), biomolecule(s), molecule(s), atom(s), entities(s), or biological material(s).

In still some other embodiment, the apparatus, the tissue(s), organ(s), biomolecule(s), molecule(s), atom(s), entities(s), and/or biological material(s) belong(s) to, originate(s) from, is/are produced by a living organism.

In still some other embodiment, the apparatus, the tissue(s), organ(s), biomolecule(s), molecule(s), atom(s), entities(s), and/or biological material(s) doesn't/don't: i), belong to, ii), originate from, and/or iii) is/are produced by a living organism.

In still some other embodiment, the body part is a whole or part of a living organism.

In one embodiment of the invention, the living organism or body part is or comprises at least 1, 10, 10³, 10⁵, 10¹⁰ or 10¹⁰⁰ eukaryotic or prokaryotic cell(s), DNA, RNA, protein, lipid, biological material, cell organelle, cell nucleus, cell nucleolus, ribosome, endoplasmic reticulum, Golgi apparatus, chloroplast, or mitochondria.

In some embodiment, the body part is all or part of the head, neck, shoulder, arm, leg, knee, foot, hand, ankle, elbow, trunk, inferior members, or superior members. In some other cases, the body part can be or belong to an organ, the musculoskeletal, muscular, digestive, respiratory, urinary, female reproductive, male reproductive, circulatory, cardiovascular, endocrine, circulatory, lymphatic, nervous (peripheral or not), ventricular, enteric nervous, sensory, or integumentary system, reproductive organ (internal or external), sensory organ, endocrine glands. The organ or body part can be human skeleton, joints, ligaments, tendons, mouth, teeth, tongue, salivary glands, parotid glands, submandibular glands, sublingual glands, pharynx, esophagus, stomach, small intestine, duodenum, jejunum, ileum, large intestine, liver, gallbladder, mesentery, pancreas, nasal cavity, pharynx, larynx, trachea, bronchi, lungs, diaphragm, kidneys, ureters, bladder, urethra, ovaries, fallopian tubes, uterus, vagina, vulva, clitoris, placenta, testes, epididymis, vas deferens, seminal vesicles, prostate, bulbourethral glands, penis, scrotum, pituitary gland, pineal gland, thyroid gland, parathyroid glands, adrenal glands, pancreas, heart, arteries, veins, capillaries, lymphatic vessel, lymph node, bone marrow, thymus, spleen, gut-associated lymphoid tissue, tonsils, brain, cerebrum, cerebral hemispheres, diencephalon, brainstem, midbrain, pons, medulla, oblongata, cerebellum, spinal cord, choroid plexus, nerves, cranial nerves, spinal nerves, ganglia, eye, cornea, iris, ciliary body, lens, retina, ear, outer ear, earlobe, eardrum, middle ear, ossicles, inner ear, cochlea, vestibule of the ear, semicircular canals, olfactory epithelium, tongue, taste buds, mammary glands, or skin. The body part or organ can belong to the blood circulation or circulatory system.

In some embodiment, the body part can comprise at least one tumor, cancer, virus, bacterium, or pathological cell.

In one embodiment of the invention, the body part comprises water, an excipient, a solution, a suspension, at least one chemical element, organic material, or gel. In some embodiment, the body part can be synthetic, *i.e.* preferentially be produced with chemicals to mimic the body part of a living organism. In some other embodiment, the body part can be produced by a living organism.

In an embodiment of the invention, the body part comprises a pathological site, a healthy site, and/or a nanoparticle region.

In one embodiment of the invention, the body part is or comprises a pathological site or pathological cells.

In some embodiment, the pathological site is an unhealthy site, or a site that is in a different condition from a site of a healthy individual, or the site of an unhealthy individual. It can comprise pathological cells, such as tumor cells, bacteria, eukaryotic or prokaryotic cells, as well as viruses or other pathological material. Pathological cells can be cells that are: i) not arranged or working as they usual do in a healthy individual, ii) dividing more quickly than healthy cells, iii) healthy cells having undergone a transformation or modification, iv) dead, sometimes due to the presence of a virus or to other organisms, or v), in contact, in interaction, with foreign material not belonging to the individual, such as viruses, where viruses can possibly penetrate, colonize, or replicate in these cells. In some cases, pathological cells can be assimilated to viruses or to other organisms or entities that colonize cells or target cells or destroy cells or use cells or enter in interaction with cells, preferentially to enable their own reproduction, multiplication, survival, or death. In some cases, a pathological site can comprise healthy cells, preferentially with a lower number, activity or proliferation, than that of pathological cells.

In one embodiment of the invention, the body part is or comprises a healthy site or healthy cells. In some cases, the healthy site can be defined as a site or region that comprises healthy cell(s), where a healthy cell can be defined as a cell that belongs to a healthy individual or to the body part of a healthy individual.

In some embodiment, the healthy site surrounds the pathological site preferentially when it is located at a distance of less than 1, 10⁻¹, 10⁻³, 10⁻⁶ or 10⁻⁹ m from the pathological site.

In some embodiment, the number of pathological or healthy cells, preferentially comprised in the body part or volume exposed to the treatment as defined in the invention, is lower than 10¹⁰⁰, 10⁵⁰, 10²⁰, 10¹⁰, 10⁵, 10, 5, 2 or 1 cell(s) preferentially per cm³ of body part.

In some other embodiment, the number of pathological or healthy cells, preferentially comprised in the body part or volume exposed to the treatment as defined in the invention, can be larger than 1, 10, 10³, 10⁵, 10⁷, 10⁹, 10²⁰, 10⁵⁰ or 10¹⁰⁰ cell(s) preferentially per cm³ of body part.

In still some other embodiment, the ratio between the number of pathological cells and the number of healthy cells, preferentially comprised in the body part or volume exposed to the treatment according to the invention, is lower than 10¹⁰⁰, 10⁵⁰, 10²⁰, 10¹⁰, 10⁵, 10³, 10², 10, 5, 2 or 1.

In still some other embodiment, the ratio between the number of pathological cells and the number of healthy cells, preferentially comprised in the body part or volume exposed to the treatment according to the invention, is larger than 1, 2, 5, 10, 10³, 10⁵, 10²⁰ or 10¹⁰⁰.

In another embodiment of the invention, the body part, healthy or pathological site, or nanoparticle region, has a length, surface area, or volume, which is larger than 10⁻⁵⁰, 10⁻¹⁰, 10⁻⁵, 10⁻³, 10⁻¹, 1, 5, 10 or 10³ m (for length), m² (for surface) or m³ (for volume).

In another embodiment of the invention, the body part, healthy or pathological site, or nanoparticle region, has a length, surface area, or volume, which is lower than 10⁵⁰, 10¹⁰, 10³, 1, 10⁻¹, 10⁻², 10⁻³, 10⁻⁴, 10⁻⁵, 10⁻⁶, 10⁻⁷, 10⁻⁸ or 10⁻⁹ m (for length), m² (for surface) or m³ (for volume).

In one embodiment of the invention, an amount of nanoparticle that enables to induce cellular toxicity by the method according to the invention is 100 µg of nanoparticles per 250 000 cells (0.5 ng of nanoparticle per cell or 0.01 ng per µm of body part). In some embodiment, a lower quantity of nanoparticle, preferentially by a factor of at least 2, 5, 10, 10³ or 10⁵, can be efficient in destroying cells by the method, for example if the temperature is decreased below 0 °C during the method and/or if the method involves an important number of cycles. In some other embodiment, a larger quantity of nanoparticles, preferentially by a factor of at least 2, 5, 10, 10³ or 10⁵, can be efficient in destroying cells by the method, for example if the temperature is decreased above 0 °C during the method and/or if the method involves a limited number of cycles.

In another embodiment of the invention, the nanoparticles remain in the body part during the treatment according to the invention, preferentially during more than 1, 2, 5, 10, 20, 50, 100, 10³ or 10⁴ step(s) or cycle(s) of the treatment, according to the invention, preferentially during more than 1, 2, 5, 10, 50, 100 or 10³ second(s), hour(s), day(s), month(s) or year(s).

In some other embodiment, the nanoparticles remain in the body part during the treatment according to the without decreasing in size by more than 10⁻⁴, 10⁻¹, 1, 10, 20, 50, 100, 500, 10³ or 10⁴% between before and after nanoparticle administration in/to the body part, where this percentage can be the ratio between the size of the nanoparticle after administration of the nanoparticles in the body part and the size of the nanoparticle before administration of the nanoparticles in the body part.

In some other embodiment, the nanoparticles are in the body part during the treatment according to the invention, where they decrease in size by more than 10⁻⁴, 10⁻¹, 1, 10, 20, 50, 100, 500, 10³ or 10⁴% between before and after nanoparticle administration in/to the body part.

In some other embodiment, more than 1, 5, 10, 50, 90 or 99% of body part, preferentially by mass or volume, is/are destroyed or is/are treated by the treatment according to the invention, preferentially when more than 10⁻¹⁰, 10⁻⁵, 10⁻¹, 1, 5, 10, 10³, 10⁶ or 10¹⁰ nanoparticle(s) or mg of nanoparticle(s) per cm³ or mg of body part are comprised in the body part,

In still some other embodiment, less than 100, 99, 90, 50, 10, 5 or 1% of body part, preferentially by mass or volume, is/are destroyed, preferentially when less than 10¹⁰, 10⁶, 10³, 10, 5, 1, 10⁻¹ or 10⁻³ nanoparticle(s) or mg of nanoparticle(s) per mg or cm³ of body part are comprised in the body part.

### TREATMENT

In one embodiment of the invention, the treatment is: i) a medical, diagnostic, therapeutic and/or cosmetic treatment, or ii) the mixing of nanoparticles with a medium or body part followed by at least one step of the method according to the invention.

In still some other embodiment, the treatment, in particular with radiation, preferentially the warming step, induces or is characterized by a temperature increase of the body part, which is preferentially larger, by a factor of at least 1.001, 1.1, 1.2, 1.5, 2, 5, 10 or 10³, when the body part comprises nanoparticles than when the body part does not comprise nanoparticles.

In still some other embodiment, the treatment, in particular with radiation, preferentially the warming step, induces or is characterized by a temperature increase of the body part, which is preferentially smaller, by a factor of at least 1.001, 1.1, 1.2, 1.5, 2, 5, 10 or 10³, when the body part comprises nanoparticles than when the body part does not comprise nanoparticles.

In still some other embodiment, the treatment, in particular with a temperature adjuster, preferentially the cooling step, induces or is characterized by a temperature decrease of the body part, which is preferentially larger, by a factor of at least 1.001, 1.1, 1.2, 1.5, 2, 5, 10 or 10³, when the body part comprises nanoparticles than when the body part does not comprise nanoparticles.

In still some other embodiment, the treatment, in particular with a temperature adjuster, preferentially the cooling step, induces or is characterized by a temperature decrease of the body part, which is preferentially smaller, by a factor of at least 1.001, 1.1, 1.2, 1.5, 2, 5, 10 or 10³, when the body part comprises nanoparticles than when the body part does not comprise nanoparticles.

In an embodiment of the invention, the treatment is characterized by the production of radical or reactive species such as radical or reactive oxygen species (ROS) or radical or reactive nitrogen species (RNS). In some embodiment, ROS can be, originate from, or produce peroxides, superoxide, hydroxyl radical, singlet oxygen, and alpha-oxygen. In some other embodiment, RNS is/are, originate from, or produce nitric oxide, superoxide, peroxynitrite, peroxynitrous acid, nitrogen dioxide, hydroxyl radical, carbon dioxide, nitrosoperoxycarbonate, nitrogen dioxide, carbonate radical, dinitrogen trioxide.

In another embodiment of the invention, the treatment is characterized by the absence of production of radical or reactive species.

In some cases, the concentration of radical or reactive species produced during at least one step of the method is lower than 10⁵⁰, 10¹⁰, 10⁵, 10³, 10², 10, 5, 2, 1, 10⁻¹, 10⁻⁵, or 10-¹⁰ µM of radical or reactive species, preferentially comprised in the body part, preferentially per cm³ of body part.

In still another embodiment of the invention, radical or reactive species are produced during at least one step of the method according to the invention.

In some other embodiment, radical or reactive species are produced by the nanoparticles or body part under a variation in temperature, a temperature increase, or a temperature decrease, preferentially of the body part or nanoparticles, preferentially larger than 10⁻¹⁰, 10⁻⁵, 10⁻¹, 1, 5, 10 or 10³ °C, preferentially per second or minute, preferentially per cm³ of body part.

In some other embodiment, radical species are produced by the nanoparticles or body part under: i) no temperature increase, or no temperature decrease, preferentially of the body part or nanoparticles or ii) a variation in temperature, a temperature increase, or a temperature decrease, preferentially of the body part or nanoparticles, preferentially lower than 10¹⁰, 10⁵, 10³, 10², 50, 10, 5, 2, 1, 10⁻¹ or 10⁻⁵ °C.

In some other embodiment, the concentration of radical or reactive species produced during at least one step of the method is larger than 10⁻¹⁰, 10⁻⁵, 10⁻¹, 1, 5, 10, 10³, 10⁵, 10¹⁰ or 10⁵⁰ µM of radical or reactive species, preferentially comprised in the body part, preferentially per cm³ of body part.

In still another embodiment of the invention, the production of radical or reactive species, the temperature increase, and/or the temperature decrease, is responsible for the efficacy of the treatment according to the invention, for the destruction or damage of at least one cell, preferentially pathological cell, or body part, partly or fully.

In one embodiment of the invention, the treatment according to the invention leads to or is associated with: i), the destruction or damage of at least 1, 10, 10³, 10¹⁰ or 10⁵⁰ pathological cell(s), of a portion of the body part, or of the whole body part, or ii), the cure or healing of the body part.

In some embodiment, the treatment according to the invention can be combined with a heat therapy, such as hyperthermia or thermo-ablation, or with another treatment such as radiotherapy, chemotherapy, surgery, or immunotherapy.

In one embodiment of the invention, the destruction or damage of the body part is or is associated with: i), variations in sizes, thickness, or morphology of the body part, ii), conformation change such as a change from a three or two dimensional conformation to a two or one conformation or geometry change or denaturation of protein, lipid, DNA, RNA or biological material comprised in the body part.

In some embodiment, the destruction or damage or healing or detection of the body part is partial. It may occur for or within less than 99, 90, 80, 70, 50, 20, 10 or 5% in mass or volume of the body part.

In some other embodiment, the destruction or damage or healing or detection of the total. It may occur for or within more than 10⁻¹⁰, 10⁻⁵, 10⁻¹, 1, 5, 10, 50, 70, 90 or 99% in mass or volume of the body part.

In one embodiment of the invention, the treatment, also preferentially designated as the treatment of the body part, is the repair of the body part, the destruction or detection or healing or cure of the pathological site or of pathological cells such as tumor cells, preferentially comprised in the body part.

In one embodiment of the invention, the treatment according to the invention is carried out by cryotherapy, where cryotherapy preferentially consists in cooling the body part, where cooling can be the temperature decrease of the body part between the beginning and the end of the treatment by cryotherapy or during the treatment by cryotherapy.

In one embodiment of the invention, the treatment according to the invention is carried out by cryosurgery, where cryosurgery is preferentially a treatment by cryotherapy comprising a step of introducing a substance or equipment in the body part or a step of removing a substance or equipment from the body part.

In one embodiment of the invention, the treatment by cryosurgery can comprise, preferentially before, during or after step(s) a) to d) of the method according to the invention, the introduction/administration to/in the body part and/or the removal from the body part of: i) the nanoparticle, ii) the sensor as defined in the invention, iii) the temperature adjuster, iv) an equipment or substance used to warm up the body part, preferentially during the warming step, v) part of the body part.

In one embodiment of the invention, cryosurgery is carried out with the help of a catheter, syringe, utensil, substance, also designated as cryosurgery equipment, enabling the insertion/introduction/administration of an equipment/substance to/in the body part or the removal of an equipment/substance from the body part.

In some embodiment, the cryosurgery equipment is located in the body part or is in contact with the body part, or is located at a distance from the body part that is lower than 10³, 10², 10, 1, 10⁻¹, 10⁻³, 10⁻⁶ or 10⁻⁹ m (meter). This can be the case when the cryosurgery equipment is directly introduced in the body part.

In some other embodiment, the cryosurgery equipment is located outside of the body part or is not in contact with the body part or is located at a distance from the body part of more than 10⁻⁵⁰, 10⁻¹⁰, 10⁻⁹, 10⁻⁵, 10⁻³, 10⁻¹ or 1 m. This can be the case when the cryosurgery equipment is an imaging equipment or an equipment of electromagnetic radiation located at some distance from the body part.

In another embodiment of the invention, cryosurgery or cryotherapy is nano-cryosurgery or nano-cryotherapy, where nano-cryosurgery and nano-cryotherapy are types of cryosurgery and cryotherapy, respectively, in or during which nanoparticles are used, preferentially administered to/in the body part.

In some embodiment, nano-cryosurgery or nano-cryotherapy can enable to reach efficient treatment at a larger minimum temperature or with less side effects compared with cryosurgery or cryotherapy.

In one embodiment of the invention, the method or treatment or the treatment of the body part according to the invention is or corresponds to a cryotherapy, cryosurgery, nano-cryotherapy, or nano-cryosurgery treatment.

In still another embodiment of the invention, the treatment according to the invention comprises or corresponds to: i), the duration of the treatment or of at least one step of the treatment, ii) the initial temperature, iii) the cooling temperature, iv) the final temperature, v) the rate(s) at which the initial, cooling, and/or final temperature(s) is/are reached, vi) the use of an equipment or substance to reach the initial, cooling, and/or final temperature, and/or vii), the use of an equipment or substance to measure the temperature or ROS or NOS, preferentially of or produced by the body part or nanoparticle(s) during the treatment.

In another embodiment of the invention, cryotherapy occurs or takes place in the presence of at least 1, 10, 10³, 10⁵, 10¹⁰, 10⁵⁰ or 10¹⁰⁰ nanoparticle(s) or mg of nanoparticle(s), preferentially comprised in the body part, preferentially per cm³ of body part.

In another embodiment of the invention, the treatment according to the invention occurs or takes place in the presence of less than 10¹⁰⁰, 100⁵⁰, 10¹⁰, 10⁵, 10³, 10, 5, 2 or 1 nanoparticle(s) or mg of nanoparticle(s), preferentially comprised in the body part, preferentially per cm³ of body part.

### ADMINISTRATION STEP

The cryotherapy treatment according to the invention is a treatment comprising at least one step or administration step during or in which the nanoparticles are administered to the body part. The administration of the nanoparticles to/in the body part can correspond to the first step or administration step of the treatment.

In one embodiment of the invention, the nanoparticles are administered to the body part only once.

In one embodiment of the invention, the nanoparticles are administered to/in the body part more than 2, 5, 10 or 10³ times. In some embodiment, the administration of the nanoparticles to/in the body part is repeated several times when the nanoparticles leave the body part or are degraded in the body part during the treatment and/or the nanoparticles in the body part are not sufficiently efficient to destroy the body part or to reach the desired medical, therapeutic or diagnostic activity.

In one embodiment of the invention, the nanoparticles are administered to/in the body part less than 10¹⁰, 10⁵, 10³, 10, 5 or 2 time(s). In some embodiment, the administration of the nanoparticles to/in the body part is repeated a limited number of times when the nanoparticles remain in the body part or are not degraded in the body part during the treatment and/or the nanoparticles in the body part are sufficiently efficient to destroy the body part or to reach the desired medical, therapeutic or diagnostic activity.

In one embodiment of the invention, the nanoparticles are administered to or in the body part when they are directly administered to the body part or when they are administered close to the body part, preferentially less than 1, 10⁻¹, 10⁻², 10⁻³, 10⁻⁴, 10⁻⁵, 10⁻⁶ or 10⁻⁹ m away from the body part. In this case, the nanoparticles may not need to be transported or diffuse, for example in blood circulation, from the region or site where they are administered to the body part.

In another embodiment of the invention, the nanoparticles are administered to or in the body part, when they are indirectly administered to the body part or when they are administered far from the body part, preferentially more than 1, 10⁻¹, 10⁻², 10⁻³, 10⁻⁴, 10⁻⁵, 10⁻⁶ or 10⁻⁹ m away from the body part. In this case, the nanoparticles may be transported or diffuse from the region or site where they are administered to the body part.

In still another embodiment of the invention, administering nanoparticles to or in the body part comprises the steps of: i), localizing or having localized nanoparticles in the body part, ii) having nanoparticles diffuse or be transported to the body part, iii) transport nanoparticles to the body part, or iv) imaging nanoparticles, preferentially to verify that nanoparticles reach or are in the body part or that they are transported or diffusing towards the body part or that they are distributed or localized in the body part.

In another embodiment of the invention, the nanoparticles are administered to or in the body part when they occupy more than 10⁻⁹, 10⁻⁷, 10⁻⁵, 10⁻³, 1, 10, 25, 50 or 75 %, preferentially by mass or volume, of the body part, where this percentage can be the ratio between the volume of the region occupied by the nanoparticles in the body part or nanoparticle region and the volume of the body part. This occupation can correspond to that measured 10⁻⁵, 10⁻³, 10⁻¹, 1, 10, 10³ or 10⁵ minute(s) following nanoparticle administration.

In another embodiment of the invention, the nanoparticles are administered to or in the body part following at least one of the following administration routes: local, enteral, gastrointestinal, parenteral, topical, oral, inhalation, intramuscular, subcutaneous, intra-tumor, in an organ, in a vein, in arteries, in blood, or in tissue.

In another embodiment of the invention, the nanoparticles are in suspension, where the concentration of the nanoparticle suspension is lower than 10¹⁰, 10⁵, 10³, 500, 200, 100, 50, 20, 10, 5, 2, 1, 10⁻¹, 10⁻³ or 10⁻⁵ mg of nanoparticles or of at least one chemical element comprised in nanoparticles, preferentially metallic, per ml or cm³ of suspension. In some embodiment, the concentration of the nanoparticle suspension is lower than the threshold concentration, above which the nanoparticles are not soluble or dispersible in suspension or in the medium of the suspension. In some embodiment, the nanoparticle concentration is sufficiently low to avoid side effects.

In another embodiment of the invention, the concentration of the nanoparticle suspension, preferentially of the administered nanoparticle suspension, is larger than 10⁻⁵⁰, 10⁻³⁰, 10⁻²⁰, 10⁻¹⁰, 10⁻⁵, 10⁻¹, 1, 5, 10, 100, 250, 500, 10³ or 10⁵ mg of nanoparticles or of at least one chemical element comprised in nanoparticles, preferentially metallic, per ml or cm³ of suspension. In some embodiment, the nanoparticle concentration is sufficiently large to be efficient.

In another embodiment of the invention, the concentration of the nanoparticle suspension, preferentially of the administered nanoparticle suspension, is between 10⁻⁵⁰ and 10⁵⁰, 10⁻¹⁰ and 10¹⁰, 10⁻⁵ and 10⁵, 10⁻³ and 10³, or between 10⁻¹ and 10³ mg of nanoparticles or of at least one chemical element comprised in nanoparticles, preferentially metallic, per ml or cm³ of suspension.

In another embodiment of the invention, the nanoparticles are administered, preferentially in/to the body part, at a rate or speed that is larger than 10⁻¹⁰⁰, 10⁻⁵⁰, 10⁻²⁰, 10⁻¹⁰, 10⁻⁵, 10⁻³, 10⁻¹, 1, 5, 10, 50, 100 or 10³ mg of nanoparticles or of at least one chemical element comprised in nanoparticles, preferentially metallic, per second, preferentially per second of administration time. In some embodiment, a fast administration is necessary, for example when the administration needs to be carried out under anesthesia and the time of anesthesia needs to be short.

In another embodiment of the invention, the nanoparticles are administered, preferentially in/to the body part, at a rate or speed that is smaller than 10¹⁰⁰, 10⁵⁰, 10²⁰, 10¹⁰, 10⁵, 10³, 10¹, 5, 1, 10⁻³ or 10⁻⁵ mg of nanoparticles or of at least one chemical element comprised in nanoparticles, preferentially metallic, per second, preferentially per second of administration time. In some embodiment, a slow administration is necessary, for example when the pressure in the body part is large pushing the nanoparticles outside of the body part following administration.

In another embodiment of the invention, the nanoparticles are administered, preferentially in/to the body part, at a rate or speed that is between 10⁻¹⁰⁰ and 10¹⁰⁰, 10⁻⁵⁰ and 10⁵⁰, 10⁻²⁰ and 10²⁰, 10⁻¹⁰ and 10¹⁰, 10⁻⁵ and 10⁵, 10⁻⁵ and 10³, or between 10⁻³ and 10² mg of nanoparticles or of at least one chemical element comprised in nanoparticles, preferentially metallic, per second, preferentially per second of administration time.

In one embodiment of the invention, the equipment or substance used for the administration of the nanoparticle, such as a syringe or catheter or medium in which the nanoparticles are suspended, is such that it enables reaching a sufficient nanoparticle concentration in the body part for the method or treatment according to the invention to be efficient.

### REPETITION/DURATION OF THE STEPS, CYCLES AND SESSIONS OF THE METHOD

Surprisingly, the present inventors also discovered that a sequential cryotherapy according to the present invention further improved cellular destruction.

In one embodiment, the invention relates to nanoparticles for use according to the invention or to the method according to the invention, wherein the succession of steps of at least the cooling step and the warming step, preferably the cooling step, the maintaining step and the warming step is repeated more than once, preferably more than 2, 3, 5, 6, 10, or 10³ time(s), more preferably more than 6 times, even more preferably more than 3 times. In some embodiment, an important number of steps is carried out to reach the medical, cosmetic, therapeutic or cosmetic effect or activity.

In one embodiment, the succession of steps is repeated less than 10¹⁰, 10⁵, 10³, 100, 75, 50, 20, 10, 5, or 2 times. In some embodiment, a limited number of steps is carried out to avoid side effects that can be associated with a too large number of steps.

In one embodiment, the succession of steps is repeated between 1 and 10⁵⁰, 1 and 10¹⁰, 1 and 10⁵, 1 and 10³, 1 and 10², 1 and 50, or between 1 and 20 times.

In one embodiment of the invention, a cycle comprises: i) the administration step, the cooling step, the maintaining step, and the warming step, ii) the cooling step, the maintaining step, and the warming step, and/or iii) the cooling step and the warming step.

In one embodiment of the invention, the administration step is carried out only once or a limited number of times, preferentially less than 10⁵, 10³, 100, 50, 20, 10, 5 or 2 times, preferentially to avoid side effects or difficulties associated with a large number of nanoparticle administrations.

In one embodiment of the invention, the maintaining step is not carried out or is carried out for a short period of time, preferentially shorter than 10⁵, 10³, 100, 50, 20, 10, 5, 2, 1 or 10⁻¹ second(s), preferentially to avoid side effects due to a too long maintaining step.

In one embodiment, a cycle is repeated more than 1, 2, 3, 5, 6, 10, or 10³ times, preferably more than 6 times, most preferably more than 3 times.

In one embodiment, a cycle is repeated less than 10⁵⁰, 10²⁰, 10¹⁰, 10⁵, 10³, 100, 75, 50, 20, 10, 5 or 2 times, preferably less than 10³ times, most preferably less than 100 times

In one embodiment, a cycle is repeated between 1 and 10⁵⁰, 1 and 10²⁰, 1 and 10¹⁰, 1 and 10⁵, 1 and 10³, or between 1 and 100 time(s).

In one embodiment of the invention, several cycles correspond to a session and a session preferably comprises more than 1, 2, 3, 5, 10, 10², 10³, 10⁵ or 10¹⁰ cycles.

In another embodiment of the invention, a session comprises less than 10¹⁰, 10⁵, 10³, 10², 10, 5, 3 or 2 cycles.

In still another embodiment of the invention, a session comprises between 1 and 10⁵⁰, 1 and 10¹⁰, 1 and 10³, 1 and 10², or between 1 and 50 cycles.

In still another embodiment of the invention, the duration of at least one step, the duration between two steps, the duration of at least one cycle, the duration between two cycles, the duration of at least one session, and/or the duration between two sessions is/are longer than 10⁻⁵⁰, 10⁻¹⁰, 10⁻⁵, 10⁻¹, 1, 5, 10 or 100 second(s).

In still another embodiment of the invention, the duration of at least one step, the duration between two steps, the duration of at least one cycle, the duration between two cycles, the duration of at least one session, and/or the duration between two sessions is/are shorter than 10¹⁰⁰, 10⁵⁰, 10²⁰, 10¹⁰, 10⁵, 10³, 10², 50, 20, 10, 5, 2 or 1 second(s).

In still another embodiment of the invention, the duration of at least one step, the duration between two steps, the duration of at least one cycle, the duration between two cycles, the duration of at least one session, and/or the duration between two sessions is between 10⁻⁵⁰ and 10¹⁰⁰, 10⁻¹⁰ and 10⁵⁰, 10⁻⁵ and 10¹⁰, 10⁻³ and 10⁵, 10⁻¹ and 10³, or between 10⁻¹ and 100 seconds.

In still another embodiment, the duration of at least one cycle is longer than the duration of at least one step, or the duration of at least one session is longer than the duration of at least one cycle or of at least one step, preferentially by a factor of at least 0, 0.5, 1, 1.1, 1.5, 2, 5, 10, 10², 10³, 10⁵ or 10¹⁰.

In still another embodiment, the duration separating two sessions is longer than the duration separating two steps and/or than the duration separating two cycles, preferentially by a factor of at least 0, 0.5, 1, 1.1, 1.5, 2, 5, 10, 10², 10³, 10⁵ or 10¹⁰.

In still another embodiment, the duration separating two steps is shorter than the duration of at least one step, preferentially by a factor of at least 0, 0.5, 1, 1.1, 1.5, 2, 5, 10, 10², 10³, 10⁵ or 10¹⁰.

In still another embodiment, the duration separating two cycles is shorter than the duration of at least one cycle, preferentially by a factor of at least 0, 0.5, 1, 1.1, 1.5, 2, 5, 10, 10², 10³, 10⁵ or 10¹⁰.

### CRYOTHERAPY

In one embodiment of the invention, cryotherapy is a therapy or medical treatment comprising at least one step in or during which the temperature of the body part is decreased, preferentially from an initial temperature to a cooling temperature. The decrease of the temperature of the body part, preferentially from an initial temperature to a cooling temperature, can correspond to the second step or the cooling step of the treatment.

In one embodiment of the invention, the method or treatment according to the invention, also designated as cryotherapy, results in or is associated with a medical, cosmetic, therapeutic, or diagnostic effect or activity, where such effect or activity can be the destruction, healing, cure, disappearance, attraction, movement, change in color or appearance, production, of compounds, substances, nanoparticles, preferentially occurring in the body part or nanoparticle region.

The step b) of cooling the body part comprising the nanoparticles by decreasing the temperature of body part from an initial temperature of said body part down to a cooling temperature of the body part, which is lower than the initial temperature, is designated as the cooling step.

In one embodiment of the invention, the cooling step is the cooling of the body part comprising the nanoparticles. In some embodiment, it can mean, be associated with, or consist in: i) cooling or decreasing the temperature of the body part from an initial temperature to a cooling temperature, or ii) cooling or decreasing the temperature of the body part to a cooling temperature. In some embodiment, cooling or decreasing the temperature of the body part from an initial temperature to a cooling temperature can be expressed more simply without any loss in meaning as cooling or decreasing the temperature of the body part.

In one embodiment of the invention, at least one of the step(s) of the method, preferentially the cooling step, is a step at which a medical, cosmetic, diagnostic, or therapeutic effect occurs.

In one embodiment of the invention, the cooling temperature reached during the treatment is the temperature at which a medical, cosmetic, diagnostic, or therapeutic effect occurs.

In one embodiment of the invention, the temperature gradient occurring during the cooling step and/or warming step is the temperature interval at which a medical, cosmetic, diagnostic, or therapeutic effect occurs.

In one embodiment of the invention, the medical, cosmetic, diagnostic, or therapeutic effect is or is associated with a change in color or appearance of the body part or the destruction or damages of the pathological cells, or the healing, partly or fully, of the body part.

In one embodiment of the invention, the medical, cosmetic, diagnostic, or therapeutic effect occurs, most preferably predominantly, at the cooling temperature or during or at the end of the cooling step, or within the temperature gradients of the cooling and/or warming step(s).

In one embodiment of the invention, the medical, cosmetic, diagnostic, or therapeutic effect does not occur at the initial and/or final temperature(s).

In one embodiment of the invention, the medical, cosmetic, diagnostic, or therapeutic effect is more pronounced when the temperature is decreased from the initial temperature to the cooling temperature during the cooling step and/or when the temperature is increased from the cooling temperature to the final temperature during the warming step than when the temperature is maintained at the cooling temperature during the maintaining step.

In one embodiment of the invention, the medical, cosmetic, diagnostic, or therapeutic effect is more pronounced at the cooling temperature than at the initial or final temperature.

In one embodiment of the invention, the initial temperature, preferentially of the body part or nanoparticle, is the temperature occurring: i), before, during, or at the end of the administration step, or ii) at the beginning of the cooling step. The situations i) and ii) can correspond to temperatures, T, preferentially of the body part or nanoparticles, that have decreased by less than 100, 75, 50, 25, 10, 5, 2 or 1%, where this percentage can be T/Tᵢ, or by less than 10⁵, 10³, 100, 10, 5, 2, 1 or 10⁻¹ °C following at least one step of the method according to the invention.

In another embodiment of the invention, the initial temperature, preferentially of the body part or nanoparticles, is: i), the maximum temperature reached during the treatment or method according to the invention ii), the temperature reached when no equipment or substance is used to adjust the temperature of the body part or before an equipment or substance is used to adjust the temperature of the body part, or iii), the temperature reached when a thermal equilibrium of the body part is established with its surrounding environment or with the living organism or individual comprising the body part, preferentially in the absence of equipment or substance used to adjust the temperature of the body part.

In some embodiment, the thermal equilibrium of the body part with its surrounding environment occurs when the temperature of the body part does not vary by more than 10⁻⁵, 10⁻³, 10⁻¹, 1, 5, 10, 10² or 10³ °C, preferentially °C per second.

In one aspect, the invention relates to nanoparticle for use according to the invention, wherein the initial temperature and/or the final temperature is/are physiological temperature(s).

As used herein, physiological temperatures relate to normal body temperature, also known as normothermia or euthermia temperatures of the body part or of whole organism of an individual or of part of an individual.

In some embodiment, physiological temperatures are temperatures of a healthy individual, or of an individual with fever, or of an individual with a maximal body temperature of 43 °C. In some embodiment, the physiological temperature can be 37 ± 6 °C. In some embodiment, physiological temperatures can be significantly larger than 37, 40, 45, 50, 60, 70, 80, 90 or 100 °C, for example when the individual is treated by a method of hyperthermia or thermo-ablation or when the individual is different from a human, for example an extremophile that can live in conditions of high temperature, larger than 100 °C. In some embodiment, physiological temperature can be significantly smaller than 37, 30, 20, 10, 5, 0, -10, -20, -50, -100 °C, for example when the individual is suffering from hypothermia or when the individual is different from a human, for example an extremophile that can live in conditions of low temperatures, lower than 0 °C.

In one embodiment, the initial temperature is the temperature or the corporal temperature of a healthy individual, most preferentially between 36 °C et 37.8 °C. In some embodiment, the initial temperature can be lower than 36 °C, preferentially below 30, 20 or 10 °C when the individual is a state of hypothermia. In some other embodiment, the initial temperature can be above 37.8 °C, preferentially above 38, 39, 40, 41 °C when the individual has fever. In still some other embodiment, the initial temperature can be above 38, 40, 43, 45, 50 or 55 °C, for example when the individual is treated by hyperthermia such as whole-body hyperthermia. In still some other embodiment, the initial temperature is above 55, 60, 65, 70, 80, 90 or 100 °C, for example when the individual is treated by thermoablation or high intensity focused ultrasound.

In some other embodiment, the cooling temperature, which can be designated as T_{c}, is the temperature of the body part or individual at the end of the cooling step or during the maintaining step. In some embodiment, the temperature at the end of the cooling step, T_{ECS}, has decreased by at least 10⁻¹, 1, 5, 10, 25, 50, 75, 80, 90, 95 or 99% or by at least 10⁻⁵⁰, 10⁻¹⁰, 10⁻¹, 1, 5 or 10 °C compared with the temperature at the beginning of the cooling step, T_{BCS}, where this percentage can be equal to |T_{ECS}/T_{BCS}| or |T_{ECS}-T_{BCS}|/|T_{BCS}|, where the symbols I I designate the absolute value.

In some embodiment of the invention, T_{BCS}, is the temperature occurring at least 10⁻⁵⁰, 10⁻³, 10⁻¹, 1, 5, 10, 10² or 10³ second(s) following the administration step or following the use or switching on of the temperature adjuster.

In some embodiment of the invention, T_{ECS}, is the temperature occurring at least 10⁻⁵⁰, 10⁻³, 10⁻¹, 1, 5, 10, 10² or 10³ second(s) before the beginning of the warming step or before the end of use or switching off of the temperature adjuster.

In some embodiment of the invention, T_{ECS} is separated from T_{BCS} by a lapse of time of at least 10⁻⁵⁰, 10⁻³, 10⁻¹, 1, 5, 10, 10² or 10³ second(s).

In another embodiment of the invention, the cooling temperature is the minimum temperature reached during the treatment.

In another embodiment of the invention, the cooling temperature is a temperature that is lower than the initial temperature by a quantity or variation of temperature of the second step, ΔT_{2S} = |T_{C} - Tᵢ|, which is larger than 1, 2, 5, 10, 20, 50, 100 or 200 °C.

In another embodiment of the invention, the cooling temperature is a temperature that is lower than the initial temperature by a quantity, factor or percentage |T_{C} - Tᵢ|/|Tᵢ|, and/or |T_{C}|/|Tᵢ|, which is/are larger than 10⁻⁵, 10⁻³, 10⁻¹, 0, 1, 5, 10, 25, 50, 75, 80, 90, 95, 99, 100, 10³ or 10⁵.

In some embodiment of the invention, a low value of the cooling temperature is desired when one wishes to form ice-balls or to limit the number of cycles of the method according to the invention.

In another embodiment of the invention, the cooling temperature is a temperature that is lower than the initial temperature by a quantity ΔT = |T_{C} - Tᵢ|, which is lower than 10³, 100, 50, 20, 10, 5, 2 or 1 °C.

In another embodiment of the invention, the cooling temperature is a temperature that is lower than the initial temperature by a quantity, factor or percentage |T_{C} - Tᵢ|/|Tᵢ| and/or |T_{C}|/|Tᵢ|, which is/are lower than 10⁵, 10³, 100, 99, 90, 75, 50, 20, 10, 0, 10⁻¹, 10⁻³ or 10⁻⁵.

In some embodiment of the invention, a large value of the cooling temperature, preferentially above 0°C, is desired when one wishes to avoid the side effects associated with a too low cooling temperature such as those associated with the formation of ice-balls.

In another embodiment of the invention, the cooling temperature is a temperature that is lower than the initial temperature by a quantity or variation of temperature of the second step, ΔT_{2S} = |T_{C} - Tᵢ|, which is between 10⁻¹⁰ and 10¹⁰, 10⁻⁵ and 10⁵, 10⁻³ and 10³, or 10⁻¹ and 10 °C.

In another embodiment of the invention, the cooling temperature is a temperature that is lower than the initial temperature by a quantity, factor or percentage ΔT_{2S}/Tᵢ = |T_{C} - Tᵢ|/|Tᵢ| and/or |T_{C}/Tᵢ|, which is/are between 10⁻⁵⁰ and 10⁵⁰, 10⁻¹⁰ and 10¹⁰, 10⁻⁵ and 10⁵, 10⁻³ and 10³, or 10⁻¹ and 10.

In one embodiment of the invention, the initial temperature, cooling temperature and/or final temperature is/are larger than -273, -150, -100, -50, -20, -10, -5, -2, -1, 0, 1, 5, 10, 20, 50, 70 or 100 °C.

In another embodiment of the invention, the initial temperature, cooling temperature and/or final temperature is/are lower than 10³, 10², 70, 50, 20, 10, 5, 2 or 1 °C.

In still another embodiment of the invention, the initial temperature, cooling temperature and/or final temperature is/are between -273 and 10³, -50 and 100, -20 and 20, -20 and 10, -10 and 10, -5 and 5, -2 and 5, -1 and 5, or between 0 and 5 °C.

In still another embodiment of the invention, the difference between the cooling and initial temperature or the difference between the cooling temperature is lower than the difference in temperature between the maximum temperature at which an organism can live or at which an enzyme or protein or DNA strand can be non-denatured or non-destroyed and 0 degree.

In one embodiment of the invention, cryotherapy is a therapy or medical treatment comprising at least one step during or in which the temperature of the body part is maintained at the cooling temperature. The step in or during which the body part is maintained at the cooling temperature for a duration of time can correspond to the third step or maintaining step of the treatment according to the invention.

In one embodiment of the invention, there is no lapse of time or duration during the method, preferentially during the maintaining step, which is preferentially longer than 10⁻⁵⁰, 10⁻²⁰, 10⁻¹⁰, 10⁻⁵, 10⁻³, 10⁻¹, 1, 5, 10, 10³ or 10⁵ second(s), during or in which the temperature varies by less than 10¹⁰, 10⁵, 10³, 100, 75, 50, 25, 10, 5, 2, 1 or 10⁻¹ °C or by less than 100, 75, 50, 25, 10, 5, 2, 1 or 10⁻¹%, where this percentage can be equal to |ΔT/Tₘᵢₙ| or |ΔT/Tₘₐₓ|, where ΔT, Tₘᵢₙ and Tₘₐₓ are the temperature variation, minimum and maximum temperatures reached during this lapse of time or duration.

In one embodiment of the invention, the duration of the maintaining step is shorter than 10⁵⁰, 10³⁰, 10²⁰, 10¹⁰, 10⁵, 10³, 100, 75, 50, 25, 10, 5, 2, 1, 10⁻¹ or 10⁻³ second(s).

In one embodiment of the invention, the temperature of the body part is maintained at the cooling temperature, when the temperature of the body part differs by less than 100, 90, 80, 70, 60, 50, 20, 10, 5, 1, 10⁻¹ % from the cooling temperature, where this percentage can be the absolute values of (T_{BP}-T_{CT})/T_{CT}, T_{CT}/T_{BP} or T_{BP}/T_{CT}, where T_{BP} and T_{CT} are the temperature of the body part and the cooling temperature, respectively.

In one embodiment of the invention, one can avoid that the temperature is maintained at the cooling temperature by : i) stop using or switching off the temperature adjuster when or before the cooling temperature is reached, or ii) let the body part warming up, for example by being exposed to the body part, blood, tissue, ambient air or a medium that is maintained at the cooling temperature or below the cooling temperature.

In one embodiment of the invention, cryotherapy is a therapy or medical treatment comprising at least one step during or in which the temperature of the body part is increased, preferentially from the cooling temperature to a final temperature. The increase of the temperature of the body part, preferentially from the cooling temperature to a final temperature, can correspond to the fourth step or warming step of the treatment.

The step d) of warming the body part comprising the nanoparticles by increasing the temperature of the body part from the cooling temperature of the body part to a final temperature of the body part, which is above the cooling temperature, can be designated as the warming step.

In one embodiment of the invention, the warming step consists in warming the body part comprising the nanoparticles by increasing the temperature of the body part from the cooling temperature to the final temperature.

In one embodiment of the invention, the final temperature of the body part is the temperature measured or occurring at the end of the cryotherapy treatment or at the end of the method according to the invention or at the end of the warming step.

In one embodiment of the invention, the medical, cosmetic, diagnostic, or therapeutic effect occurs, most preferably predominantly, during or at the beginning of the warming step.

In some embodiment, the final temperature, which can be designated as T_{f}, is the temperature of the body part or individual at the end of the treatment. In some embodiment, the end of the treatment is reached: i) at the end of the warming step, ii) at least 10⁻⁵⁰, 10⁻²⁰, 10⁻¹⁰, 10⁻⁵, 10⁻¹, 1, 5, 10 or 10³ second(s) following the beginning of the treatment or the switching on or use of the temperature adjuster, iii) when or after the temperature of the body part is stabilized.

In one embodiment of the invention, the temperature of the body part is stabilized when the variation of temperature of the body part, ΔT, within a lapse of time, δt, which is preferentially longer than 10⁻⁵⁰, 10⁻²⁰, 10⁻¹⁰, 10⁻⁵, 10⁻³, 10⁻¹, 1, 5, 10, 10³ or 10⁵ second(s), is smaller than 10¹⁰, 10⁵, 10³, 100, 75, 50, 25, 10, 5, 2, 1 or 10⁻¹ °C or is smaller by a factor of at least 0, 0.5, 1, 1.1, 1.5, 2, 5, 10, 10³ or 10⁵ than the temperature variation of the cooling and/or warming step(s).

In one embodiment, the final temperature T_{f} has the same value as the initial temperature Tᵢ or differs from the initial temperature by less than:
i) 10⁻⁵⁰, 10⁻¹⁰, 10⁻¹, 0, 1, 5, 10, 10³, 10⁵, 10¹⁰, 10²⁰ or 10⁵⁰ °C, and/or
ii) 10⁻⁵⁰, 10⁻¹⁰, 10⁻¹, 1, 5, 10, 25, 50, 75, 90, 99 or 100%, where this percentage can be equal to |T_{f}/Tᵢ|, |Tᵢ/T_{f}|, |T_{f}-Tᵢ|/|Tᵢ| or |T_{f}-Tᵢ|/|T_{f}|.

In another embodiment of the invention, the final temperature, which is preferentially designated as T_{f}, is a temperature that is larger than the cooling temperature by a quantity or variation of temperature of the fourth step, ΔT_{4T} = |T_{f} - T_{C}|, which is larger than 10⁻¹⁰, 10⁻¹, 1, 2, 5, 10, 20, 50, 100 or 200 °C.

In another embodiment of the invention, the final temperature is a temperature that is larger than the cooling temperature by a quantity, factor or percentage T_{f}/T_{c}, ΔT_{4T}/_{c}, and/or |T_{f}/T_{c}|, which is/are larger than 10⁻⁵, 10⁻³, 10⁻¹, 0, 1, 5, 10, 25, 50, 75, 80, 90, 95, 99, 100, 10³ or 10⁵.

In one embodiment of the invention, the final temperature is much above the cooling temperature by: i) having a large temperature gradient during the warming step, ii) having a low cooling temperature, and/or iii) having a high final temperature, where the values i) to iii) preferentially enable reaching the desired medical or cosmetic or therapeutic or diagnostic.

In another embodiment of the invention, the final temperature is a temperature that is larger than the cooling temperature by a quantity or temperature variation ΔT_{4S} = |T_{f} - T_{c}|, which is lower than 10³, 100, 50, 20, 10, 5, 2 or 1 °C.

In another embodiment of the invention, the final temperature is a temperature that is larger than the cooling temperature by a quantity, factor or percentage ΔT_{4S}/T_{c} and/or |T_{f}/T_{c}|, which is/are lower than 10⁵, 10³, 100, 99, 90, 75, 50, 20, 10, 0, 10⁻¹, 10⁻³ or 10⁻⁵.

In one embodiment of the invention, the different in temperature between the final temperature and the cooling temperature is small when : i) the warming step is characterized by a small temperature gradient, ii) the number of cycles is large, preferentially within a short lapse of time, iii) the small temperature gradient is compensated by a large number of cycles, where the points i) to iii) preferentially enable reaching the medical or cosmetic or therapeutic or diagnostic.

In another embodiment of the invention, the final temperature is a temperature that is larger than the cooling temperature by a quantity ΔT_{4S} = |T_{f} - T_{c}|, which is between 10⁻¹⁰ and 10¹⁰, 10⁻⁵ and 10⁵, 10⁻³ and 10³, or 10⁻¹ and 10 °C.

In another embodiment of the invention, the final temperature is a temperature that is larger than the cooling temperature by a quantity, factor or percentage |T_{f} - T_{c}|/|T_{c}| and/or |T_{f}/T_{c}|, which is/are between 10⁻⁵⁰ and 10⁵⁰, between 10⁻¹⁰ and 10¹⁰, between 10⁻⁵ and 10⁵, between 10⁻³ and 10³, or between 10⁻¹ and 10.

In one embodiment of the invention, the initial temperature is the temperature measured at the beginning of the cooling step.

In one embodiment of the invention, the cooling temperature is the temperature measured at the end of the cooling step, during the maintaining step, or at the beginning of the warming step.

In another embodiment of the invention, the final temperature is the temperature measured at the end of the warming step.

### RADIATION

In one embodiment of the invention, a radiation is applied on the body part or nanoparticle or the body part or nanoparticle is exposed to a radiation, preferentially during at least one step of the method, most preferentially during the warming step of the method.

In some embodiment, the radiation can be an electromagnetic radiation, light, monochromatic or polychromatic, a laser, a magnetic field, preferentially an alternating magnetic field, acoustic wave, preferentially an infra-sound, an ultrasound, or a hyper sound.

In some embodiment, the radiation can be thermal, and preferentially induce a temperature increase of the body part or nanoparticles, preferentially of more than 10⁻¹⁰, 10⁻⁵, 10⁻¹, 1, 2, 5, 10, 10³, 10⁵ or 10¹⁰ °C, preferentially per second or minute, preferentially per cm³ of body part.

In some other embodiment, the radiation can be non-thermal, and preferentially not induce a temperature increase, or induce a temperature increase of less than 10¹⁰, 10⁵, 10³, 10, 5, 2, 1, 10⁻¹ or 10⁻⁵ °C, preferentially per second or minute, preferentially per cm³ of body part.

### NON-PREDOMINANT ICE-BALL CRYOTHERAPY

According to the present invention, the cooling temperature of the body part is:
- above the freezing temperature of the body part or above 5, 0 or -5°C, preferably above -5 or 0°C, more preferably above 0°C ; and/or
- above a threshold temperature, where the threshold temperature has at least one property selected from the group consisting of:
   i) above the threshold temperature, the body part comprises a quantity of ice-balls smaller than the quantity of nanoparticles;
   ii) above the threshold temperature, the volume occupied by ice-balls in the body part is smaller than the volume occupied by the nanoparticles in the body part;
   iii) below the threshold temperature, the body part comprises a quantity of ice-balls larger than the quantity of nanoparticles;
      and
   iv) below the threshold temperature, the volume occupied by ice-ball in the body part is larger than the volume occupied by the nanoparticles in the body part.

Preferably, in order to avoid any ice-balls formation, the cooling temperature of the body part according to the present invention is above 0°C which is the freezing point of water. However, depending of the body part of the individual, the type of individual treated (mammal, plant, bacteria...) and due notably to the electrolyte content of the cells and extracellular fluid, the freezing point can be below 0°C. For instance, the skin surface reportedly freezes from -3.7 to -4.8°C.
As such, in one embodiment, the cooling temperature of the body part according to the present invention can be above 10, 5, 2, 1, 0, -5, -10 or -20°C, preferably above -5°C, most preferably above 0°C.

In one embodiment, the cooling temperature of the body part according to the present invention is between 10 and -20°C, preferably between 10 and -10°C, more preferably between 5 and -5°C, even more preferably between 2 and -2°C, even more preferably between 1 °C and -1 °C, even more preferably about 0°C.

In one embodiment, ice-balls are nanoparticles that are cooled down below the freezing temperature of the body part, 0 °C, or the threshold temperature.

In another embodiment, ice-balls are nanoparticles and additional substances such as water or biological material surrounding the nanoparticles that are cooled down below the freezing temperature of the body part, 0 °C, or the threshold temperature.

In another embodiment, the size of the ice-balls is larger than the size of the nanoparticles, preferentially by: i) at least 0, 0.5, 1, 5, 10 or 10³ nm, or ii) by a factor of at least 0, 0.5, 1, 1.1, 1.5, 2, 5, 10, 10³ or 10⁵. This may be the case when ice-balls surround or comprise nanoparticles.

In another embodiment, the size of the ice-balls is smaller than the size of the nanoparticles, preferentially by: i) at least 0, 0.5, 1, 5, 10 or 10³ nm, or ii) by a factor of at least 0, 0.5, 1, 1.1, 1.5, 2, 5, 10, 10³ or 10⁵. This may be the case when ice-balls do not surround or do not comprise nanoparticles.

In another embodiment, below the threshold temperature, the body part comprises ice-balls with at least one property selected from the group consisting of:
i) a quantity of ice-balls that is smaller than 1, 5, 10, 10³, 10⁵ or 10⁵⁰ ice-ball(s) or ice-ball(s) per cm³ of body part;
ii) a quantity of ice-balls that is smaller than the quantity of nanoparticles, preferentially by: i) at least 0, 0.5, 1, 5, 10 or 10³ ice-ball(s), or ii) by a factor of at least 0, 0.5, 1, 1.1, 1.5, 2, 5, 10, 10³ or 10⁵;
iii) a volume occupied by ice-balls in the body part that is smaller than 10¹⁰, 10⁵, 10³, 10, 10⁻¹, 10⁻³, 10⁻⁶ or 10⁻⁹ cm3, preferentially as measured per cm³ of body part;
iv) a volume occupied by ice-balls in the body part that is smaller than the volume occupied by the nanoparticles in the body part, preferentially by: i) at least 0, 10⁻⁵⁰, 10⁻²⁰, 10⁻¹⁰, 10⁻³, 1, 5, 10 or 10³ cm³, or ii) by a factor of at least 0, 0.5, 1, 1.1, 1.5, 2, 5, 10, 10³ or 10⁵;
v) a size or diameter of at least one ice-ball that is smaller than 10⁵⁰, 10¹⁰, 10⁵, 10³, 1, 10⁻³ or 10⁻⁵ nm;
   and
vi) a size or diameter of at least one ice-ball that is smaller than the size of at least one nanoparticle, preferentially by: i) at least 0, 10⁻⁵⁰, 10⁻²⁰, 10⁻¹⁰, 10⁻³, 1, 5, 10, 10³ or 10⁶ nm, or ii) by a factor of at least 0, 0.5, 1, 1.1, 1.5, 2, 5, 10, 10³ or 10⁵.

In another embodiment, above the threshold temperature, the body part comprises ice-balls with at least one property selected from the group consisting of:
i) a quantity of ice-balls that is larger than 1, 5, 10, 10³, 10⁵ or 10⁵⁰ ice-ball(s) or ice-ball(s) per cm³ of body part;
ii) a quantity of ice-balls that is larger than the quantity of nanoparticles, preferentially by: i) at least 0, 0.5, 1, 5, 10 or 10³ ice-ball(s), or ii) by a factor of at least 0, 0.5, 1, 1.1, 1.5, 2, 5, 10, 10³ or 10⁵;
iii) a volume occupied by ice-balls in the body part that is larger than 10⁻⁵⁰, 10⁻¹⁰, 10⁻⁵, 10⁻¹, 1, 2, 5, 10, 10³ or 10⁶ cm³, preferentially as measured per cm³ of body part;
iv) a volume occupied by ice-balls in the body part that is larger than the volume occupied by the nanoparticles in the body part, preferentially by: i) at least 0, 10⁻⁵⁰, 10⁻²⁰, 10⁻¹⁰, 10⁻³, 1, 5, 10 or 10³ cm³, or ii) by a factor of at least 0, 0.5, 1, 1.1, 1.5, 2, 5, 10, 10³ or 10⁵;
v) a size or diameter of at least one ice-ball that is larger than 10⁻³, 1, 5, 10, 10³, 10⁵ or 10⁵⁰ nm;
   and
vi) a size or diameter of at least one ice-ball that is larger than the size of at least one nanoparticle, preferentially by: i) at least 0, 10⁻⁵⁰, 10⁻²⁰, 10⁻¹⁰, 10⁻³, 1, 5, 10, 10³ or 10⁶ nm, or ii) by a factor of at least 0, 0.5, 1, 1.1, 1.5, 2, 5, 10, 10³ or 10⁵.

In one embodiment of the invention, temperatures above that threshold temperature do not result predominantly in ice-balls formations and temperatures below that threshold temperature result predominantly in ice-balls formations.

In some embodiment, temperatures above said threshold temperature do not result predominantly in ice-balls formations if ice-balls are present in less than 50, 20, 5, 2 or 1 percent, preferentially in mass or volume, of the body part.

In some embodiment, temperatures below said threshold temperature result predominantly in ice-balls formations if ice-balls are present in more than about 50, 70, 80, 90 percent or present in about 100 percent, preferentially in mass or volume, of the body part.

the temperature is measured during at least one step of the method or treatment according to the invention.

As used herein, the ice-ball temperature is the temperature below which iceball(s) form(s) in the body part. Preferentially, below the ice-ball temperature, iceball(s) is/are formed. Preferentially, above the ice-ball temperature, iceball(s) is/are not formed.

In some embodiment of the invention, the ice-ball temperature is a threshold temperature that is such that:
- above the ice-ball temperature, or more than 10⁻³, 10⁻¹, 1, 5 or 10 °C above the ice-ball temperature, less than 10¹⁰⁰, 10⁵⁰, 10¹⁰, 10, 5, 2 or 1 ice-ball(s) or ice-ball(s) per cm³ of body part, is/are formed,
   and/or
- below the ice-ball temperature, or more than 10⁻³, 10⁻¹, 1, 5 or 10 °C below the ice-ball temperature, more than 10¹⁰⁰, 10⁵⁰, 10¹⁰, 10, 5, 2 or 1 ice-ball(s) or ice-ball(s) per cm³ of body part, is/are formed.

In some other embodiment, ice-ball(s) represent(s) or is/are an assembly of or of more than 1, 2, 5, 10, 10³, 10⁵, 10¹⁰, 10²⁰ or 10⁵⁰ ice-ball(s).

In some other embodiment, ice-ball(s) represent(s) or is/are an assembly of or of less than 10⁵⁰, 10²⁰, 10¹⁰, 10⁵, 10³, 10, 5 or 2 ice-ball(s).

The treatment or method according to the invention is a non-predominant ice-ball cryotherapy. A non-predominant ice-ball cryotherapy relates to a cryotherapy resulting in no or a negligible amount of ice-balls.

In some embodiment of the invention, a non-predominant ice-ball cryotherapy designates a cryotherapy in which the time during which the temperature is below the ice-ball temperature is smaller than:
i) 10⁻⁵⁰, 10⁻²⁰, 10⁻¹⁰, 10⁻⁵, 10⁻¹, 0, 1, 5, 10, 10³, 10⁵ or 10¹⁰ second(s),
   and/or
ii) 10⁻⁵⁰, 10⁻²⁰, 10⁻¹⁰, 10⁻⁵, 10⁻¹, 0, 1, 5, 10, 25, 50, 75, 80, 90, 99, 99.9 or 100% of the duration of the whole method or of at least one step of the method according to the invention, where this percentage can be equal to t_{BIBT}/t_{M} or (t_{M}-t_{BIBT})/t_{M}, where t_{BIBT} is the time or duration during which the temperature is below the ice-ball temperature and t_{M} is the time or duration of the whole method or of at least one step of the method according to the invention.

In some other embodiment of the invention, a non-predominant ice-ball cryotherapy designates a cryotherapy in which a minority of temperatures or less than 100, 50, 20, 10, 5, 2 or 1% of temperatures of at least one step of the method, preferentially resulting in or associated with the medial, cosmetic, diagnostic or therapeutic effect, is below the ice-ball temperature or is at least 10⁻⁵, 10⁻¹, 0, 1, 10, 10², 10³ or 10⁵ °C below the ice-ball temperature.

In some other embodiment of the invention, a non-predominant ice-ball cryotherapy designates a cryotherapy in which: i) the quantity of ice-ball(s), preferentially per cm³ of body part, is smaller than 10¹⁰, 10⁵, 10³, 100, 50, 10 or 1 and/or the size of at least one ice-ball is smaller than the size of the nanoparticle or than 10¹⁰, 10⁵, 10³, 100, 50, 20, 10, 5, 2, 1, 10⁻³, 10⁻⁵ or 10⁻¹⁰ nm.

In still some other embodiment of the invention, the treatment or method according to the invention is a non ice-ball cryotherapy. A non- ice-ball cryotherapy relates to a cryotherapy resulting in no ice-balls or no ice-ball formation.

In some embodiment of the invention, a non ice-ball cryotherapy designates a cryotherapy in which the temperature of the treatment or method according to the invention is above the ice-ball temperature during the whole method or treatment.

In an embodiment of the invention, the non ice-ball cryotherapy or non-predominant ice-ball cryotherapy, is characterized in that nanoparticle and/or body part do/does not comprise or is/are not surrounded by or is/are not covered by or is/are not enveloped by or is/are not in contact with ice or at least one ice-ball, preferentially during at least one step of the method according to the invention.

In some other embodiment, the treatment, preferentially the non ice-ball cryotherapy or non-predominant ice-ball cryotherapy, is characterized in that the at least one ice-ball is located at a distance of more than 10⁻², 10⁻¹, 1, 5, 10, 10³, 10⁵ or 10⁹ nm from the nanoparticle or body part.

In some other embodiment, the treatment, preferentially the non ice-ball or non-predominant ice-ball cryotherapy, is characterized in that ice-balls are present in less than 50, 20, 5, 2 or 1 percent, preferentially in mass or volume, of the body part.

In one embodiment of the invention, the treatment, preferentially the non ice-ball or non-predominant ice-ball cryotherapy, is characterized in that: i), it leads to the formation of a less than 10¹⁰, 10⁵, 10³, 10, 5, 2 or 1 iceball(s), preferentially per cm³ of body part, preferentially comprised in the body part, ii), the cooling temperature is above 0 °C or above the temperature at which iceball(s) start(s) to form, and/or iii) it leads to the destruction, healing, detection of at least one pathological cells or to the cure or healing of the body part without iceball formation.

As used herein, an ice-ball can be a ball of ice or a round-shaped volume of ice.

In some embodiment, an ice-ball can be characterized by at least one of the following properties: i), a size or dimension in at least one direction larger than 10⁻², 10⁻¹, 1, 5, 10, 10³, 10⁶ or 10⁹ nm or nm² or nm³, ii) a size or dimension in at least one direction between 10⁻² and 10⁵⁰ nm or nm² or nm³, 10⁻² and 10⁹, 10⁻¹ and 10⁶, or between 1 and 10³ nm or nm² or nm³, iii) a size or dimension in at least one direction larger than the size or dimension in at least one direction of a nanoparticle by a factor of at least 1.0001, 1.1, 1.5, 2, 5 or 10, where this factor can be the ratio between the size of the iceball and the size of the nanoparticle.

In some other embodiment, an ice-ball characterized by at least one of the following properties: i), a size or dimension in at least one direction smaller 10²⁰, 10¹⁰, 10⁵, 10², 10, 5, 2 or 1 nm or nm² or nm³, ii) a size or dimension in at least one direction smaller than the size or dimension in at least one direction of a nanoparticle by a factor of at least 1.0001, 1.1, 1.5, 2, 5 or 10, where this factor can be the ratio between the size or dimension in at least one direction of the iceball and the size or dimension in at least one direction of the nanoparticle.

In one embodiment of the invention, an ice-ball is a macroscopic ice-ball. In some embodiment, a macroscopic iceball is an ice-ball with a size or dimension in at least one direction larger than 10⁻⁵⁰, 10⁻¹⁰, 10⁻⁵, 10⁻¹, 1, 5, 10 or 10³ nm, preferentially larger than 100 or 10³ nm, most preferably between 100 nm and 1 meter in size.

In still some other embodiment, a macroscopic iceball is an iceball with a size larger than: i), 1, 10, 10², 10³, 10⁵, 10⁹ or 10¹⁰ nm, ii) 1, 5, 10, 20, 50, 70, 90 or 99% in mass or in volume of the body part, iii) an assembly of more than 1, 5, 10, 10², 10³, 10⁵ or 10¹⁰ nanoparticle(s), or iv) the size of at least one iceball produced when the body part comprises at least one nanoparticle.

In another embodiment of the invention, an ice-ball is a nanoscopic iceball. In some embodiment, a nanoscopic ice-ball is an ice-ball with a size or dimension in at least one direction smaller than 10⁵⁰, 10¹⁰, 10⁵, 10³, 10, 5, 2 or 1 nm, preferentially smaller than 100, 10 or 5 nm, most preferentially between 0.1 and 100 nm.

In still some other embodiment, a nanoscopic iceball is an iceball with a size smaller than: i) 10⁵⁰, 10¹⁰, 10⁵, 10³, 10², 10, 5, 2 or 1 nm, ii) 99, 90, 70, 50, 25, 20, 5 or 1% in mass or in volume of the body part, or iii) an assembly of more than 1, 5, 10, 10², 10³, 10⁵ or 10¹⁰ nanoparticle(s).

In some embodiment, an ice-ball has at least one property in common with a macroscopic or nanoscopic ice-ball.

In some other embodiment, an ice-ball has at least one property that is different from that of a macroscopic or nanoscopic ice-ball.

In still some other embodiment, an ice-ball, preferentially nanoscopic, is an iceball that covers, surrounds, envelops, partly or fully, at least one nanoparticle or that is located at a distance of less than 10¹⁰⁰, 10⁵⁰, 10²⁰, 10¹⁰, 10⁵, 10³, 10, 5, 2 or 1 nm from at least one nanoparticle.

In one embodiment of the invention, an ice-ball, preferentially a nanoscopic iceball, is an ice-ball that is comprised in a cell, a cell organelle, or the cell cytoplasm. In some embodiment, such ice-ball can lead to or be associated with a variation in at least one cell or body part property such as a variation of: i), size, volume, length, surface, thickness of a cell or body part, ii), cell membrane permeability. In some embodiment, such variation is observed under the microscope, preferentially by comparing the cell or body part property(ies) before and after the cryo-therapy treatment.

In some embodiment, an ice-ball is ice comprised inside a cell or intracellular ice, and can be designated as intracellular ice-ball.

In some other embodiment, an ice-ball is ice comprised outside a cell or extracellular ice, and can be designated as extracellular ice-ball.

In some other embodiment, intracellular and/or extracellular ice induce(s) the swelling or dilatation or elongation of cells or the increase in the size or volume of cells, preferentially by a factor of at least 1.001, 1.1, 1.5, 2, 5, 10, 10³ or 10⁵, between before and after ice formation.

In some other embodiment, intracellular and/or extracellular ice induce(s) the decrease in the size or volume of cells, preferentially by a factor of at least 1.001, 1.1, 1.5, 2, 5, 10, 10³ or 10⁵, between before and after ice formation

In some other embodiment, the factor of decrease or increase of the volume of cells between before and after ice formation can be increased in the presence of nanoparticles, preferentially nanoparticles internalized in cells.

In still some other embodiment, the treatment is non-iceball cryotherapy or non-predominant ice-ball cryotherapy means that:
i) the treatment does not comprise a step of cooling the body part to a minimum temperature that is below the ice-ball temperature,
   and/or
ii) the majority of the treatment, or more than 10⁻¹⁰⁰, 10⁻⁵⁰, 10⁻¹⁰, 10⁻⁵, 10⁻³, 10⁻¹, 1, 5, 10, 50, 90, 99 or 99.9% of the treatment is carried out above the ice-ball temperature, where this percentage can be T_{AIBT}/Tₜₒₜ, where T_{AIBT} is the lapse of time during which the temperatures is above the ice-ball temperature during the treatment and Tₜₒₜ is the total duration of the treatment.

In one embodiment of the invention, the ice-ball temperature is a threshold temperature characterized in at least one of the following properties:
- temperatures above the ice-ball temperature do not result in ice or ice-ball formation, while temperatures below the ice-ball temperature result in ice or ice-ball formation,
- temperatures above the ice-ball temperature are non-freezing temperatures, while temperatures below the ice-ball temperature are freezing temperatures,
   and
- the ice-ball temperature is lower than 20, 10, 5, 2, 1, 0, -5 or -10 °C.

In still another embodiment of the invention, non-freezing temperatures are temperatures at which ice do not form.

In still some other embodiment of the invention, a non ice-ball cryotherapy or a non-predominant ice-ball cryotherapy is a cryo-therapy that does not lead to the formation of at least one macroscopic iceball but can lead to the formation of at least one nanoscopic iceball.

In still some other embodiment of the invention, a non ice-ball cryotherapy or a non-predominant ice-ball cryotherapy is a cryo-therapy that does not lead to the formation of at least one nanoscopic iceball but can lead to the formation of at least one macroscopic iceball.

In still some other embodiment of the invention, a non ice-ball cryotherapy or a non-predominant ice-ball cryotherapy is a cryo-therapy that does not lead to the formation of at least one nanoscopic iceball and that does not lead to the formation of at least one macroscopic iceball.

In still some other embodiment, an ice-ball comprises crystallized ice, where the presence of crystallized ice can in some embodiment be revealed by the presence of at least one crystallographic plane or ordered atomic arrangement within the iceball.

In some embodiment, an ice-ball is intracellular, *i.e.* it preferentially forms inside cells, cell organelles, cell cytoplasm, or cell membrane.

In some other embodiment, an ice-ball is extracellular, *i.e.* it preferentially forms outside cells, cell organelles, cell cytoplasm, or cell membrane.

In some other embodiment of the invention, an iceball comprises or embeds at least 1, 5, 10, 10³, 10⁵, or 10¹⁰ nanoparticles.

In some embodiment, the size of the ice-ball comprising or embedding the nanoparticle(s) can be at least 1.01, 1.1, 1.5, 2, 5, 10 or 10³ smaller than the size of the ice-ball not comprising or not embedding the nanoparticle(s), where such comparison can be made by creating ice-ball(s) in similar conditions in terms of cooling conditions with and without nanoparticle(s). This can be the case when nanoparticles favor the formation of small ice-balls, preferentially ice-balls that comprise a limited number of nanoparticles, preferentially a number of nanoparticle(s) smaller than 10⁵⁰, 1010, 10⁵, 10³, 100, 10, 5, 2 or 1, or ice-ball(s) that surround or coat at least one nanoparticle with a coating or layer or ice that has a thickness smaller than: i) the diameter of the nanoparticle and/or ii) 10⁵⁰, 10²⁰, 10¹⁰, 10⁵, 10³, 500, 100, 75, 50, 20, 10, 5, 2, 1 or 10⁻¹ nm.

In some embodiment, the size of the ice-ball(s) comprising or embedding the nanoparticle(s) can be at least 1.01, 1.1, 1.5, 2, 5, 10 or 10³ smaller than the size of the ice-ball(s) not comprising or not embedding the nanoparticle(s), where such comparison can be made by creating ice-ball(s) in similar conditions in terms of cooling conditions with and without nanoparticle(s). This can be the case when nanoparticle(s) favor the formation of large ice-balls, preferentially ice-balls that comprise a large number of nanoparticles, preferentially a number of nanoparticle(s) larger than 1, 5, 10, 100, 10³, 10⁵ or 10¹⁰, or ice-ball(s) that surround or coat at least one nanoparticle with a coating or layer or ice that has a thickness larger than: i) the diameter of the nanoparticle and/or ii) 10⁻⁵⁰, 10⁻²⁰, 10⁻¹⁰, 10⁻⁵, 10⁻³, 10⁻¹, 0, 1, 5, 10, 50 or 100 nm.

In still some other embodiment, non-predominant ice-ball or non-ice ball cryotherapy means that ice-ball(s) do/does not form during or in at least one step of the method or treatment according to the invention.

In still some other embodiment, non-predominant ice-ball or non-ice ball cryotherapy means that ice-ball(s) do/does not form within a lapse of time shorter than 10³, 10, 5, 2, 1, 10⁻⁵ or 10⁻¹⁰ year(s), hour(s), minute(s) or second(s), or between 10⁻¹⁰ and 10³ year(s), hour(s), minute(s) or second(s).

In still some other embodiment, non-predominant ice-ball or non-ice-ball cryotherapy means that iceball(s) do/does not form within a lapse of time longer than 10⁻¹⁰, 10⁻⁵, 1, 2, 5, 10 or 10³ year(s), hour(s), minute(s) or second(s).

In still some other embodiment, the non-predominant ice-ball cryotherapy or non-ice ball cryotherapy, is characterized in that:
- it is carried out at temperatures, preferentially initial, cooling, and/or final temperature(s), larger than the ice-ball temperature, preferentially by at least 10⁻¹⁰, 10⁻⁵, 10⁻³, 10⁻¹, 1, 5 or 10 °C,
   and/or
- it results in the formation of less than 10⁵⁰, 10²⁰, 10¹⁰, 10⁵, 10³, 10, 5, 2 or 1 iceball(s).

In some other embodiment, the treatment, preferentially ice-ball cryotherapy, is characterized in that:
i) it is carried out at temperatures, preferentially initial, cooling, and/or final temperature(s), lower than the ice-ball temperature, preferentially by at least 10⁻¹⁰, 10⁻⁵, 10⁻³, 10⁻¹, 1, 5 or 10 °C,
   and/or
ii) it results in the formation of more than 1, 2, 5, 10, 10³, 10⁵, 10¹⁰ or 10⁵⁰ iceball(s).

In another embodiment, the ice-ball temperature is such that when the temperature is decreased from a temperature above the ice-ball temperature, preferentially at least 10⁻²⁰, 10⁻¹⁰, 10⁻⁵, 10⁻³, 10⁻¹, 1, 5, 10 or 100 °C above the ice-ball temperature, and a temperature below the ice-ball temperature, preferentially at least 10⁻²⁰, 10⁻¹⁰, 10⁻⁵, 10⁻³, 10⁻¹, 1, 5, 10 or 100 °C below the ice-ball temperature, the number or size of ice-ball increases by a factor of at least 1, 1.1, 2, 5, 10, 10³ or 10¹⁰, where this factor can be N₂/N₁ or S₂/S₁, where N₂ and N₁ are the numbers of ice-balls below and above the ice-ball temperature, respectively, and S₂ and S₁ are the sizes of iceballs below and above the ice-ball temperature, respectively.

In one aspect, the invention relates to nanoparticles for use according to the invention, wherein the succession of at least one of the cooling step, the maintaining step, and the warming step is repeated more than 1, 2, 3, 5, 6, 10, or 10³ times, preferably more than 6 times, most preferably more than 3 times.

In one aspect, the invention relates to nanoparticles for use according to the invention, wherein a cycle is repeated more than 1, 2, 3, 5, 6, 10, or 10³ times, preferably more than 6 times, most preferably more than 3 times

In one aspect, the invention relates to nanoparticle for use according to the invention, wherein the cooling temperature has at least one characteristic selected from the group consisting of:
i) a difference ΔT₁ between the initial and the cooling temperature lower than 57 °C,
   and/or
ii) a difference ΔT₂ between the final and the cooling temperature lower than 57 °C,
and preferably wherein the difference(s) ΔT₁ and/or ΔT₂ decrease(s) when the nanoparticle concentration increases in the body part.

In one embodiment of the invention, the difference ΔT₁ between the initial and the cooling temperature is lower than 10¹⁰, 10⁵, 10³, 100, 90, 80, 60, 57, 50, 40, 30 or 20 °C, preferably lower than 10³ or 100, more preferably lower than 57 °C.

In one embodiment of the invention, the difference ΔT₂ between the final and the cooling temperature is lower than 10¹⁰, 10⁵, 10³, 100, 90, 80, 60, 57, 50, 40, 30 or 20 °C, preferably lower than 10³ or 100, more preferably lower than 57 °C

In one embodiment of the invention, small value(s) of ΔT₁ and/or ΔT₂ is/are reached or desired to carry out a large number of cycles and/or to avoid reaching the ice-ball temperature or too low temperatures that can result in side effects.

In another embodiment of the invention, the difference ΔT₁ between the initial and the cooling temperature is larger than 10⁻⁵⁰, 10⁻¹⁰, 10⁻⁵, 10⁻¹, 1, 5, 10, 20, 30, 50, 70, 80, 100 or 1000 °C, preferably larger than 1 or 5 °C, more preferably larger than 10 °C.

In one embodiment of the invention, the difference ΔT₂ between the final and the cooling temperature is larger than 10⁻⁵⁰, 10⁻¹⁰, 10⁻⁵, 10⁻¹, 1, 5, 10, 20, 30, 50, 70, 80, 100 or 1000 °C, preferably larger than 1 or 5 °C, more preferably larger than 10 °C.

In some embodiment of the invention, large value(s) of ΔT₁ and/or ΔT₂ is/are reached or desired to carry out a small number of cycles and/or to reach the ice-ball temperature or temperature below 0 °C that can increase the medical or cosmetic or therapeutic or diagnostic.

In another embodiment of the invention, the difference ΔT₁ between the initial and the cooling temperature is between 10⁻⁵⁰ and 10⁵⁰, 10⁻¹⁰ and 10¹⁰, 10⁻⁵ and 10⁵, 10⁻³ and 10³, 10⁻² and 10², 10⁻¹ and 100, preferably between 1 and 70, more preferably between 2 and 50 °C.

In one embodiment of the invention, the difference(s) ΔT₁ and/or ΔT₂ can be decreased by increasing the nanoparticle concentration in the body part. This can mean that by introducing nanoparticles in the body part, preferentially at a concentration larger than 10⁻⁵⁰, 10⁻¹⁰, 10⁻⁵, 10⁻³, 10⁻¹, 1, 5, 10, 10³ or 10⁵ mg of nanoparticles per cm3 of body part, the medical or cosmetic or therapeutic or diagnostic can be reached by using value(s) of ΔT₁ and/or ΔT₂ that is/are at least 0, 0.5, 1, 1.1, 1.5, 2, 5, 10, 10³ or 10⁵ smaller than the value(s) of ΔT₁ and/or ΔT₂ reached in the absence of nanoparticle.

In one aspect, the invention also relates to nanoparticle for use according to any of the preceding claims, wherein:
i) in step d) the warming time to reach the final temperature from the cooling temperature is increased with increasing nanoparticle concentration in the body part,
   and/or
ii) in step b) the cooling time to reach the cooling temperature from the initial temperature is not predominantly dependent on nanoparticle concentration in the body part.

In another embodiment of the invention, the time to reach the final temperature from either the initial or cooling temperature increases or is increased, preferentially by a factor of at least 0, 0.5, 1, 1.1, 2, 5, 10, 10³ or 10⁵, when the nanoparticle concentration in the body part is increased, preferentially by a factor of at least 0, 0.5, 1, 1.1, 2, 5, 10, 10³ or 10⁵.

In the experimental example, when PC3-Luc cells in the presence (or not) of 1 mg/mL of N-CMD are cooled down from RT (room temperature) to a cooling temperature of 10 °C or 0°C and let warming up from 10°C or 0°C to RT, the duration of the warming step warming increases : i) for the cooling temperature of 0°C, from 311 seconds without N-CMD to 461 seconds with 1 mg/mL of N-CMD, ii) for the cooling temperature of 10 °C, from 256 seconds without N-CMD to 393 seconds with 1 mg/mL of N-CMD. In some embodiment, the difference between the warming time of the cells with N-CMD and the warming time of the cells without N-CMD can be increased when the nanoparticle concentration is increased, the cooling temperature is decreased, and/or the final temperature is increased. In some embodiment, the difference between the warming time of the cells with N-CMD and the warming time of the cells without N-CMD can be decreased when the nanoparticle concentration is decreased, the cooling temperature is increased, and/or the final temperature is decreased.

In another embodiment of the invention, the cooling time to reach the cooling temperature from the initial temperature is not predominantly dependent on nanoparticle concentration in the body part. In some embodiment, when the nanoparticle concentration in the body part increases, preferentially by a factor of at least 0, 0.5, 1, 1.1, 1.5, 2, 5, 10 or 10³, or preferentially from less than 10¹⁰, 10⁵, 10³, 10, 5, 2 or 1 mg of nanoparticles per cm³ of body part to more than 10⁻¹⁰, 10⁻⁵, 10⁻³, 10⁻¹, 1, 5, 10, 100 or 500 mg of nanoparticles per cm³ of body part, the cooling time varies by less 100, 50, 20, 10, 5, 2 or 1 %. This can be the case when the temperature adjuster is used during the cooling step to cool the body part.

In the experimental example, when PC3-Luc cells in the presence (or not) of 1 mg/mL of N-CMD are cooled down from RT (room temperature) to a cooling temperature of 10 °C or 0°C and let warming up from 10°C or 0°C to RT, the duration of the cooling step remains similar for the cooling temperature of 0°C and 10°C with/without N-CMD at 25-45 seconds. The difference between 25 and 45 seconds can be due to the conditions of use of the temperature adjuster (URGO).

In one aspect, the invention relates to nanoparticles for use according to the invention, wherein in step b) the temperature decreases from the initial temperature to the cooling temperature in the cooling step according to at least one of:
i) a cooling rate in the range from 10⁻⁶ °C/sec to 10⁶ °C/sec, preferably 10⁻³ °C/sec to 10³ °C/sec,
ii) a cooling rate with the nanoparticle(s) that differs by less than 10 °C/sec from the cooling rate without the nanoparticles,
iii) a cooling rate that does vary with varying nanoparticle concentration or vary by a factor in the range of less than 10⁻⁶ °C/sec to 10⁶ °C/sec, preferably less than 10⁻³ °C/sec to 10³ °C/sec, when the nanoparticle concentration increases, preferably either by a factor of at least 1.1 or from a concentration lower than 100 µg of nanoparticles per cm³ of body part to a concentration larger than 100 µg of nanoparticles per cm³ of body part,
iv) a cooling time of between 10⁻⁶ and 10⁶ seconds, preferably of between 10⁻³ and 10³ seconds,
v) a cooling rate that is smaller than the rate of increasing the temperature in the warming step d) by a factor in the range from 10⁻¹⁰ to 10⁵, preferably 10⁻⁵ to 10³, and
vi) a cooling time shorter than the warming time in the warming step d) by a factor in the range of 10⁻⁶ to 10⁶, preferably 10⁻³ to 10³.

In one embodiment of the invention, the temperature, preferentially of the body part, is decreased or decreases from the initial temperature down to the cooling temperature, preferentially in or during the cooling step, at a rate designated as the rate of temperature decrease or cooling rate.

In still another embodiment of the invention, the cooling rate is larger than 10⁻²⁰, 10⁻¹⁰, 10⁻⁵, 10⁻³, 10⁻¹, 1, 5, 10, 10³ or 10⁵ °C (degree Celsius) per second or °C per minute, preferentially per cm³ of body part, preferentially per mg of nanoparticle.

In still another embodiment of the invention, the cooling rate is smaller than 10¹⁰⁰, 10⁵⁰, 10¹⁰, 10⁵, 10³, 10, 5, 2, 1, 10⁻¹, 10⁻⁵ or 10⁻¹⁰ °C per second or °C per minute, preferentially per cm³ of body part, preferentially per mg of nanoparticles.

In still another embodiment of the invention, the cooling rate is between 10⁻¹⁰⁰ and 10¹⁰⁰, 10⁻⁵⁰ and 10²⁰, 10⁻¹ and 10⁵, or between 1 and 100 °C per second or °C per minute, preferentially per cm³ of body part, preferentially per mg of nanoparticles.

In the experimental example, when PC3-Luc cells in the presence (or not) of 1 mg/mL of N-CMD are cooled down from RT (room temperature) to a cooling temperature of 10 °C or 0°C and let warming up from 10°C or 0°C to RT, the cooling rate is relatively similar for the cooling temperature of 0 °C and 10 °C, with/without N-CMD, at 0.6 to 1 °C/sec. The difference between 0.6 °C/sec and 1 °C/sec can be due to the conditions of use of the temperature adjuster (URGO). In some embodiment, the cooling rate can be larger if/when the temperature adjuster enables reaching the cooling temperature faster. In some other embodiment, the cooling rate can be lower if/when the temperature adjuster enables reaching the cooling temperature slower.

In still another embodiment of the invention, the cooling rate is lower by a factor of at least 1.001, 1.1, 1.5, 2, 5 or 10, in the presence than in the absence of ice-ball(s).

In still another embodiment of the invention, the cooling rate is larger by a factor of at least 1.001, 1.1, 1.5, 2, 5 or 10, in the presence than in the absence of ice-ball(s).

In still another embodiment of the invention, the cooling rate is lower, preferentially by a factor of at least 1.001, 1.1, 1.5, 2, 5 or 10, in the presence than in the absence of nanoparticles.

In still another embodiment of the invention, the cooling rate is larger, preferentially by a factor of at least 1.001, 1.1, 1.5, 2, 5 or 10, in the presence than in the absence of nanoparticles.

In still another embodiment of the invention, the cooling rate is similar in the presence and absence of nanoparticles.

In still another embodiment of the invention, the cooling rate of the body part with nanoparticles differs by less than 10¹⁰⁰, 10⁵⁰, 10³, 10, 10⁻¹, 10⁻³ °C/sec from the cooling rate of the body part without the nanoparticles.

In still another embodiment of the invention, the cooling rate of the body part with nanoparticles differs by more than 10⁻¹⁰⁰, 10⁻⁵⁰, 10⁻³, 10⁻¹, 1, 10 or 10³ °C/sec from the cooling rate of the body part without the nanoparticles.

In still another embodiment of the invention, the cooling rate of the body part comprising the nanoparticle varies by less than 10²⁰, 10¹⁰, 10⁵, 10, 5, 2, 1, 10⁻³ or 10⁻⁵ °C/sec. when the nanoparticle concentration increases by a factor of at least 0, 0.5, 1, 1.1, 2, 5, 10 or 10³ or when the nanoparticle concentration increases from a concentration lower than 10³, 10², 10, 1, 10⁻¹, 10⁻³, 10⁻⁶ or 10⁻⁹ mg of nanoparticle per cm³ of body part to a concentration larger than 10⁻⁹, 10⁻⁶, 10⁻³, 10⁻¹, 1 or 10 mg of nanoparticles per cm³ of body part.

In one embodiment of the invention, the temperature of the body part is decreased or decreases from the initial temperature to the cooling temperature in or during the cooling step within a lapse of time designated as the duration of temperature decrease or cooling time.

In still another embodiment of the invention, the cooling time is longer than 10⁻¹⁰⁰, 10⁻⁵⁰, 10⁻¹⁰, 10⁻⁵, 10⁻³, 10⁻¹, 1, 5, 10 or 10² seconds.

In still another embodiment of the invention, the cooling time is shorter than 10¹⁰⁰, 10⁵⁰, 10¹⁰, 10⁵, 10³, 10, 5, 2, 1, 10⁻¹ or 10⁻² seconds.

In still another embodiment of the invention, the cooling time is between 10⁻¹⁰⁰ and 10¹⁰⁰, between 10⁻⁵⁰ and 10⁵⁰, between 10⁻¹⁰ and 10¹⁰, between 10⁻⁵ and 10⁵, between 10⁻³ and 10³, or between 10⁻³ and 10 seconds.

In one embodiment of the invention, the temperature is increased or increases from the cooling temperature to the final temperature, preferentially in or during the warming step, at a rate designated as the rate of temperature increase or warming rate.

In one embodiment of the invention, the cooling rate is smaller than the warming rate, preferentially by: i) a factor of at least 10⁻⁵⁰, 10⁻¹⁰, 0, 0.5, 1, 1.1, 1.5, 2, 5, 10, 10³ or 10⁵ or ii) more than 10⁻⁵⁰, 10⁻¹⁰, 10⁻⁵, 10⁻¹, 0, 0.5, 1, 1.1, 1.5, 2, 5, 10, 10³ or 10⁵ °C/sec.

In one embodiment of the invention, the cooling rate is smaller than the warming rate, preferentially by: i) a factor of less than 10¹⁰, 10⁵, 10, 5, 2, 1, 1.1, 1, 0.5, 0, 10⁻³ or 10⁻¹⁰ or ii) less than 10⁵⁰, 10¹⁰, 10⁵, 10, 5, 2, 1.1, 1, 0.5, 0, 10⁻³ or 10⁻⁵ °C/sec.

In one embodiment of the invention, the cooling rate is smaller than the warming rate, preferentially by: i) a factor of between 10⁻¹⁰⁰ and 10¹⁰⁰, 10⁻¹⁰ and 10¹⁰, 10⁻⁵ and 10⁵, 10⁻³ and 10³, or between 10⁻¹ and 10, or ii) between 10⁻¹⁰⁰ and 10¹⁰⁰, 10⁻¹⁰ and 10¹⁰, 10⁻⁵ and 10⁵, 10⁻³ and 10³, or between 10⁻¹ and 10 °C/sec.

In the experimental example, when PC3-Luc cells in the presence (or not) of 1 mg/mL of N-CMD are cooled down from RT (room temperature) to a cooling temperature of 10 °C or 0°C and let warming up from 10°C or 0°C to RT, the rate of temperature increase or warming rate, R_{TI}, is between 0.05 and 0.08 °C/sec while the rate of temperature decrease or cooling rate, R_{TD}, is between 0.6 and 1 °C/sec. Therefore, the ratio R_{TD}/R_{TI} is between 7.5 and 20. Smaller or larger values of this ratio may also be obtained by using other experimental conditions (different temperature adjuster, nanoparticle type or concentration, or another cell type).

In one embodiment of the invention, the temperature is increased or increases from the cooling temperature to the final temperature, preferentially in or during the warming step, within a lapse of time designated as the duration of the warming step or warming time.

In one embodiment of the invention, the cooling time is shorter than the warming time, preferentially by: i) a factor of at least 10⁻⁵⁰, 10⁻¹⁰, 0, 0.5, 1, 1.1, 1.5, 2, 5, 10, 10³ or 10⁵ or ii) more than 10⁻⁵⁰, 10⁻¹⁰, 10⁻⁵, 10⁻¹, 0, 0.5, 1, 1.1, 1.5, 2, 5, 10, 10³ or 10⁵ sec.

In one embodiment of the invention, the cooling time is shorter than the warming time, preferentially by: i) a factor of less than 10¹⁰, 10⁵, 10, 5, 2, 1, 1.1, 1, 0.5, 0, 10⁻³ or 10⁻¹⁰ or ii) less than 10⁵⁰, 10¹⁰, 10⁵, 10, 5, 2, 1.1, 1, 0.5, 0, 10⁻³ or 10⁻⁵ sec.

In one embodiment of the invention, the cooling time is shorter than the warming time, preferentially by: i) a factor of between 10⁻¹⁰⁰ and 10¹⁰⁰, 10⁻¹⁰ and 10¹⁰, 10⁻⁵ and 10⁵, 10⁻³ and 10³, or between 10⁻¹ and 10, or ii) between 10⁻¹⁰⁰ and 10¹⁰⁰, 10⁻¹⁰ and 10¹⁰, 10⁻⁵ and 10⁵, 10⁻³ and 10³, or between 10⁻¹ and 10 sec.

In the experimental example, when PC3-Luc cells in the presence (or not) of 1 mg/mL of N-CMD are cooled down from RT (room temperature) to a cooling temperature of 10 °C or 0°C and let warming up from 10°C or 0°C to RT, the time of temperature increase, t_{TI}, is between 256 and 469 seconds while the time of temperature decrease, t_{TD}, is between 12 and 46 sec. Therefore, the ratio t_{TI} / t_{TD} is between 6 and 39.

In some embodiment, smaller values of the ratio t_{TI} / t_{TD} and/or R_{TD}/R_{TI} is/are reached when the temperature adjuster cools down the body part at a slower rate and/or when the warming step is carried out in the presence of a larger quantity of nanoparticle, of radiation, or of a medium than enables to increase the rate of temperature increase in the warming step.

In some embodiment, larger values of the ratio t_{TI} / t_{TD} and/or R_{TD}/R_{TI} is/are reached when the temperature adjuster cools down the body part at a faster rate and/or when the warming step is carried out in the presence of a lower quantity of nanoparticle, of radiation, or of a medium that enables to decrease the rate of temperature increase in the warming step.

In still another embodiment of the invention, the cooling time is shorter by a factor of at least 1.001, 1.1, 1.5, 2, 5 or 10, in the presence than in the absence of ice-ball(s).

In still another embodiment of the invention, the cooling time is larger by a factor of at least 1.001, 1.1, 1.5, 2, 5 or 10, in the presence than in the absence of ice-ball(s).

In one aspect, the invention relates to nanoparticles for use according to the invention, wherein the maintaining step is carried out according to at least one of:
i) a maintaining time that is shorter than the cooling time and/or warming time, preferably by a factor of at least 1.5, more preferably by a factor of at least 10,
   and
ii) fluctuation of the temperature during the maintaining step c) that is smaller, preferably by a factor of at least 1.5, than ΔT₁ and/or ΔT₂ according to the invention.

In one embodiment of the invention, the step of not maintaining the body part comprising the nanoparticles at the cooling temperature for a duration of time of more than 10⁵⁰, 10²⁰, 10³, 10², 10, 5, 2, 1, 10⁻¹ or 10⁻³ seconds is designated as the maintaining step.

In one embodiment of the invention, the temperature is maintained at the cooling temperature, preferentially in or during the maintaining step, within a lapse of time designated as the duration of the maintaining step or maintaining time.

In one embodiment of the invention, the maintaining time is shorter than 10²⁰, 10¹⁰, 10⁵, 10³, 10, 5, 2, 1, 10⁻¹, 10⁻³ or 10⁻⁶ second(s).

In another embodiment of the invention, the maintaining time is longer than 10⁻⁵⁰, 10⁻²⁰, 10⁻⁶, 10⁻³, 10⁻¹, 1, 2, 5, 10, 10³, 105 or 10¹⁰ second(s).

In still another embodiment of the invention, the maintaining time is between 10⁻⁵⁰ and 10⁵⁰, 10⁻¹⁰ and 10¹⁰, 10⁻⁵ and 10⁵, 10⁻³ and 10³, or between 10⁻¹ and 10³ seconds.

In the experimental example, the duration of the maintaining steps is less than 1 seconds for the short cycles and longer than 1 second for the long cycles. The maintaining time can be decreased by starting the warming step quicker after the cooling step. The maintaining time can be longer by using a temperature adjuster that enables maintaining the temperature at the cooling temperature for a longer period of time.

In still another embodiment of the invention, the maintaining time is at least 1.001, 1.1, 2, 3, 5, 10, 15, 20, 50, 10², 10³, 10⁵ or 10¹⁰ longer than the cooling time and/or than the warming time. In some cases, the duration of the maintaining step can be long when the temperature is maintained at the minimum or cooling temperature for a long period of time, notably when an equipment is used to maintain the temperature of the body part at the minimum temperature for a long period of time.

In still another embodiment of the invention, it can be useful to maintain the temperature at the cooling temperature during a certain time, preferentially to be in similar conditions as a usual cryotherapy treatment that would be improved in terms of efficacy and/or reduction of side effects by the presence of nanoparticles.

In still another embodiment of the invention, the duration of the maintaining step is at least 1.001, 1.1, 2, 3, 5, 10, 15, 20, 50, 10², 10³, 10⁵ or 10¹⁰ shorter than the duration of the cooling step and/or than the duration of the warming step. In some embodiment, the duration of the maintaining step can be short when the temperature is not maintained at the minimum or cooling temperature for a long period of time, notably when an equipment is not used to maintain the temperature at the minimum temperature for a long period of time. This method can be used when one desires making an important number of cycles, which can be more efficient than maintaining the temperature at the cooling temperature. This method can also be easy to implement since it does not necessitate to measure the temperature during the treatment, but just to know the times of the cooling and/or warming step(s).

In still another embodiment of the invention, the maintaining step is a step in or during which the temperature of the body part is maintained at the cooling temperature, where the cooling temperature is preferentially the temperature of the body part reached during the maintaining step.

In some other embodiment, the fluctuation of temperature in or during the maintaining step, preferentially designated as maintaining step temperature fluctuation, is estimated by the absolute value of (T_{cool}-Tₘᵢₙ)/Tₘᵢₙ, (T_{BP}-Tₘᵢₙ)/Tₘᵢₙ, T_{BP}/Tₘᵢₙ, or T_{cool}/Tₘᵢₙ, where T_{BP}, T_{cool}, and Tₘᵢₙ are the temperature of the body part, the cooling temperature and the minimum temperature, preferentially of or reached during the maintaining step.

In still some other embodiment, the maintaining step temperature fluctuation is smaller than 10¹⁰⁰, 10⁵⁰, 10¹⁰, 10⁵, 10³, 10², 50, 10, 5, 2, 1, 0.5, 0, 10⁻¹, 10⁻³, 10⁻⁵ or 10⁻¹⁰%.

In still some other embodiment, the maintaining step temperature fluctuation is larger than 10⁻¹⁰⁰, 10⁻⁵⁰, 10⁻¹⁰, 10⁻⁵, 10⁻³, 10⁻¹, 0, 1, 5, 10, 10³ or 10⁵%.

In still some other embodiment, the maintaining step temperature fluctuation is within the range from 10⁻²⁰% to 10²⁰%, 10⁻⁵% and 10⁵%, 10⁻³% to 10³%, or from 10⁻¹% and 10%.

In some other embodiment, the maintaining step temperature fluctuation is small or is smaller, preferentially by a factor of at least 0, 0.1, 0.5, 1, 1.1, 1.5, 2, 5, 10, 10², 10³ or 10⁵, than the temperature gradient(s) of the cooling step and/or of the warming step, ΔT₁ and/or ΔT₂.

In the experimental examples, the temperature variation of the maintaining step is typically between 0 °C and 1 °C while the temperature variation of the cooling and/or warming step(s) is typically between 15 and 65 °C.

In one aspect, the invention relates to nanoparticles for use according to the invention, wherein in step d) the temperature increases from the cooling temperature to the final temperature according to at least one of:
i) a warming rate in the range of 10⁻⁶ °C/sec to 10⁶ °C/sec, preferably in the range of 10⁻³ °C/sec to 10³ °C/sec,
ii) a warming rate with the nanoparticle(s) that differs or is larger by a factor of more than 10⁻³ °C from the warming rate without the nanoparticle(s),
iii) a warming rate that is smaller than the cooling rate by a factor in the range from 10⁻¹⁰ to 10⁵, preferably 10⁻⁵ to 10³,
iv) a warming time of between 10⁻⁶ and 10⁶ seconds, preferably of between 10⁻³ and 10³ seconds,
   and
vi) a warming time longer than the cooling time by a factor in the range of 10⁻⁶ to 10⁶, preferably a factor in the range 10⁻³ to 10³.

In one embodiment of the invention, the temperature, preferentially of the body part, is increased or increases from the cooling temperature up to the final temperature, preferentially in or during the warming step, at a rate designated as the rate of temperature increase or warming rate.

In still another embodiment of the invention, the warming rate is larger than 10⁻²⁰, 10⁻¹⁰, 10⁻⁵, 10⁻³, 10⁻¹, 1, 5, 10, 10³ or 10⁵ °C (degree Celsius) per second or °C per minute, preferentially per cm³ of body part, preferentially per mg of nanoparticle.

In still another embodiment of the invention, the warming rate is smaller than 10¹⁰⁰, 10⁵⁰, 10¹⁰, 10⁵, 10³, 10, 5, 2, 1, 10⁻¹, 10⁻⁵ or 10⁻¹⁰ °C per second or °C per minute, preferentially per cm³ of body part, preferentially per mg of nanoparticles.

In still another embodiment of the invention, the warming rate is between 10⁻¹⁰⁰ and 10¹⁰⁰, 10⁻⁵⁰ and 10²⁰, 10⁻³ and 10³, 10⁻¹ and 10⁵, or between 1 and 100 °C per second or °C per minute, preferentially per cm³ of body part, preferentially per mg of nanoparticles.

In still another embodiment of the invention, the warming rate is lower by a factor of at least 1.001, 1.1, 1.5, 2, 5 or 10, in the presence than in the absence of ice-ball(s).

In still another embodiment of the invention, the warming rate is larger by a factor of at least 1.001, 1.1, 1.5, 2, 5 or 10, in the presence than in the absence of ice-ball(s).

In still another embodiment of the invention, the warming rate is lower, preferentially by a factor of at least 1.001, 1.1, 1.5, 2, 5 or 10, in the presence than in the absence of nanoparticles. In some cases, the presence of the nanoparticles in the body part can decrease the rate at which the temperature increases in the warming step, for example when nanoparticles capture or absorb the heat of the body part, preferentially without releasing it, or when nanoparticles act as isolating material.

In still another embodiment of the invention, the warming rate is larger, preferentially by a factor of at least 1.001, 1.1, 1.5, 2, 5 or 10, in the presence than in the absence of nanoparticles. In some cases, the presence of the nanoparticles in the body part can increase the rate at which the temperature increases in the warming step, for example when the nanoparticles release heat during the warming step.

In still another embodiment of the invention, the warming rate is similar in the presence and absence of nanoparticles.

In still another embodiment of the invention, the rate of temperature increase of the body part with nanoparticles differs by less than 10¹⁰⁰, 10⁵⁰, 10³, 10, 10⁻¹, 10⁻³ °C/sec from the rate at which the temperature of the body part without the nanoparticles increases from the cooling temperature to the final temperature.

In still another embodiment of the invention, the rate of temperature increase of the body part with nanoparticles differs by more than 10⁻¹⁰⁰, 10⁻⁵⁰, 10⁻³, 10⁻¹, 1, 10 or 10³ °C/sec from the rate at which the temperature of the body part without the nanoparticles increases from the cooling temperature to the final temperature.

In still another embodiment of the invention, the rate of temperature increase of the body part comprising the nanoparticle varies by more than 10²⁰, 10¹⁰, 10⁵, 10, 5, 2, 1, 10⁻³ or 10⁻⁵ °C/sec. when the nanoparticle concentration increases by a factor of at least 0, 0.5, 1, 1.1, 2, 5, 10 or 10³ or when the nanoparticle concentration increases from a concentration lower than 10³, 10², 10, 1, 10⁻¹, 10⁻³, 10⁻⁶ or 10⁻⁹ mg of nanoparticle per cm³ of body part to a concentration larger than 10⁻⁹, 10⁻⁶, 10⁻³, 10⁻¹, 1 or 10 mg of nanoparticles per cm³ of body part.

In one embodiment of the invention, the temperature of the body part is increased or increases from the cooling temperature to the final temperature in or during the warming step within a lapse of time designated as the duration of temperature increase or warming time.

In still another embodiment of the invention, the warming time is longer than 10⁻¹⁰⁰, 10⁻⁵⁰, 10⁻¹⁰, 10⁻⁵, 10⁻³, 10⁻¹, 1, 5, 10 or 10² seconds.

In still another embodiment of the invention, the warming time is shorter than 10¹⁰⁰, 10⁵⁰, 10¹⁰, 10⁵, 10³, 10, 5, 2, 1, 10⁻¹ or 10⁻² seconds.

In still another embodiment of the invention, the warming time is between 10⁻¹⁰⁰ and 10¹⁰⁰, between 10⁻⁵⁰ and 10⁵⁰, between 10⁻¹⁰ and 10¹⁰, between 10⁻⁵ and 10⁵, between 10⁻³ and 10³, or between 10⁻³ and 10 seconds.

Experimentally, the parameters of the different treatments (initial, cooling, final temperatures as well as duration and rate of temperature decrease and temperature increase) are summarized in **tables 1 to 6** for 1, 3, 6 cycles during which PC3-Luc cells alone or PC3-Luc cells mixed with 1 mg of nanoparticles (N-MCD) are cooled down during the cooling step and let warming up during the warming step. The cooling temperatures are either just above 0°C (>0°C) or just below 0°C (<0°C). We observed that the rate of temperature increase is usually smaller in the presence than in the absence of nanoparticle. Hence, the presence of nanoparticles enables: i) extending the duration of the warming step and/or ii) decreasing the rate of temperature increase of the warming step.

In one embodiment, in the absence of the application of a radiation that can heat the nanoparticles, such as a laser, acoustic wave, magnetic field, preferentially alternating magnetic field, the rate of temperature increase of the warming step is smaller, preferentially by a factor of at least 0, 0.5, 1, 1.5, 2, 5, 10, 10³ or 10⁵, in the presence than in the absence of the nanoparticle.

In one embodiment, in the presence of the application of a radiation that can heat the nanoparticles, such as a laser, acoustic wave, magnetic field, preferentially alternating magnetic field, the rate of temperature increase of the warming step is larger, preferentially by a factor of at least 0, 0.5, 1, 1.5, 2, 5, 10, 10³ or 10⁵, in the presence than in the absence of the nanoparticle.

In one embodiment of the invention, the warming rate is smaller than the cooling rate, preferentially by: i) a factor of at least 10⁻⁵⁰, 10⁻¹⁰, 0, 0.5, 1, 1.1, 1.5, 2, 5, 10, 10³ or 10⁵ or ii) more than 10⁻⁵⁰, 10⁻¹⁰, 10⁻⁵, 10⁻¹, 0, 0.5, 1, 1.1, 1.5, 2, 5, 10, 10³ or 10⁵ °C/sec. This may occur when no radiation is applied during the warming step and/or a substance or equipment is used during the cooling step to adjust the temperature during this step.

In one embodiment of the invention, the warming rate is smaller than the cooling rate, preferentially by: i) a factor of less than 10¹⁰, 10⁵, 10, 5, 2, 1, 1.1, 1, 0.5, 0, 10⁻³ or 10⁻¹⁰ or ii) less than 10⁵⁰, 10¹⁰, 10⁵, 10, 5, 2, 1.1, 1, 0.5, 0, 10⁻³ or 10⁻⁵ °C/sec. This may occur when radiation is applied during the warming step and/or no substance or no equipment is used during the cooling step to adjust the temperature during this step.

In one embodiment of the invention, the warming rate is smaller than the cooling rate, preferentially by: i) a factor of between 10⁻¹⁰⁰ and 10¹⁰⁰, 10⁻¹⁰ and 10¹⁰, 10⁻⁵ and 10⁵, 10⁻³ and 10³, or between 10⁻¹ and 10, or ii) between 10⁻¹⁰⁰ and 10¹⁰⁰, 10⁻¹⁰ and 10¹⁰, 10⁻⁵ and 10⁵, 10⁻³ and 10³, or between 10⁻¹ and 10 °C/sec.

In one embodiment of the invention, the warming time is longer than the cooling time, preferentially by: i) a factor of at least 10⁻⁵⁰, 10⁻¹⁰, 0, 0.5, 1, 1.1, 1.5, 2, 5, 10, 10³ or 10⁵ or ii) more than 10⁻⁵⁰, 10⁻¹⁰, 10⁻⁵, 10⁻¹, 0, 0.5, 1, 1.1, 1.5, 2, 5, 10, 10³ or 10⁵ sec.

In one embodiment of the invention, the warming time is longer than the cooling time, preferentially by: i) a factor of less than 10¹⁰, 10⁵, 10, 5, 2, 1, 1.1, 1, 0.5, 0, 10⁻³ or 10⁻¹⁰ or ii) less than 10⁵⁰, 10¹⁰, 10⁵, 10, 5, 2, 1.1, 1, 0.5, 0, 10⁻³ or 10⁻⁵ sec.

In one embodiment of the invention, the warming time is longer than the cooling time, preferentially by: i) a factor of between 10⁻¹⁰⁰ and 10¹⁰⁰, 10⁻¹⁰ and 10¹⁰, 10⁻⁵ and 10⁵, 10⁻³ and 10³, or between 10⁻¹ and 10, or ii) between 10⁻¹⁰⁰ and 10¹⁰⁰, 10⁻¹⁰ and 10¹⁰, 10⁻⁵ and 10⁵, 10⁻³ and 10³, or between 10⁻¹ and 10 sec.

In still another embodiment of the invention, the warming time is shorter by a factor of at least 1.001, 1.1, 1.5, 2, 5 or 10, in the presence than in the absence of ice-ball(s).

In still another embodiment of the invention, the warming time is larger by a factor of at least 1.001, 1.1, 1.5, 2, 5 or 10, in the presence than in the absence of ice-ball(s).

In one aspect, the invention relates to nanoparticle for use according to the invention, wherein the temperature is controlled during the cooling step b) and/or the maintaining step c), whereas the temperature is not controlled during the warming step d), preferably wherein the temperature control is carried out using an equipment or a substance, preferably a cryoprobe.

In some embodiment, the equipment or substance used to adjust the temperature or temperature variation or temperature fluctuation or temperature gradient, preferentially designated as temperature adjuster, does not belong to the living organism, body part, or their environment, preferentially before or without the treatment. In some cases, it is different from blood, or ambient air or tissue or organ or body part or cell.

In some embodiment, the temperature adjuster is an equipment used during cryotherapy or cryosurgery such as a cryosurgery unit, more specifically a gynecological or oncological or medical or dermatology or neurosurgery cryosurgery unit.

In some embodiment, the temperature adjuster is selected from the group consisting of: i) freezing liquid, ii), cryogenic gas, iii), NO₂ gas, iv), nitrous oxide gas, v), liquid nitrogen, and vi), dimethyl-ether gas.

In some embodiment, the temperature adjuster is a liquid, a gas, or a solid.

In some embodiment, the temperature adjuster is a metal, a rod, preferentially a metal rod or a piece of metal, preferentially connected to a unit that controls the heat of the rod or metal.

In some embodiment, the temperature adjuster is an equipment that produces radiation, preferentially a radiation that generates heat, such as electromagnetic radiation, light or laser radiation, a magnetic field, preferentially an alternating magnetic field, an acoustic wave, preferentially an ultrasound, or radiofrequency.

In some embodiment of the invention, the temperature adjuster maintains the temperature: i), in some embodiment below 10¹⁰, 10⁵, 10³, 10², 10, 5, 2, 1, 0, -10, -20, - 50, -100, -150 or -200°C, ii) in some other embodiment above -200, -150, -100, -50, -20, -10, 0, 2, 5, 10, 20, 50, 10² or 10³ °C, iii) in still some other embodiment between -200 and 10³, -100 and 10³, -50 and 100, or between -10 and 100 °C (degree Celsius). In some embodiment, the temperature is the temperature of the body part in thermal equilibrium with the temperature adjuster, preferentially during at least one step of the method. In some other embodiment, the temperature is the temperature of the temperature adjuster before it enters into contact with or diffuses heat towards the body part.

In one embodiment of the invention, when the temperature adjuster is used during at least one step of the method, the initial, cooling, and/or final temperature(s) of treatment is/are reached, while when no temperature adjuster is used during at least one step of the method, the initial, cooling, and/or final temperature(s) of the treatment is/are not reached.

In one embodiment of the invention, the temperature adjuster adjusts the temperature of the body part, preferentially comprising the nanoparticles, without modifying the temperature of the remaining part of the living organism, which does not comprise the body part, preferentially without modifying the temperature of the remaining part of the living organism which does not comprise the body part by more than 10⁻⁵⁰, 10⁻¹⁰, 10⁻³, 10⁻¹, 1, 5, 10 or 10³ °C.

In one embodiment of the invention, the method uses or comprises an equipment or substance that measures the temperature or the production of ROS or NOS or the release of at least one compound from the nanoparticle, preferentially designated as the sensor. In some embodiment, the sensor can be a thermometer, a thermocouple, an infrared camera. In some other embodiment, the sensor can be a fluorescent probe. The sensor can be used to adjust the temperature or quantity of ROS or NOS produced by the nanoparticles or to control the release of at least one compound from the nanoparticles, preferentially to specific values that are desired or targeted, preferentially values that yield an efficient treatment and/or minimal side effects and/or maximal benefit to risk ratio of the treatment.

In one embodiment of the invention, the method uses or comprises an equipment or substance that images nanoparticle in the body part and/or that images the body part. Such equipment or substance, also designated as imaging equipment, can be MRI (magnetic resonance imaging), a scanner, CT scanner, PET (positron emission tomography), biopsy, fluorescence, luminescence, absorption, histology, microscopy, transmission or scanning electron microscopy, X-ray, electron, neutron, particle, elemental particle, light diffusion or diffraction or scattering.

In one aspect, the invention also relates to the method according to the invention, wherein temperature is controlled during at least one of the cooling step, the maintaining step, and the warming step without using the temperature adjuster.

In one embodiment of the invention, the cooling step occurs without using the temperature adjuster to cool down the body part, for example when the living organism is entering a state of hypothermia that decreases the temperature of the body part.

In one embodiment of the invention, the warming step occurs without using the temperature adjuster to warm up the body part, for example when the body part is left to warm up by being in contact with air or blood circulation without the need of using a temperature adjuster that does not belong to the organism or body part.

In one aspect, the invention relates to nanoparticles for use according to the invention, wherein at least one of the cooling step, the maintaining step, and the warming step is carried out in the presence of:
i) a percentage of nanoparticles internalized in cells of the body part is in a range from 10⁻³% to 90%,
   and/or
ii) a concentration of nanoparticles in the body part in the range from 10⁻⁹ mg of nanoparticles per cm³ of body part to 10⁹ mg of nanoparticles per cm³ of body part or from 10⁻¹⁰ to 10¹⁰ pg of nanoparticles per cell.

In another embodiment of the invention, the cooling region is the volume, surface or length that is cooled down to the cooling temperature.

In some embodiment, the nanoparticle region can be smaller by a factor of at least 1.01, 1.1, 1.2, 1.5, 2, 5, 10, 10³ or 10⁵ than the cooling region.

In some other embodiment, the nanoparticle region can be larger by a factor of at least 1.01, 1.1, 1.2, 1.5, 2, 5, 10, 10³ or 10⁵ than the cooling region.

In another embodiment of the invention, at least one step of the method is carried out in the presence of a percentage of nanoparticles internalized in cells of the body part, which is larger than 10⁻⁵⁰, 10⁻²⁰, 10⁻¹⁰, 10⁻⁵, 10⁻¹, 0, 1, 5, 10, 50, 75, 80, 90 or 99%

In another embodiment of the invention, at least one step of the method is carried out in the presence of a percentage of nanoparticles internalized in cells of the body part, which is lower than 100, 99, 90, 70, 50, 20, 10, 5, 2, 1, 10⁻¹, 10⁻⁵ or 10⁻¹⁰%.

In another embodiment of the invention, at least one step of the method is carried out in the presence of a percentage of nanoparticles internalized in cells of the body part, which is in a range from 10⁻⁵⁰ to 100%, from 10⁻¹⁰ to 99%, from 10⁻⁵ to 95%, from 10⁻³ to 90%, or from 10⁻¹ to 70%.

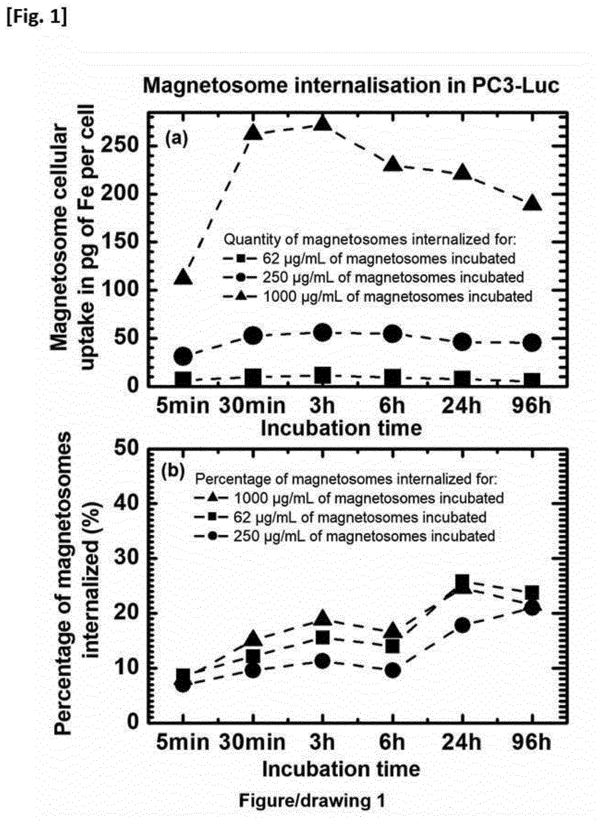

In the experimental example number 2, the percentage of N-CMD internalized in PC3-Luc cells is measured for PC3-Luc cells incubated in the presence of N-CMD for different concentrations of N-CMD (0, 62, 250 and 1000 µg/mL of N-CMD) and different incubation times (5 min, 30 min, 3 h, 6 h, 24h, 96h), where the percentage of internalized N-CMD is the ratio between the quantity of N-CMD inside cells and the quantity of N-CMD incubated with PC3-Luc cells. The percentage of internalized N-CMD varies from 6% to 26% (Figure 1(b)). This percentage increases with increasing N-CMD concentration and with increasing incubation time (Figure 1(b)). In some embodiment, larger values of this percentage can be obtained, preferentially larger by a factor of at least 0, 0.5, 1, 1.5, 2, 5, 10 or 10³, by increasing the nanoparticle concentration or the incubation time, preferentially by a factor of at least 0, 0.5, 1, 1.5, 2, 5, 10 or 10³, or by using a different cell type, and/or by carrying out the internalization in the presence of a radiation. In some other embodiment, lower values of this percentage can be obtained, preferentially lower by a factor of at least 0, 0.5, 1, 1.5, 2, 5, 10 or 10³, by decreasing the nanoparticle concentration or the incubation time, preferentially by a factor of at least 0, 0.5, 1, 1.5, 2, 5, 10 or 10³, or by using a different cell type.

In one aspect, the invention relates to nanoparticles for use according to the invention, wherein at least one of the cooling step, the maintaining step, and the warming step is carried out when nanoparticles occupy a portion of the body part, which is between 10⁻⁵% and 90% by mass or volume of the body part as a whole.

In one embodiment of the invention, the cooling step, maintaining step, and/or warming step is/are carried out on nanoparticles occupying a portion of the body part, which is smaller than 99.9, 90, 80, 70, 60, 50, 30, 20, 10, 5, 2 or 1% by mass or volume of the body part as a whole.

In one embodiment of the invention, the cooling step, maintaining step, and/or warming step is/are carried out on nanoparticles occupying a portion of the body part, which is larger than 10⁻¹⁰, 10⁻⁵, 1, 2, 5, 10, 20, 30, 50, 60, 70, 80, 90 or 99% by mass or volume of the body part as a whole.

In one embodiment of the invention, the cooling step, maintaining step, and/or warming step is carried out on nanoparticles occupying a portion of the body part, which is smaller than 99.9, 90, 80, 70, 60, 50, 30, 20, 10, 5, 2 or 1% by mass or volume of the body part as a whole.

In one embodiment of the invention, the cooling step, maintaining step, and/or warming step is carried out on nanoparticles occupying a portion of the body part, which is larger than 10⁻¹⁰, 10⁻⁵, 1, 2, 5, 10, 20, 30, 50, 60, 70, 80, 90 or 99% by mass or volume of the body part as a whole.

In another embodiment of the invention, the cooling region is the volume, surface or length that is cooled down to the minimum temperature.

In some cases, the nanoparticle region can be smaller by a factor of at least 1.01, 1.1, 1.2, 1.5, 2, 5, 10, 10³ or 10⁵ than the cooling region.

In some other cases, the nanoparticle region can be larger by a factor of at least 1.01, 1.1, 1.2, 1.5, 2, 5, 10, 10³ or 10⁵ than the cooling region.

In one aspect, the invention relates to nanoparticle for use according to the invention, wherein the individual in need thereof is suffering from an infectious disease, and the body part comprises cells affected by the infectious disease.

In one embodiment of the invention, the treatment or method according to the invention is the treatment of an infectious disease or the treatment of hypothermia.

In one embodiment, when the method is the treatment of hypothermia, it may not involve or comprise the cooling step. It can then consist in treating a body part of an individual, preferentially by non-predominant ice-ball cryotherapy, using the following steps:
a) administering nanoparticles to a body part of an individual;
   and
b) Warming the body part comprising the nanoparticles by increasing the temperature of the body part from the cooling temperature of said body part to a final temperature of said body part that is above the cooling temperature,
   characterized in that said cooling temperature of the body part is preferentially the temperature reached by the body part or individual when it is in hypothermia.

In one embodiment of the invention, the temperature reached by the individual when it is in state of hypothermia is preferentially a temperature that is lower than 37 °C or lower than the temperature of the individual without or before being in hypothermia by at least 10⁻¹, 1, 5, 10, 20, 50, 100 or 150 °C.

In one embodiment of the invention, the method according to the invention enables to control, adjust, decrease or increase the rate at which the individual in hypothermia is warmed up, hence preferentially enabling to save the life of the individual.

In one embodiment of the invention the infectious disease is due to, originates from, or is associated with the presence in the body part of: i), bacteria, preferentially pathological bacteria, ii), viruses, iii), tumor cells or, iv), foreign biological material not belonging to the living organism or body part.

In one embodiment of the invention, the disease is selected from the group consisting of: a malfunction of the living organism or body part, a disease associated with a proliferation of cells that is different from the cellular proliferation in a healthy individual, a disease associated with the presence of pathological cells in the body part, a disease associated with the presence of a pathological site in an individual or body part, a disease or disorder or malfunction of the body part, a disease associated with the presence of radio-resistant or acoustic-resistant cells, an infectious disease, an auto-immune disease, a neuropathology, a cancer, a tumor, a disease comprising or due to at least one cancer or tumor cell, a cutaneous condition, an endocrine disease, an eye disease or disorder, an intestinal disease, a communication disorder, a genetic disorder, a neurological disorder, a voice disorder, a vulvovaginal disorder, a liver disorder, a heart disorder, a heating disorder, a mood disorder, anemia, preferentially iron anemia, and a personality disorder.

In one embodiment of the invention, the cancer or tumor is selected from the group consisting of: the cancer of an organ, cancer of blood, cancer of a system of a living organism, adrenal cancer, anal cancer, bile duct cancer, bladder cancer, bone cancer, brain cancer, breast cancer, cervical cancer, colon/rectum cancer, endometrial cancer, esophagus cancer, eye cancer, gallbladder cancer, heart cancer, kidney cancer, laryngeal and hypopharyngeal cancer, leukemia, liver cancer, lung cancer, nasal cavity and paranasal sinus cancer, nasopharyngeal cancer, neuroblastoma, non-Hodgkin lymphoma, oral cavity and oropharyngeal cancer, osteosarcoma cancer, ovarian cancer, pancreatic cancer, pancreatic penile cancer, prostate cancer, retinoblastoma, rhabdomyosarcoma, salivary gland cancer, sarcoma, skin cancer, small intestine cancer, stomach cancer, testicular cancer, thymus cancer, thyroid cancer, uterine cancer, uterine sarcoma cancer, vaginal cancer, vulvar cancer, waldenstrom macroglobulinemia wilms tumor, castleman disease ewing family of tumor, gastrointestinal carcinoid tumor, gastrointestinal stromal tumor, myelodysplastic syndrome pituitary tumor, and a cancerous disease such as gestational trophoblastic disease, Hodgkin disease, kaposi sarcoma, malignant mesothelioma, and multiple myeloma.

In one embodiment of the invention, the disorder or malfunction of the body part is associated with the malfunction of cells, which divide more rapidly or enter in an apoptotic or necrotic state for example, or with the malfunction of the immune system or immune cell(s).

In one embodiment of the invention, the method according to the invention is a method, which preferentially detects diagnoses, heals, or cures a disease such as one of those mentioned in the previous embodiments.

In one aspect, the invention relates to nanoparticles for use according to the invention, wherein the nanoparticles have a heating capacity with at least one property selected from the group consisting of:
i) the heating capacity is comprised between 10⁻¹⁰ and 10¹⁰ °C per second per mg of nanoparticle,
ii) the heating capacity is the difference between the heating rate of the body part comprising the nanoparticles and the heating rate of the body part without the nanoparticles,
iii) the heating capacity is measured during the heating step,
iv) the heating capacity can be increased by applying an external source of radiation such as an acoustic wave, laser, or magnetic field on the body part or nanoparticle, and
v) when the heating capacity is increased, the nanoparticles more efficiently destroy cells.

In one embodiment of the invention, the nanoparticle heating capacity is the number of degrees Celsius gained by the nanoparticles, preferentially per second, preferentially per mg of nanoparticles, preferentially during the heating step.

In some embodiment, the nanoparticle heating capacity is estimated without exposing the nanoparticles to a source of radiation, preferentially a source of radiation that produces heat.

In some embodiment, the nanoparticle heating capacity is estimated by exposing the nanoparticles to a source of radiation, preferentially a source of radiation that produces heat. Such conditions may increase the value of the heating capacity compared with the heating capacity measured in the absence of the application of a source of radiation.

In another embodiment of the invention, the heating capacity is the difference between the number of degrees Celsius gained by the body part comprising the nanoparticles and the number of degrees gained by the body part not comprising the nanoparticles. The number of degrees that is gained is preferentially estimated per second, preferentially per mg of nanoparticles, preferentially during the heating step.

In an embodiment of the invention, the nanoparticle heating capacity is lower than 10⁵⁰, 10²⁰, 10¹⁰, 10⁵, 10³, 10, 1, 10⁻¹, 10⁻³ or 10⁻⁵ °C, preferentially per second, preferentially per mg of nanoparticle.

In another embodiment of the invention, the nanoparticle heating capacity is larger than 10⁻⁵⁰, 10⁻¹⁰, 10⁻⁵, 10⁻¹, 1, 5, 10 or 10³ °C, preferentially per second, preferentially per mg of nanoparticle.

In still another embodiment of the invention, the nanoparticle heating capacity is between 10⁻⁵⁰ and 10⁵⁰, 10⁻²⁰ and 10²⁰, 10⁻¹⁰ and 10¹⁰, or between 10⁻⁵ and 10⁵ °C, preferentially per second, preferentially per mg of nanoparticle.

In still another embodiment of the invention, the nanoparticle heating capacity is the nanoparticle heating rate, preferentially of or during the warming step.

In another embodiment of the invention, the nanoparticle cooling capacity is the number of degrees Celsius lost by the nanoparticles, preferentially per second, preferentially per mg of nanoparticles, preferentially during the cooling step.

In some embodiment, the cooling capacity is estimated without exposing the nanoparticles to the substance or equipment that enables to adjust the temperature, preferentially during the cooling step or maintaining step.

In some embodiment, the cooling capacity is estimated by exposing the nanoparticles to the temperature adjuster, preferentially during the cooling or maintaining step. Such conditions may increase the value of the nanoparticle cooling capacity compared with the nanoparticle cooling capacity measured in the absence of the temperature adjuster.

In one embodiment of the invention, the efficacy to treat and/or destroy a disease increases with increasing heating and/or cooling capacity of the nanoparticles.

In another embodiment of the invention, the cooling capacity is the difference, preferentially in absolute value, between the number of degrees lost by the body part comprising the nanoparticles and the number of degrees lost by the body part not comprising the nanoparticles. The number of degrees that is lost is preferentially estimated per second, preferentially per mg of nanoparticles, preferentially during the cooling step.

In an embodiment of the invention, the nanoparticle cooling capacity is lower than 10⁵⁰, 10²⁰, 10¹⁰, 10⁵, 10³, 10, 1, 10⁻¹, 10⁻³ or 10⁻⁵ °C, preferentially per second, preferentially per mg of nanoparticle.

In another embodiment of the invention, the nanoparticle cooling capacity is larger than 10⁻⁵⁰, 10⁻¹⁰, 10⁻⁵, 10⁻¹, 1, 5, 10 or 10³ °C, preferentially per second, preferentially per mg of nanoparticle.

In still another embodiment of the invention, the nanoparticle cooling capacity is between 10⁻⁵⁰ and 10⁵⁰, 10⁻²⁰ and 10²⁰, 10⁻¹⁰ and 10¹⁰, or between 10⁻⁵ and 10⁵ °C, preferentially per second, preferentially per mg of nanoparticle.

In another embodiment of the invention, the nanoparticle cooling capacity is the nanoparticle cooling rate, preferentially of or during the cooling step.

In one aspect, the invention relates to nanoparticles for use according to the invention, wherein the nanoparticles are magnetosomes.

In one embodiment of the invention, the magnetosomes are nanoparticles synthesized by, comprised in, originating from, extracted from, or isolated from magnetotactic bacteria.

In one embodiment of the invention, magnetotactic bacteria are selected from the group consisting of: Magnetospirillum magneticum strain AMB-1, magnetotactic coccus strain MC-1, three facultative anaerobic vibrios strains MV-1, MV-2 and MV-4, the Magnetospirillum magnetotacticum strain MS-1, the Magnetospirillum gryphiswaldense strain MSR-1, a facultative anaerobic magnetotactic spirillum, Magnetospirillum magneticum strain MGT-1, an obligate anaerobe, Desulfovibrio magneticus RS-1, Nitrospira, Nitrospira moscoviensis, Magnetobacterium bavaricum, Desulfovibrio magneticus RS-1, Desulfovibrio desulfuricans, Geobacter metallireducens, δ-Protobacteria, MMP5, MMP2, where MM designates magnetotactic many-celled prokaryote, magnetic cossus, MC-1, CS103, NKMC5, α-Protobacteria, Rhodospirillum rubrum, Agrobacterium vitis, Magnetospirillum magnetotacticum MS-1, Magnetospirillum magneticum AMB-1, Magnetospirillum magneticum MGT-1, Magnetospirillum gryphiswaldense MSR-1, marine magnetic vibrio MV-1, Magnetospirillum gryphiswaldense MSR- 1, Magnetospirillum magneticum AMB-1, Magnetospirillum magnetotacticum MS-1, Magnetospirillum magneticum strain MGT-1, magnetotactic coccus strain MC-1, Desulfovibrio magneticus RS-1, anaerobic vibrio strains, MV-1, MV-2, and MV-4.

In one embodiment of the invention, a magnetotactic bacterium is defined as a bacterium able to synthesize magnetosomes, wherein these magnetosomes are preferentially characterized by at least one of the following properties: i) they are produced intracellularly, ii) they are magnetic, iii) they comprise a mineral, iv) their core is preferentially composed of a metallic oxide such as iron oxide, v) their core is surrounded by biological material such as lipids, proteins, endotoxins, which can preferentially be removed, vi) they are arranged in chains, vii) they produce heat under the application of an alternating magnetic field.

In one embodiment of the invention, the magnetosomes possess one or several property(ies) in common with the nanoparticles such as at least one magnetic, size, composition, chain arrangement, charge, core, mineral, coating, or crystallinity property.

In one embodiment of the invention, magnetosomes comprise the mineral part synthesized by magnetotactic bacteria, *i.e.* preferentially the crystallized iron oxide produced by these bacteria. In this case, magnetosomes or magnetosome mineral parts preferentially do not comprise proteins, lipids, endotoxins, or biological materials comprising carbon or do not comprise more or comprise less than 0.1, 1, 10, 30, 50 or 75% or percent in mass of carbon, which is/are produced by these bacteria.

The invention also relates to nanoparticles for use, wherein nanoparticles are or are assimilated to chemical analogues of magnetosomes.

In some cases, chemical analogues of magnetosomes can be synthesized chemically and/or are not synthesized by magnetotactic bacteria.

In some cases, chemical analogues of magnetosomes possess at least 1, 2, 3, 4, 5, 6, 7, 8, 9, or 10 common property(ies) with the magnetosomes, where these common properties are preferentially a ferrimagnetic behavior, preferentially a coercivity larger that 10⁻⁵⁰, 10⁻¹⁰, 10⁻², 1, 5, 10 or 100 Oe at a temperature preferentially larger than 0, 5, 10, 50, 100, 200, 300, 500 or 1000 K, a large size, preferentially a size larger than 1, 5, 10, 20, 50 or 70 nm, and/or a chain arrangement, preferentially an arrangement of more than 1, 2, 5 or 10 nanoparticles in chain.

In one embodiment of the invention, the nanoparticles or magnetosomes are purified to remove more than 10, 50 or 90 percent or percent in mass of endotoxins and/or other biological material such as proteins or lipids originating from the synthetizing living organism or magnetotactic bacteria. In some other cases, the nanoparticles or magnetosomes are purified to remove less than 100, 99.9, 99, 95 or 90 percent or percent in mass of endotoxins and/or other biological material. This purification step preferentially yields purified nanoparticles or magnetosomes. In some cases, this percentage can be equal to (Q_{BP}-Q_{AP})/Q_{BP} or Q_{AP}/Q_{BP}, where Q_{BP} and Q_{AP} are the quantities of endotoxins, biological material, proteins, or lipids before and after the purification step, respectively.

In some cases, the purification step can consist in using a method or detergent(s) such as NaOH and/or KOH, which is/are preferentially mixed with the synthetizing living organism or magnetotactic bacteria or bacterial debris, preferentially to remove organic material or separate the organic material from the inorganic material comprised in the nanoparticles or magnetosomes and preferentially then be able to harvest the nanoparticle or magnetosome mineral, preferentially comprised in the nanoparticles or magnetosomes.

In some cases, the purified nanoparticles or magnetosomes are nanoparticle or magnetosome minerals.

In one embodiment of the invention, the invention relates to a method according to the invention comprising the following steps:
a) administering nanoparticles to a medium;
b) cooling the medium comprising the nanoparticles by decreasing the temperature of body part from an initial temperature of said medium, to a cooling temperature of the body part, which is lower than the initial temperature;
c) optionally not maintaining the medium comprising the nanoparticles at the cooling temperature for a duration of time of more than x seconds;
   and
d) warming the medium comprising the nanoparticles by increasing the temperature of the body part from the cooling temperature to a final temperature above the cooling temperature,
characterized in that said cooling temperature of the medium is:
- above the freezing temperature of the body part or above 5, 0 or -5°C, preferably above -5 or 0°C, more preferably above 0°C;
and/or
- above a threshold temperature - above a threshold temperature, where the threshold temperature has at least one property selected from the group consisting of:
   i) above the threshold temperature, the body part comprises a quantity of ice-balls smaller than the quantity of nanoparticles;
   ii) above the threshold temperature, the volume occupied by ice-balls in the body part is smaller than the volume occupied by the nanoparticles in the body part;
   iii) below the threshold temperature, the body part comprises a quantity of ice-balls larger than the quantity of nanoparticles;
   iv) below the threshold temperature, the volume occupied by ice-ball in the body part is larger than the volume occupied by the nanoparticles in the body part; and
   v) below the threshold temperature, the size or diameter of at least one ice-ball is larger than the size or diameter of at least one nanoparticle.

In one embodiment of the invention, the medium is a body part or has at least one property in common with a body part.

In another embodiment of the invention, the medium is different from a body part or has at least one property that is different from a property of a body part.

In one embodiment of the invention, a medium that is different from a body part can be a medium that does not belong, is not synthesized, and/or does not originate from a living organism and/or individual. Such medium can be an assembly of substances or compounds, preferentially chemical ones, which is preferentially the environment of the nanoparticle.

In one embodiment of the invention, the environment of the nanoparticle(s) is a liquid, solid or at least 1, 10, 10², 10³, 10⁶, 10¹⁰ or 10⁴⁰ substance(s), preferentially one or more substance(s) different from the compound, which surround(s) or include(s) the nanoparticle over a distance measured from the center or the outer surface of the nanoparticle preferably lower than 1 m, 1 dm, 1 cm, 1 mm, 1 µm, 100 nm, or 10 nm.

In one embodiment of the invention, the environment of the nanoparticle(s) is a liquid, solid or at least 1, 10, 10², 10³, 10⁶, 10¹⁰, or 10⁴⁰ substance(s), preferentially one or more substance(s) different from the compound, which surround(s) or include(s) the particle over a distance measured from the center or the outer surface of the particle preferably larger than 1 m, 1 dm, 1 cm, 1 mm, 1 µm, 100 nm, or 10 nm.

In one embodiment of the invention, a substance of the environment of the nanoparticle according to the invention may be an atom, a molecule, a polymer, a chemical or biological substance, preferentially a substance different from the compound or nanoparticle, DNA, RNA, a protein, a lipid, an enzyme, or a nucleic or amino acid contained in this environment.

In one embodiment of the invention, the environment of the nanoparticle according to the invention may be a biological environment, that is to say an environment comprising at least one biological substance such as a cell, an organelle, DNA, RNA, a protein, a lipid, an amino acid, or a nucleic acid.

In an embodiment of the invention, the nanoparticles according to the invention are drugs, medical devices, cosmetic products, biological products, products used for research purposes, or products used to determine the properties of biological samples.

In one embodiment of the invention, the initial temperature, the cooling temperature, and the final temperature are temperatures measured or occurring or reached in: i), the body part, or ii), an environment or region of the body part.

In another embodiment of the invention, the body part is the body part treated by the method according to the invention.

In still another embodiment of the invention, the property(ies) or features, preferentially of the nanoparticle(s) or method or treatment, described in each individual embodiment or section or sentence of this patent application can be combined to result in a combination of property(ies) or features, preferentially of the nanoparticle(s) or method or treatment.

In still another embodiment of the invention, when an entity such as the compound, substance, nanoparticle, radiation, has a property with a value of P₁ that is higher, longer, or larger by a factor α than a property with a value of P₂, it means that: P1=α.P2 (α preferentially larger than 1) or P1=α+P2 (α preferentially larger than 0).

In still another embodiment of the invention, when an entity such as the compound, substance, nanoparticle, radiation, has a property with a value of P₁ that is lower, smaller, or shorter by a factor β than a property with a value of P₂, it means that: P₁=β.P₂ (β is preferentially smaller than 1), P₁=P₂/β (β is preferentially larger than 1), P₁=P₂-β (β is preferentially larger than 0) or P₁=β- P₂.

In one aspect of the invention, the invention relates to magnetosomes for use in a method according to the invention comprising the following steps:
a) administering magnetosomes to a medium or body part;
b) cooling the medium or body part comprising the magnetosomes by decreasing the temperature of medium of body part from an initial temperature of said medium or body part, to a cooling temperature of the medium or body part, which is lower than the initial temperature;
c) optionally not maintaining the medium or body part comprising the magnetosomes at the cooling temperature for a duration of time of more than 100 seconds;
   and
d) warming the medium or body part comprising the magnetosomes by increasing the temperature of the body part or medium from the cooling temperature to a final temperature above the cooling temperature.

The invention will be further described by the following non-limiting figures and examples.

### FIGURES

**Figure 1****:** (a), Quantity of N-MCD (magnetosome minerals coated with CMD) inside PC3-Luc cells, estimated in pg of Fe per cell, when various quantity of N-MCD (62 µg/mL, 250 µg/mL, or 1000 µg/mL of magnetosomes) are brought into contact with PC3-Luc cells during incubation times of 5 minutes, 30 minutes, 3 hours, 6 hours, 24 hours, and 96 hours. (b), Percentage of N-MCD internalized in cells when various quantity of N-MCD (62 µg/mL, 250 µg/mL, or 1000 µg/mL of magnetosomes) are brought into contact with PC3-Luc cells during incubation times of 5 minutes, 30 minutes, 3 hours, 6 hours, 24 hours, and 96 hours. This percentage is the ratio between the quantity of N-MCD in iron inside cell and the quantity of N-MCD in iron that is incubated with cells.

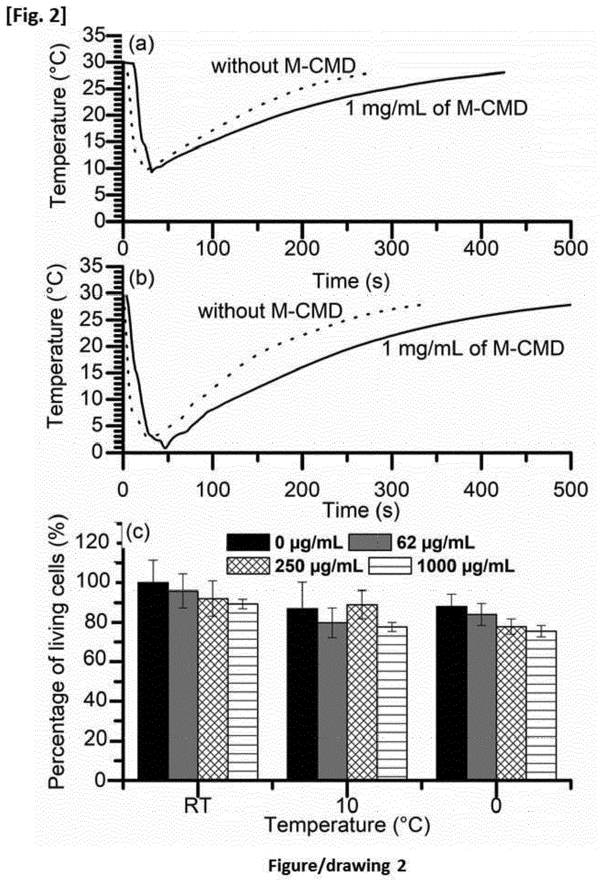

**Figure 2****:** (a) Temperature variation of PC3-Luc cells without N-CMD or PC3-Luc cells incubated with 1 mg/mL of N-CMD during 3 hours that are cooled down from room temperature (RT) to Tₘᵢₙ of 10 °C and then let warming up from Tₘᵢₙ to room temperature. (b), Temperature variation of PC3-Luc cells without N-CMD or PC3-Luc cells incubated with 1 mg/mL of N-CMD during 3 hours that are cooled down from RT to Tₘᵢₙ of 0 °C and then let warming up from Tₘᵢₙ to room temperature. (c) Percentages of living cells when PC3-Luc cells are incubated during 3 hours with only the growth medium (0 mg/mL) or with 62 µg/mL, 250 µg/mL, or 1000 µg/mL of N-CMD and are either maintained to RT (RT), cooled down from RT to Tₘᵢₙ of 10 °C and let warming up from Tₘᵢₙ to RT (10 °C), cooled down from RT to Tmin of 0°C and let warming up from Tmin to RT (0 °C).

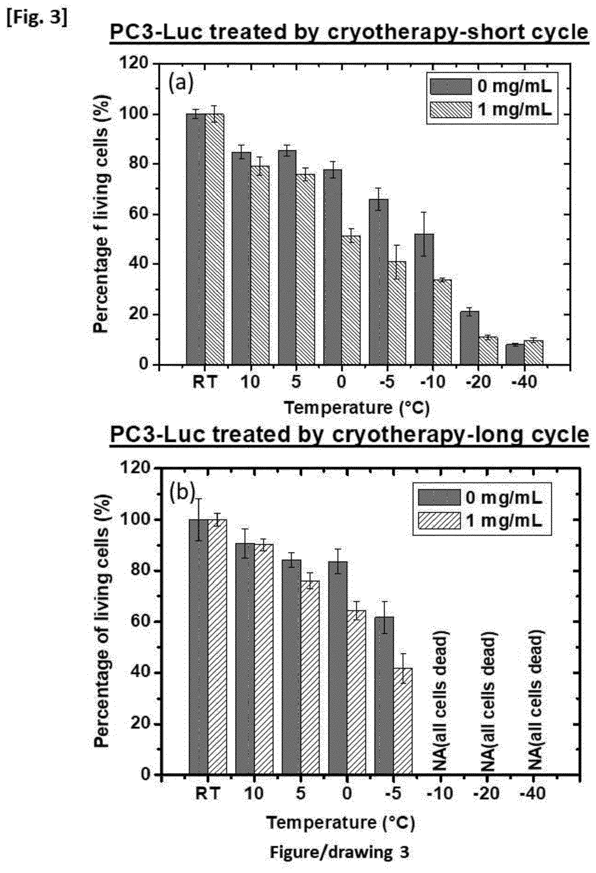

**Figure 3****:** (a) Percentages of living cells when PC3-Luc cells are incubated during 3 hours with only the growth medium (0 mg/mL) or with 1 mg/mL of N-CMD (1 mg/mL) and are either maintained to RT (RT), cooled down from RT to Tₘᵢₙ of 10 °C and let warming up from Tmin to RT, cooled down from RT to Tmin of 5°C and let warming up from Tmin to RT, cooled down from RT to Tmin of 0°C and let warming up from Tₘᵢₙ to RT, cooled down from RT to Tₘᵢₙ of -5°C and let warming up from Tₘᵢₙ to RT, cooled down from RT to Tₘᵢₙ of -10°C and let warming up from Tₘᵢₙ to RT, cooled down from RT to Tₘᵢₙ of -20°C and let warming up from Tₘᵢₙ to RT, cooled down from RT to Tₘᵢₙ of -40°C and let warming up from Tₘᵢₙ to RT. In (a), the cycles are short. This means that the temperature is maintained at Tₘᵢₙ for less than 10 seconds. (b) Percentages of living cells when PC3-Luc cells are incubated during 3 hours with only the growth medium (0 mg/mL) or with 1 mg/mL of N-CMD (1 mg/mL) and are either maintained to RT (RT), cooled down from RT to Tₘᵢₙ of 10 °C and let warming up from Tₘᵢₙ to RT, cooled down from RT to Tₘᵢₙ of 5°C and let warming up from Tₘᵢₙ to RT, cooled down from RT to Tₘᵢₙ of 0°C and let warming up from Tₘᵢₙ to RT, cooled down from RT to Tₘᵢₙ of -5°C and let warming up from Tmin to RT, cooled down from RT to Tₘᵢₙ of -10°C and let warming up from Tₘᵢₙ to RT, cooled down from RT to Tₘᵢₙ of -20°C and let warming up from Tₘᵢₙ to RT, cooled down from RT to Tₘᵢₙ of -40°C and let warming up from Tₘᵢₙ to RT. In (b), the cycles are long. This means that the temperature is maintained at Tₘᵢₙ for more than 1 minute.

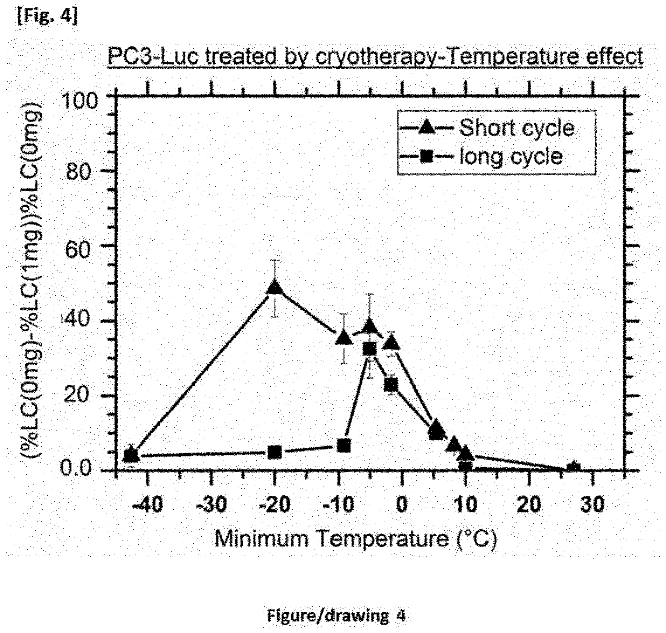

**Figure 4****:** Value of [%LC(Omg)-%LC(1mg)]/%LC(0mg) as a function of minimal temperatures reached during the various treatments (RT, 10°C, 5 °C, 0°C, -5°C, - 10°C, -20 °C, -40 °C), where %LC(0mg) and %LC(1mg) are the percentages of living cells obtained when PC3-Luc cells are incubated only with the cellular growth medium during 3 hours (0 mg) and PC3-Luc cells are incubated with 1 mg/mL of N-CMD during 3 hours (1 mg).

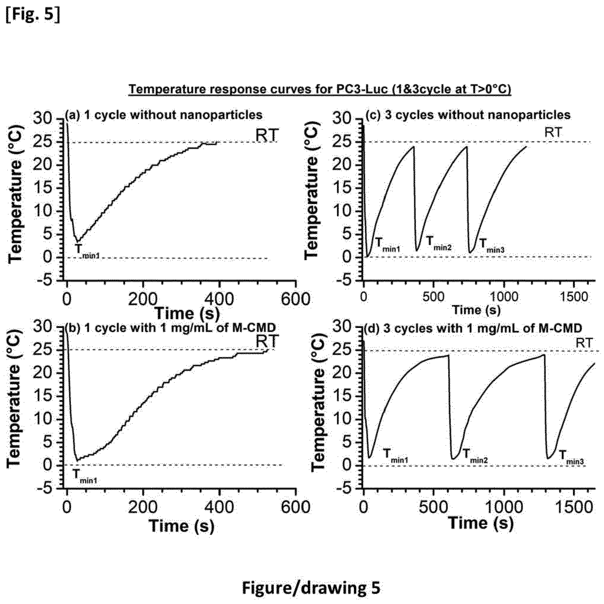

**Figure 5****:** (a) Temperature variation of PC3-Luc cells incubated without N-CMD during 3 hours, cooled down from room temperature (RT) to Tₘᵢₙ of 3 °C and then let warming up from Tₘᵢₙ to room temperature. (b) Temperature variation of PC3-Luc cells incubated with 1 mg/mL of N-CMD during 3 hours, cooled down from room temperature (RT) to Tₘᵢₙ of 1 °C, and then let warming up from Tₘᵢₙ to room temperature. (c), Temperature variation of PC3-Luc cells incubated with growth medium without N-CMD during 3 hours, cooled down from RT to Tₘᵢₙ₁ of 0.2 °C and then let warm up from Tₘᵢₙ₁ of 0.2 °C to room temperature (first cycle), cooled down from RT to Tₘᵢₙ₂ of 2 °C and then let warming up from Tₘᵢₙ₂ of 2 °C to RT (second cycle), cooled down from RT to Tₘᵢₙ₃ of 1 °C and then let warming up from Tₘᵢₙ₃ to RT (third cycle). (d) Temperature variation of PC3-Luc cells incubated during 3 hours with 1 mg/mL of N-CMD, cooled down from RT to Tₘᵢₙ₁ of 2 °C and let warming up from Tₘᵢₙ₁ of 2 °C to RT (first cycle), cooled down from RT to Tₘᵢₙ₂ of 1 °C and let warming up from Tₘᵢₙ₂ of 1 °C to RT (second cycle), cooled down from RT to Tₘᵢₙ₃ of 2 °C and let warming up from Tₘᵢₙ₃ of 2 °C to RT (third cycle).

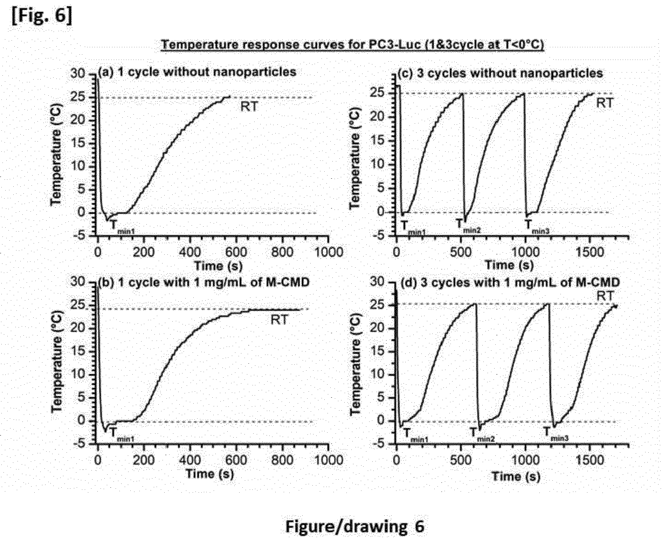

**Figure 6****:** (a) Temperature variation of PC3-Luc cells incubated without N-CMD during 3 hours, cooled down from room temperature (RT) to Tₘᵢₙ of -2 °C and then let warming up from Tₘᵢₙ of -2 °C to room temperature. (b) Temperature variation of PC3-Luc cells incubated with 1 mg/mL of N-CMD during 3 hours, cooled down from room temperature (RT) to Tₘᵢₙ of -2 °C, and then let warming up from Tₘᵢₙ of -2 °C to room temperature. (c), Temperature variation of PC3-Luc cells incubated with growth medium without N-CMD during 3 hours, cooled down from RT to Tₘᵢₙ₁ of - 1 °C and then let warming up from Tₘᵢₙ₁ to room temperature (first cycle), cooled down from RT to Tₘᵢₙ₂ of -2 °C and then let warming up from Tₘᵢₙ₂ of -2 °C to RT (second cycle), cooled down from RT to Tₘᵢₙ₃ of -1 °C and then let warming up from Tₘᵢₙ₃ of -1 °C to RT (third cycle). (d) Temperature variation of PC3-Luc cells incubated during 3 hours with 1 mg/mL of N-CMD, cooled down from RT to Tₘᵢₙ₁ of - 1 °C and let warming up from Tₘᵢₙ₁ of -1 °C to RT (first cycle), cooled down from RT to Tₘᵢₙ₂ of -2°C and let warming up from Tₘᵢₙ₂ of -2°C to RT (second cycle), cooled down from RT to Tₘᵢₙ₃ of -1 °C and let warming up from Tₘᵢₙ₃ of -1 °C to RT (third cycle).

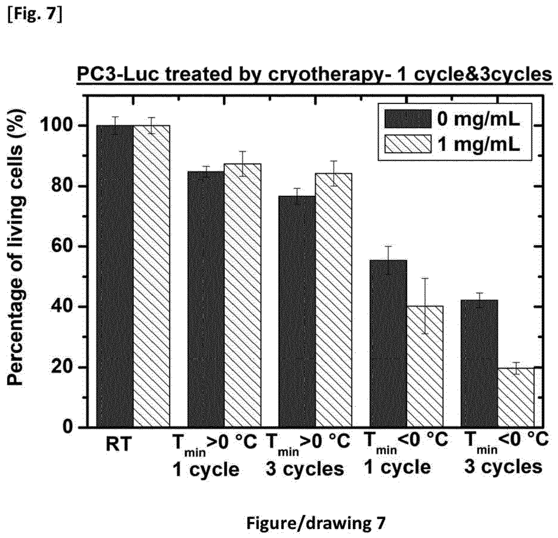

**Figure 7****:** Percentages of living cells resulting from the treatment where PC3-Luc cells are incubated during 3 hours with growth medium without N-CMD (0 mg/mL) or with 1 mg/mL of N-CMD (1 mg/mL), and treated by 1 or 3 cycles at Tmin>0°C (minimum temperatures just above 0 °C) or at Tmin<0°C (minimum temperatures just below 0 °C), where the temperature variations of the different cycles are shown in Figures 5 and 6.

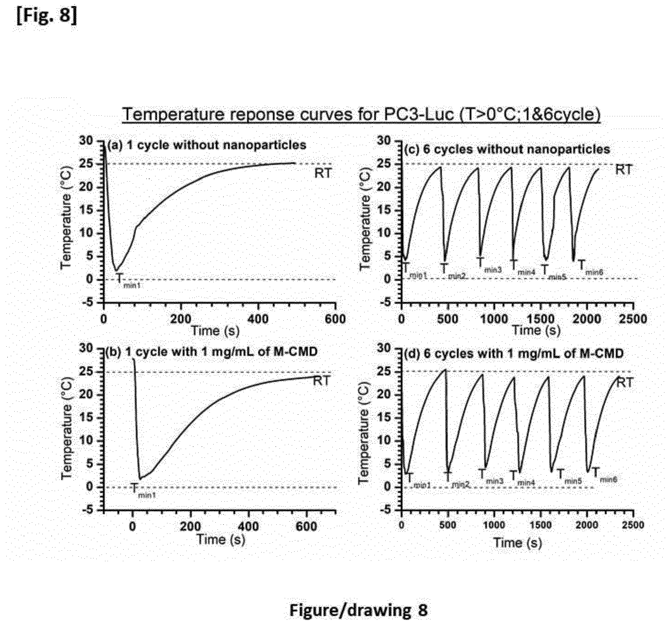

**Figure 8****:** (a) Temperature variation of PC3-Luc cells incubated without N-CMD during 3 hours, cooled down from room temperature (RT) to Tₘᵢₙ of 2 °C and then let warming up from Tₘᵢₙ of 2 °C to room temperature. (b) Temperature variation of PC3-Luc cells incubated with 1 mg/mL of N-CMD during 3 hours, cooled down from room temperature (RT) to Tₘᵢₙ of 2°C, and then let warming up from Tₘᵢₙ of 2°C to room temperature. (c), Temperature variation of PC3-Luc cells incubated with growth medium without N-CMD during 3 hours, cooled down from RT to Tₘᵢₙ₁ of 4 °C and then let warming up from Tₘᵢₙ₁ of 4 °C to room temperature (first cycle), cooled down from RT to Tₘᵢₙ₂ of 4 °C and then let warming up from Tₘᵢₙ₂ of 4 °C to RT, cooled down to Tₘᵢₙ₃ of 4 °C and then let warming up from Tₘᵢₙ₃ of 4 °C to RT (third cycle), cooled down from RT to Tₘᵢₙ₄ of 5 °C and then let warming up from Tₘᵢₙ₄ of 5 °C to room temperature (fourth cycle), cooled down from RT to Tₘᵢₙ₅ of 5 °C and then let warming up from Tₘᵢₙ₅ of 5 °C to RT, cooled down to Tₘᵢₙ₆ of 5 °C and then let warming up from Tₘᵢₙ₆ of 5 °C to RT (sixth cycle). (d) Temperature variation of PC3-Luc cells incubated during 3 hours with 1 mg/mL of N-CMD, cooled down from RT to Tₘᵢₙ₁ of 3 °C and then let warming up from Tₘᵢₙ₁ of 3 °C to room temperature (first cycle), cooled down from RT to Tₘᵢₙ₂ of 3 °C and then let warming up from Tₘᵢₙ₂ of 3 °C to RT, cooled down to Tₘᵢₙ₃ of 4 °C and then let warming up from Tₘᵢₙ₃ of 4 °C to RT (third cycle), cooled down from RT to Tₘᵢₙ₄ of 3 °C and then let warming up from Tₘᵢₙ₄ of 3 °C to room temperature (fourth cycle), cooled down from RT to Tₘᵢₙ₅ of 3 °C and then let warming up from Tₘᵢₙ₅ of 3 °C to RT, cooled down to Tₘᵢₙ₆ of 3 °C and then let warming up from Tₘᵢₙ₆ of 3 °C to RT (sixth cycle).

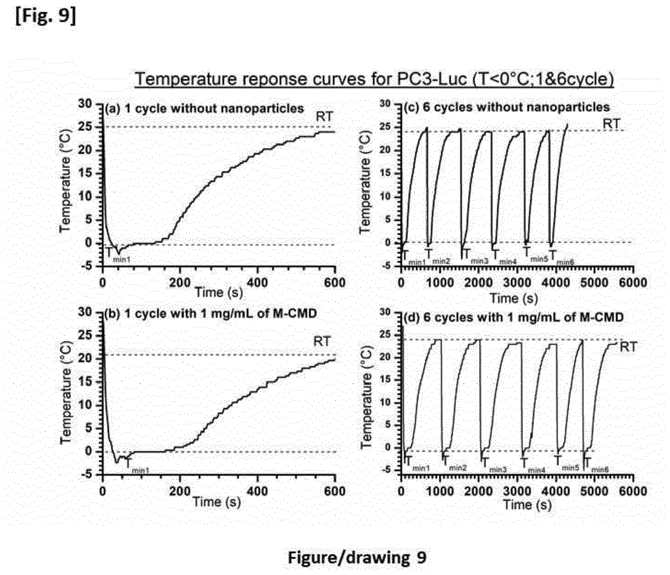

**Figure 9****:** (a) Temperature variation of PC3-Luc cells incubated without N-CMD during 3 hours, cooled down from room temperature (RT) to Tₘᵢₙ of -2 °C and then let warming up from Tₘᵢₙ of -2 °C to room temperature. (b) Temperature variation of PC3-Luc cells incubated with 1 mg/mL of N-CMD during 3 hours, cooled down from room temperature (RT) to Tₘᵢₙ of -2 °C, and then let warming up from Tₘᵢₙ of -2 °C to room temperature. (c), Temperature variation of PC3-Luc cells incubated with growth medium without N-CMD during 3 hours, cooled down from RT to Tₘᵢₙ₁ of - 2 °C and then let warming up from Tₘᵢₙ₁ -2 °C to room temperature (first cycle), cooled down from RT to Tₘᵢₙ₂ of -1 °C and then let warming up from Tₘᵢₙ₂ of -1 °C to RT, cooled down to Tₘᵢₙ₃ of -3 °C and then let warming up from Tₘᵢₙ₃ of -3 °C to RT (third cycle), cooled down from RT to Tₘᵢₙ₄ of -1 °C and then let warming up from Tₘᵢₙ₄ -1 °C to room temperature (fourth cycle), cooled down from RT to Tₘᵢₙ₅ of 0 °C and then let warming up from Tₘᵢₙ₅ of 0 °C to RT, cooled down to Tₘᵢₙ₆ of -1 °C and then let warming up from Tₘᵢₙ₆ of -1 °C to RT (sixth cycle). (d) Temperature variation of PC3-Luc cells incubated during 3 hours with 1 mg/mL of N-CMD, cooled down from RT to Tₘᵢₙ₁ of -3°C and then let warming up from Tₘᵢₙ₁ of -3°C to room temperature (first cycle), cooled down from RT to Tₘᵢₙ₂ of -3 °C and then let warming up from Tₘᵢₙ₂ of -3 °C to RT, cooled down to Tₘᵢₙ₃ of -3 °C and then let warming up from Tₘᵢₙ₃ of -3 °C to RT (third cycle), cooled down from RT to Tₘᵢₙ₄ of -2 °C and then let warming up from Tₘᵢₙ₄ of -2 °C to room temperature (fourth cycle), cooled down from RT to Tₘᵢₙ₅ of -1 °C and then let warming up from Tₘᵢₙ₅ -1 °C to RT, cooled down to Tₘᵢₙ₆ of -5 °C and then let warming up from Tₘᵢₙ₆ of -5 °C to RT (sixth cycle).

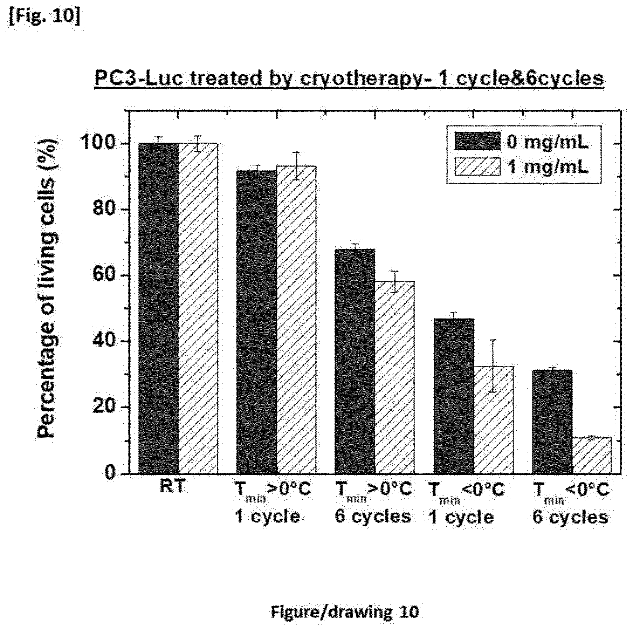

**Figure 10****:** Percentages of living cells resulting from the treatment where PC3-Luc cells are incubated during 3 hours with growth medium without N-CMD (0 mg/mL) or with 1 mg/mL of N-CMD (1 mg/mL), and treated by 1 or 6 cycles at Tmin>0°C (minimum temperatures just above 0 °C) or at Tmin<0°C (minimum temperatures just below 0 °C), where the temperature variations of the different cycles are shown in Figures 8 and 9.

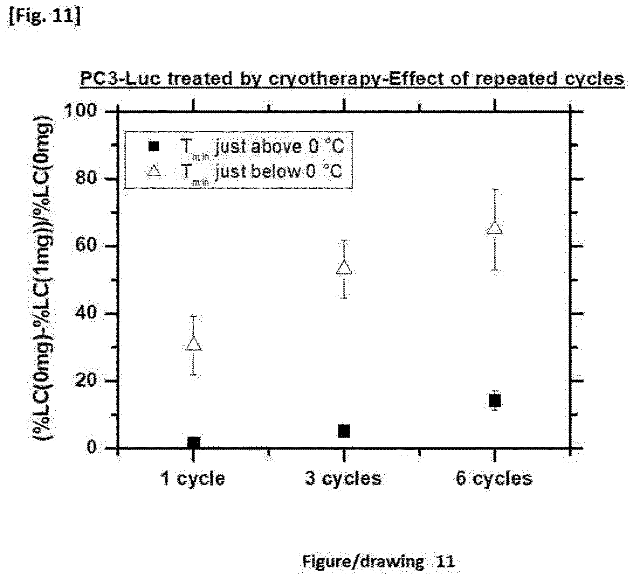

**Figure 11****:** Values of [%LC(0mg)-%LC(1mg)]/%LC(0mg) as a function of the number cycles for Tmin>0°C (minimum temperatures above 0°C) and Tmin<0°C (minimum temperatures below 0 °C), where %LC(0mg) and %LC(1mg) are the percentages of living cells obtained when PC3-Luc cells are incubated only with the cellular growth medium during 3 hours (0 mg) and PC3-Luc cells are incubated with 1 mg/mL of N-CMD during 3 hours (1 mg) and the cells with/without nanoparticles are exposed to 1 cycle, 3 cycles, or 6 cycles.

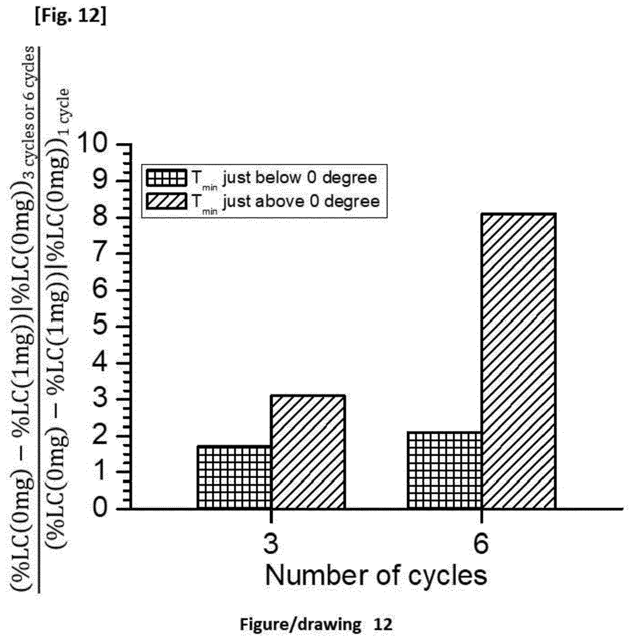

**Figure 12****:** Value of:
   [[%LC(0mg)-%LC(1mg)]/%LC(0mg)]_{3cycle}/[[%LC(0mg)-%LC(1mg)]/%LC(0mg)]_{1cycle} (increase of [%LC(0mg)-%LC(1mg)]/%LC(0mg)] between 1 and 3 cycles)
      and
   [[%LC(0mg)-%LC(1mg)]/%LC(0mg)]_{6cycle}/[[%LC(0mg)-%LC(1mg)]/%LC(0mg)]_{1cycle} (increase of [[%LC(0mg)-%LC(1mg)]/%LC(0mg)] between 1 and 6 cycles)
      estimated for Tmin>0°C (minimum temperatures above 0°C) and Tmin<0°C (minimum temperatures below 0°C), where %LC(0mg) and %LC(1mg) are the percentages of living cells obtained when PC3-Luc cells are incubated only with the cellular growth medium during 3 hours (0 mg) and PC3-Luc cells are incubated with 1 mg/mL of N-CMD during 3 hours (1 mg) and the cells with/without nanoparticles are exposed to 1 cycle (%LC(0mg)_{1cycle} and %LC(1mg)_{1cycle}), 3 cycles (%LC(0mg)_{3cycle} and %LC(1mg)_{3cycle}), or 6 cycles (%LC(0mg)_{6cycle} and %LC(1mg)_{6cycle}).

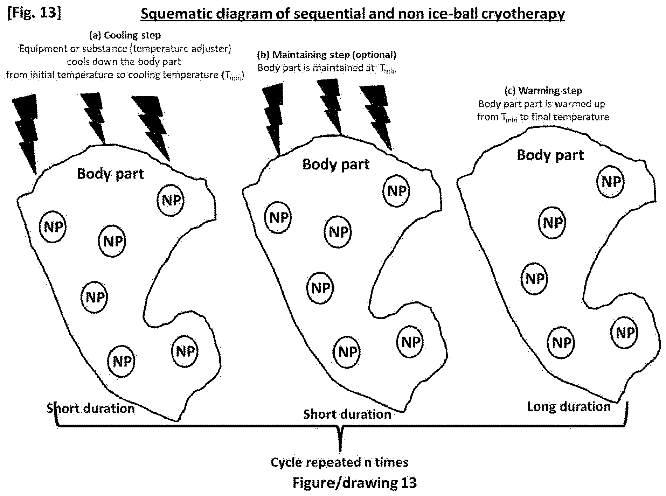

**Figure 13****:** Schematic diagram illustrating how the method according to the invention can be implemented. A cycle is divided in three steps. The body part comprising the nanoparticles is first cooled down from an initial temperature to Tₘᵢₙ, which is preferentially above 0°C, during the cooling step, preferentially of short duration, using an equipment or substance (temperature adjuster) that cools down the body part to Tₘᵢₙ. The temperature of the body part comprising the nanoparticles is optionally maintained at Tₘᵢₙ during the maintaining step, preferentially of short duration. The temperature of the body part comprising the nanoparticles is then increased during the warming step, preferentially of long duration, from Tₘᵢₙ, preferentially above 0 °C, to the final temperature. A cycle can be repeated n times, preferentially until the desired medical, therapeutic or medical or cosmetic or therapeutic or diagnostic is reached. The body part is indicated by the large truncated cylinder. Nanoparticles are indicated by circles inside which NP is inserted. The use of temperature adjuster during the cooling and maintaining steps is indicated by thunder signs.

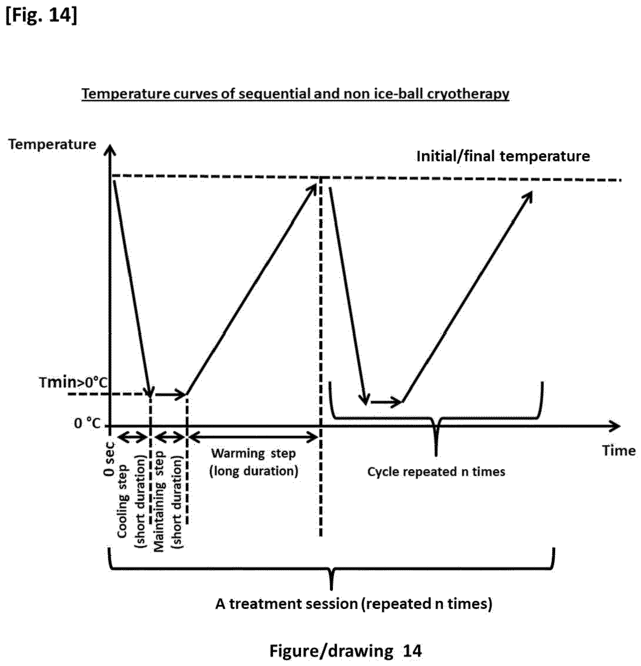

**Figure 14****:** Schematic diagram illustrating how the temperature can vary as a function of time during the different steps of two cycles and one session of the method according to the invention. The different steps of the method are described in the legend of Figure 13.

### Example 1: Preparation of magnetosomes coated with carboxy-methyl dextran (N-CMD).

Growth of MSR-1 magnetotactic bacteria. *Magnetospirillum gryphiswaldense* strain MSR-1 (DSM6361) was purchased from Deutsche Sammlung von Mikro-organismen und Zellkulturen (Brunswick, Germany). First, MSR-1 cells were deposited on a solid activated charcoal agar medium, containing 20 mM filtered iron citrate and 100 mM DTT (1,4-dithiothreitol) and incubated at 29 °C under microaerobic conditions for 7 days. Then, several black-brown colonies were collected from the solid agar medium and were cultivated and amplified at 29 °C under stirring. Cells were then introduced in a 35 L fermentation medium. Fermentation was carried out at 29-30 °C under agitation at 200 rpm for 5 days. During fermentation, the pH was maintained at 6.9 by adding an acidic feeding medium containing in 1 l of medium 118 ml of 85% lactic acid, 18 ml of 25% to 28% ammonia, 2.4 g of magnesium sulfate, 6 g of potassium phosphate, 0.2 ml of propylene glycol, 6 g of yeast extract and 7 ml of mineral elixir containing an iron source. The growth of magnetotactic bacteria was stimulated by bubbling oxygen in the growth medium. The temperature, agitation speed, pH, feeding pump flow and oxygen concentration were monitored and adjusted using an ez-Controller and BioXpert software from Applikon Biotechnology. After fermentation, MSR-1 cells were concentrated and washed in water using tangential flow filtration. To lyse the bacteria and obtain a suspension containing pyrogenic MC, concentrated MSR-1 cells were resuspended in 5 M NaOH, and heated at 60 °C for 2 hours. Then they were sonicated four times in the presence of a solution of PBS at 10 W for 20 s to remove all lysed bacterial cells that remain. This extraction step for isolating magnetosomes from MSR-1 cells was carried out about three times. The results were obtained from three replicates. MC then underwent the following four treatments: (i) they were re-suspended in a solution containing 1% Triton X-100 and 1% SDS and were then heated at 50 °C overnight; (ii) they were mixed in phenol at pH 8 and then heated at 60 °C for 2 hours in a 25 kHz sonicating bath (SB); (iii) they were re-suspended in chloroform and heated at 60 °C for 2 hours; and (iv) they were mixed with a 1M NaOH solution and heated at 60 °C for 1 hour in the SB, to remove all proteins and lipids. After bacterial lysis and each of the five treatments with detergents, magnetosomes were isolated from non-magnetic organic debris using a neodymium magnet. The supernatant was then removed and replaced by a detergent. Uncoated magnetosome minerals labeled N containing a low percentage of residual organic materials were thus obtained. They were autoclaved and stored at -80 °C. The coating procedure was carried out under sterile conditions, using a sterile flow hood. To prepare the suspension of N-CMD, a solution containing 840 mg of carboxy-methyl dextran (CMD) powder dissolved in 12 ml of pyrogen-free water was first prepared. It was filtered with a polyether sulfone filter of 0.2 mm and its pH values was adjusted to 4.1. 1.5 ml of a suspension-CMD at 20 mg of iron per ml was then positioned against a neodymium magnet of remanence 1.3 T for 5 minutes. The supernatant was removed and replaced 6 ml of a CMD solution at 70 mg/ml. The mixture was then sonicated in the SB overnight at room temperature. After sonication, the suspension of coated magnetosome minerals was centrifuged at 13 OOOg for 90 minutes, and the supernatant was removed and replaced by pure water. A neodymium magnet was then positioned against the tube containing the suspension of coated magnetosome minerals, and the supernatant was removed and replaced by pure water.

### Example 2: Internalization of N-CMD in PC3-Luc cells.

Material and Methods: PC3-Luc cells were cultivated in was grown in high-glucose DMEM containing 10% fetal calf serum (FCS), 100 U/mL penicillin and 100 µg/mL at 37°C in a 5% CO₂ atmosphere with 95% relative humidity until confluency. 5.10⁵ cells per mL were harvested. We introduced 500 µl containing 2.5 10⁵ cells in 12 wells (wells 1 to 12) and 500 µl of growth medium in 12 other wells (wells 13 to 24). Then, we left the 24 wells plate in an incubator for 24 hours at 37 °C in 5% CO₂ to let the cells adhere at the bottom of the wells. We then removed the growth medium. We added to the 12 wells containing the cells: i) 500 µl of growth medium (wells 1 to 3), ii) 500 µl of a suspension of N-CMD at 62.5 µg/ml (wells 4 to 6), iii) 500 µl of a suspension of N-CMD at 250 µg/ml (wells 7 to 9), iv), 500 µl of a suspension of N-CMD at 1 mg/ml (wells 10 to 12). We added to the 12 wells containing the growth medium without the cells: i) 500 µl of growth medium (wells 13 to 15), ii) 500 µl of a suspension of N-CMD at 62.5 µg/ml (wells 16 to 18), iii) 500 µl of a suspension of N-CMD at 250 µg/ml (wells 19 to 21), iv), 500 µl of a suspension of N-CMD at 1 mg/ml (wells 22 to 24). The magnetosomes incubated with the cells (wells 1 to 12) or the magnetosomes incubated with the growth medium alone (wells 13 to 24) were then incubated for different times of 5 minutes, 30 minutes, 3 hours, 6 hours, 24 hours, and 96 hours. The growth medium was removed from the different wells. 0.25% of trypsin EDTA was added to detach the cells from the bottom of the wells. For wells 1 to 12, the number of cells was counted using a cell counter for calculating the amount of internalized iron per cell. The content of the different wells was collected and centrifuged for 5 minutes at 1000 rpm and we harvested the growth medium with the magnetosomes originating from wells 13 to 24 or the cell pellets originating from wells 1 to 12. The magnetosomes originating from wells 13 to 24 and cell pellets originating from wells 1 to 12 were mixed with 12 N HCl overnight and then with 2% nitric acid for 48 hours to dissolve the magnetosomes into ionic iron. The amount of iron measured in samples originating from wells 13 to 24 was subtracted from the amount of iron measured in samples originating from wells 1 to 12. The ionic iron concentration was measured using an ICP-AES and we deduced the amount of iron originated from magnetosomes internalized per cell.

### Results and discussion:

For 62 µg/mL of N-CMD incubated with PC3-Luc cells, the quantity of iron coming from magnetosomes that is internalized in cells increases from 6 pg per cell at 5 minutes to 11 pg per cell at 3 hours and then decreases to 5 pg per cell at 96h (Figure 1(a)). At the same time the percentage of iron coming from the magnetosomes, which is internalized in PC3-cells increases from 7% at 5 minutes to 21% at 96 hours (Figure 1(b)).

For 250 µg/mL of N-CMD incubated with PC3-Luc cells, the quantity of iron coming from magnetosomes that is internalized in cells increases from 31 pg per cell at 5 minutes to 56 pg per cell at 3 hours and then decreases to 45 pg per cell at 96h (Figure 1(a)). At the same time the percentage of iron coming from the magnetosomes, which is internalized in PC3-Luc cells increases from 9% at 5 minutes to 24% at 96 hours (Figure 1(b)).

For 1000 µg/mL of N-CMD incubated with PC3-Luc cells, the quantity of iron coming from magnetosomes that is internalized in cells increases from 112 pg per cell at 5 minutes to 272 pg per cell at 3 hours and then decreases to 189 pg per cell at 96h (Figure 1(a)). At the same time the percentage of iron coming from the magnetosomes, which is internalized in PC3-cells increases from 8% at 5 minutes to 22% at 96 hours (Figure 1(b)).

For the three studied concentrations, we observe an increase of the quantity of magnetosomes internalized per cell between 5 minutes and 3 hours of incubation, and then a decrease of this quantity per cell between 3 and 96 hours. The maximum quantity of magnetosomes internalized per cell is observed for an incubation time of 3 hours and for a magnetosome concentration of 1 mg/mL at 272 pg of iron per cell. When we considered the percentage of internalized magnetosomes, estimated as the ratio between the quantity of magnetosomes internalized in cells and the quantity of magnetosomes incubated with cells, we observed that the maximum percentage was reached at 96 hours, because the cell multiplication over time, and the number of cells that can internalize the magnetosomes is larger at this time.

### Example 3: Cytotoxicity of PC3-Luc cells brought into contact with various concentrations of N-MCD (0 mg/mL, 62 µg/mL, 250 µg/mL, 1000 µg/mL) during 3 hours and cooled down to ∼10 °C, and ∼0 °C. Non ice-ball cellular death highlighted in the presence of nanoparticles.

Note: In examples 3 to 5, the cells with/without the nanoparticles and/or the growth medium with/without the nanoparticles are let warming up during the warming step by being exposed to ambient air.

Material and method: 0.2 mL Eppendorf were used for this experiment. A hole was made through the cap of the Eppendorf to insert a thermocouple in the Eppendorf and measure the temperature inside. In each Eppendorf, we introduced 100 µl of cellular suspension containing 2.5 10⁵ PC3-Luc cells. The tubes were centrifuged at 1000 rpm during 5 minutes. After that, we removed the growth medium and we added 100 µl of the different magnetosome suspensions containing 1000, 250, 62.5µg/mL of N-CMD or 100 µl of growth medium. We incubated the cells with magnetosomes or growth medium (0µg/mL) for 3 hours. As a whole, we used 48 Eppendorf (3 Eppendorf per condition) containing: i) 1000 µg/mL of N-CMD maintained at room temperature (tubes 1 to 3), cooled down to 10 °C (tubes 4 to 6), cooled down to 0 °C (tubes 7 to 9), ii) 250 µg/mL of N-CMD maintained at room temperature (tubes 13 to 15), cooled down to 10 °C (tubes 16 to 18), cooled down to 0 °C (tubes 19 to 21), iii), 62.5 µg/mL of N-CMD maintained at room temperature (tubes 25 to 27), cooled down to 10 °C (tubes 28 à 30), cooled down to 0 °C (tubes 31 to 33), iv) 0 µg/mL of N-CMD maintained at room temperature (tubes 37 to 39), cooled down to 10 °C (tubes 40 to 42), cooled down to 0 °C (tubes 43 to 45). Under laminar flow hood, we have exposed the Eppendorf tubes to a URGO product of reference 140111 that cools down the Eppendorf tubes by applying a spray of dimethyl ether on the Eppendorf tubes to decrease the temperature of the tubes from an initial temperature Tᵢ, corresponding to room temperature (∼ 28 °C), to a minimum temperature, Tₘᵢₙ, of ∼ 10 °C and ∼ 0°C. This step occurred during a time tᵢ. We attempted to reach these temperatures. However, in some cases, there were a few degrees difference between the minimum temperature reached during the experiments and the minimum temperature that was targeted. Concerning the minimum temperature reached during the cycle that targeted 0 degree, it was always above 0 degree. After we reached the minimum temperature, we have let the Eppendorf tubes warming up from Tₘᵢₙ up to a final temperature, T_{f}, of equilibrium, which is close to room temperature (∼ 28 °C), during a second step. This second step occurred during a time t_{f}. 48 hours later, we then measured with the MTT assay the cellular viability resulting from such treatment.

### Results and discussion:

We have observed that:
i) The duration of the cooling step does not strongly depend on nanoparticle concentration (between 0 mg/mL and 1 mg/mL of M-CMD) and minimal temperature (0 and 10 degrees). It is 22-47 seconds for the cooling step between RT and 0 degree for NP concentration between 0 mg/mL and 1 mg/mL and 13-32 seconds for the cooling step between RT and 10 degree for NP concentration between 0 mg/mL and 1 mg/mL.
ii) The rate at which the temperature is decreased during the cooling step (cooling rate) does not strongly depend on nanoparticle concentration (between 0 mg/mL and 1 mg/mL of M-CMD) and minimal temperature (0 and 10 degrees). It is 0.6-1.4 °C per second for the cooling step between RT and 0 degree for NP concentration between 0 mg/mL and 1 mg/mL and 0.6-1.7 °C per second for the cooling step between RT and 10 degree for NP concentration between 0 mg/mL and 1 mg/mL;
iii) By contrast to the duration of the cooling step, the duration of the warming step increases with increasing magnetosome concentration from 310 sec. at 0 mg/mL to 461 sec. at 1 mg/mL for the warming step from 0 degree to room temperature and from 256 sec. at 0 mg/mL to 396 sec. at 1 mg/mL for the warming step from 10 degree to room temperature;
iv) By contrast to the cooling rate, the warming rate decreases with increasing magnetosome concentration from 0.08 °C per sec for 0 mg/mL down to 0.06 °C per sec. for 1 mg/mL for the warming step between 0 degree and room temperature and from 0.07 °C/sec for 0 mg/mL down to 0.05 °C per sec. for 1 mg/mL for the rewarming step between 10 degree and room temperature.
v) The duration of the warming step is longer than the duration of the cooling step and the warming rate is shorter than the cooling rate.

At 10 degrees, the percentage of living cells remains similar between 0 mg/mL (87±13%) and 1 mg/mL (77±2%) while at 0 degree, the percentage of living cells decreases from ∼88±6% at 0 µg/mL of N-CMD to ∼75±3% at 1 mg/mL. In this example, we have therefore shown that we could induce cellular death by decreasing the temperature of PC3-Luc cells in the presence of 1 mg/mL of N-MCD to a temperature that is just above 0 °C (degree Celsius).

### Example 4: Cytotoxicity of PC3-Luc cells brought into contact with 1 mg/mL of N-MCD during 3 hours or brought into contact with growth medium without N-CMD during 3 hours and exposed to two different types of cooling treatments with short and long cycles.

For the short cycles, the cells with/without N-CMD are cooled down with URGO to a minimal temperature of 10 °C, 5°C, 0 °C, -5 °C, -10 °C, -20 °C, and -40 °C, and these assemblies are then let warming up from the minimal temperature to room temperature by being exposed to ambient air without using the URGO system.

For the long cycles, the cells with/without N-CMD are cooled down with URGO to a minimal temperature of 10 °C, 5°C, 0 °C, -5 °C, -10 °C, -20 °C, and -40 °C, the temperature of these assemblies is then maintained with URGO at the minimal temperature during 75-295 sec, and these assemblies are then let warming up from the minimal temperature to room temperature by being exposed to ambient air without using the URGO system.

Material and method: 0.2 mL Eppendorf were used for this experiment. A hole was made through the cap of the Eppendorf to insert a thermocouple in the Eppendorf and measure the temperature inside. In each Eppendorf, we introduced 100 µl of cellular suspension containing 2.5 10⁵ PC3-Luc cells. The tubes were centrifuged at 1000 rpm during 5 minutes. After that, we removed the growth medium and we added 100 µl of the different magnetosome suspensions containing 1000 µg/mL of N-CMD or 100 µl of growth medium. We incubated the cells with magnetosomes or growth medium (0µg/mL) for 3 hours. As a whole, we used 72 Eppendorf (3 Eppendorf per condition) containing: i) 1000 µg/mL of N-CMD maintained at room temperature (tubes 1 to 3), 0 µg/mL of N-CMD maintained at room temperature (tubes 4 to 6), 1000 µg/mL of N-CMD cooled down to ∼10 °C with short cycle (tubes 4 to 6), 0 µg/mL of N-CMD cooled down to ∼10 °C with short cycle (tube 7 to 9), 1000 µg/mL of N-CMD cooled down to ∼10 °C with long cycle (tubes 10 to 12), 0 µg/mL of N-CMD cooled down to ∼10 °C with short cycle (tube 13 to 15), 1000 µg/mL of N-CMD cooled down to ∼5 °C with short cycle (tubes 13 to 15), 0 µg/mL of N-CMD cooled down to ∼5 °C with short cycle (tube 16 to 18), 1000 µg/mL of N-CMD cooled down to ∼5 °C with long cycle (tubes 19 to 21), 0 µg/mL of N-CMD cooled down to ∼5 °C with long cycle (tube 22 to 23), 1000 µg/mL of N-CMD cooled down to ∼0 °C with short cycle (tubes 22 to 24), 0 µg/mL of N-CMD cooled down to ∼0 °C with short cycle (tube 22 to 24), 1000 µg/mL of N-CMD cooled down to ∼0 °C with long cycle (tubes 22 to 24), 0 µg/mL of N-CMD cooled down to ∼0 °C with long cycle (tube 22 to 24), 1000 µg/mL of N-CMD cooled down to ∼ -5 °C with short cycle (tubes 25 to 27), 0 µg/mL of N-CMD cooled down to ∼ --5 °C with short cycle (tube 28 to 30), 1000 µg/mL of N-CMD cooled down to ∼ -5 °C with long cycle (tubes 31 to 33), 0 µg/mL of N-CMD cooled down to ∼ -5 °C with short cycle (tube 34 to 36), 1000 µg/mL of N-CMD cooled down to ∼ -10 °C with short cycle (tubes 37 to 39), 0 µg/mL of N-CMD cooled down to ∼ -10 °C with short cycle (tube 40 to 42), 1000 µg/mL of N-CMD cooled down to ∼ -10 °C with long cycle (tubes 43 to 45), 0 µg/mL of N-CMD cooled down to ∼ -10 °C with short cycle (tube 46 to 48), 1000 µg/mL of N-CMD cooled down to ∼ -20 °C with short cycle (tubes 49 to 51), 0 µg/mL of N-CMD cooled down to ∼ -20 °C with short cycle (tube 52 to 54), 1000 µg/mL of N-CMD cooled down to ∼ -20 °C with long cycle (tubes 55 to 57), 0 µg/mL of N-CMD cooled down to ∼ -20 °C with short cycle (tube 58 to 60), 1000 µg/mL of N-CMD cooled down to ∼ -40 °C with short cycle (tubes 61 to 63), 0 µg/mL of N-CMD cooled down to ∼ -40 °C with short cycle (tube 64 to 66), 1000 µg/mL of N-CMD cooled down to ∼ -40 °C with long cycle (tubes 67 to 69), 0 µg/mL of N-CMD cooled down to ∼ -45 °C with short cycle (tube 70 to 72),

For the treatments with the short cycles, we have exposed under laminar flow hood the Eppendorf tubes to a URGO product of reference 140111 that cools down the Eppendorf tubes by applying a spray of dimethyl ether on the Eppendorf tubes to decrease the temperature of the tubes from an initial temperature Tᵢ, corresponding to room temperature (∼ 25 °C), to a minimum temperature, Tₘᵢₙ, of ∼10 °C, ∼5 °C, ∼0°C, ∼ -5 °C, ∼ -10 °C, ∼ -20 °C, ∼ -40°C. However, in some cases, there were a few degrees difference between the temperature Tₘᵢₙ reached during the experiments and the attempted temperature. After we reached the minimum temperature, we have let the Eppendorf tubes warming up from Tₘᵢₙ up to a final temperature, T_{f}, of equilibrium, which is close to room temperature (∼ 25 °C), during a second step. This second step occurred during a time t_{f}. Immediately after the treatment, cells were seeded in 96 well plate at a concentration of 2.10⁵ cells/mL. 48 hours later, we then measured with the MTT assay the cellular viability resulting from such treatment

For the treatments with long cycles, we have exposed under laminar flow hood the Eppendorf tubes to a URGO product of reference 140111 that cools down the Eppendorf tubes by applying a spray of dimethyl ether on the Eppendorf tubes to decrease the temperature of the tubes from an initial temperature Tᵢ, corresponding to room temperature (∼ 25 °C), to a minimum temperature, Tₘᵢₙ, of ∼10 °C, ∼5 °C, ∼0°C, ∼ -5 °C, ∼ -10 °C, ∼ -20 °C and ∼ -40°C. However, in some cases, there were a few degrees difference between the temperature Tₘᵢₙ reached during the experiments and the attempted minimum temperature. After we reached the minimum temperature, we have maintained the temperature of the assemblies of cells and magnetosomes to the minimum temperature during a lapse of time of 87-140 sec. we have then let the Eppendorf tubes warming up from Tₘᵢₙ up to a final temperature, T_{f}, of equilibrium, which is close to room temperature (∼ 28 °C), during a second step. This second step occurred during a time t_{f}. Immediately after the treatment, cells were seeded in 96 well plate at a concentration of 2.10⁵ cells/mL. 48 hours later, we then measured with the MTT assay the cellular viability resulting from such treatment.

### Results and discussion:

We have observed that:
The duration of the warming step (200-650 sec) is longer than the duration of the cooling step (10-50 sec.) in the different tested conditions.

For the short cycles, a more important cellular death in the presence of 1 mg/mL of N-CMD than in the absence of N-CMD is observed at ∼5 °C, ∼0 °C, ∼ -5 °C, ∼ -10 °C, and ∼ -20 °C. The value of A = %LC(1 mg)-%LC(0mg), where %LC(1 mg) and %LC(0mg) are the percentages of living cells in the presence and absence of N-CMD, respectively, is 10% for Tₘᵢₙ = 5 °C, 26% for Tₘᵢₙ = 0 °C, 24% for Tₘᵢₙ = - 5 °C, 19% for Tₘᵢₙ = - 10 °C, and 10% for Tₘᵢₙ = - 20 °C. Interestingly, the value of A was the largest for Tₘᵢₙ = 0°C, indicating that decreasing the value of Tₘᵢₙ much below 0°C does not increase the efficacy of cellular destruction induced by the presence of nanoparticles cooled down to Tₘᵢₙ. This is counter intuitive since cryotherapy for tumor treatment is usually believed to increase in efficacy with decreasing temperature and is usually efficient at temperatures much below 0 °C (typically - 40 °C).

For long cycles, a more important cellular death in the presence of 1 mg/mL of N-CMD than in the absence of N-CMD is observed at 5 °C, 0 °C, and - 5 °C. The value of A = %LC(1 mg) - %LC(0mg), where %LC(1 mg) and %LC(0mg) are the percentages of living cells in the presence and absence of N-CMD, respectively, is 10% for Tₘᵢₙ = 5 °C, 23% for Tₘᵢₙ = 0 °C, 33% for Tₘᵢₙ = - 5 °C. For Tₘᵢₙ = - 10°C, Tₘᵢₙ = -20 °C, and Tₘᵢₙ = -40 °C, all cells were dead and A could not be estimated.

Comparing the values of A between long and short cycles, we have noted a series of interesting and counter intuitive behaviors:
A could be estimated for a narrower range of minimum temperatures in the case of long cycles (- 5°C < Tₘᵢₙ < 5 °C) than in the case of short cycles (- 40 °C < Tₘᵢₙ < 5 °C).

The maximum value of A was larger for short cycles than for long cycles (26% at 0°C for short cycles compared with 20% at -5 °C for long cycles).

The maximum value of A was observed at a higher temperature for short cycles than long cycles (0°C for short cycles compared with - 5 °C for long cycles).

One might have expected that the efficacy of tumor cell destruction would increase by increasing the duration of the step during which the temperature is maintained at the minimum temperature. In fact, the opposite behavior is observed. The strongest efficacy of cellular destruction is observed for the shortest duration of the maintaining step.

The effect of nanoparticles on cellular death can also be evaluated by measuring B = (%LC (0mg/mL)-%LC (1 mg/mL)/%LC (Omg/mL), which is plotted in Fig. 4 as a function of minimum temperatures. B is non-zero for a wider range of minimal temperatures for short cycles (-40 °C < Tₘᵢₙ < 10 °C) than for long cycles (-10°C< Tₘᵢₙ<10°C). This is counter-intuitive since one might guess that when the minimal temperature is maintained for a longer period of time, the efficacy of cellular destruction due to the presence of nanoparticles increases.

In this example, we have therefore shown that the effect of nanoparticles on cellular death was more pronounced when the assemblies of cells and nanoparticles were cooled down to a minimum temperature without being maintained to this minimum temperature for more than 10 seconds than when they were cooled down to this minimum temperature and maintained at this minimum temperature for more than 60 seconds

### Example 5: Cytotoxicity of PC3-Luc cells brought into contact with 1 mg/mL of N-MCD during 3 hours or brought into contact with growth medium without N-CMD during 3 hours and exposed to three different types of cooling treatments consisting in 1 cooling cycle, 3 cooling cycles, and 6 cooling cycles.

Material and method: 0.2 mL Eppendorf were used for this experiment. A hole was made through the cap of the Eppendorf to insert a thermocouple in the Eppendorf and measure the temperature inside. In each Eppendorf, we introduced 100 µl of cellular suspension containing 2.5 10⁵ PC3-Luc cells. The tubes were centrifuged at 1000 rpm during 5 minutes. After that, we removed the growth medium and we added 100 µl of the different magnetosome suspensions containing 1000 µg/mL of N-CMD or 100 µl of growth medium. We incubated the cells with magnetosomes or growth medium (0µg/mL) for 3 hours.

We distinguish the above 0 °C (Tmin>0°C) cooling cycles for which the minimum temperatures are just above 0 °C from the below 0 °C (Tmin<0°C) cooling cycles for which the minimum temperatures are just below 0 °C.

For the just above 0 °C (Tₘᵢₙ>0°C) cycles, we have carried out 1 and 3 cycles in the following manner:
ia) 1 cycle during which the temperature of the cells is decreased from RT to Tₘᵢₙ₁ ∼ 3°C and the cells are then let to warm up from Tₘᵢₙ₁ ∼ 3°C to RT (tube 1 to 3, Figure 5(a));
ib) 1 cycle during which the temperature of the assembly of cells with 1 mg/mL of M-CMD is decreased from RT to Tₘᵢₙ₁ ∼ 1 °C using the URGO system and the assembly is then let to warm up from Tₘᵢₙ₁ ∼ 1 °C to RT (tube 4 to 6, Figure 5(b));
iia) 3 cycles during which the temperature of the cells is decreased from RT to Tₘᵢₙ₁ of 0.2 °C using the URGO system and the cells are then let to warm up from Tₘᵢₙ₁ of 0.2 °C to RT (first cycle), the temperature of the cells is decreased from RT to Tₘᵢₙ₂ of 1 °C with the URGO system and the cells are then let to warm up from Tₘᵢₙ₂ of 1 °C to RT (second cycle), the temperature of the cells is decreased from RT to Tₘᵢₙ₃ of 1 °C using the URGO system and the cells are then let to warm up from Tₘᵢₙ₃ of 1 °C to RT (third cycle) (tube 7 to 9, Figure 5(c));
iib) 3 cycles during which the temperature of the cells with 1 mg/mL of M-CMD is decreased from RT to Tₘᵢₙ₁ of 2 °C with the URGO system and the assembly are then let to warm up from Tₘᵢₙ₁ of 2 °C to RT (first cycle), the temperature of the assembly is decreased from RT to Tₘᵢₙ₂ of 1 °C with the URGO system and the assembly is let to warm up from Tₘᵢₙ₂ of 1 °C to RT (second cycle), the temperature of the assembly is decreased from RT to Tₘᵢₙ₃ of 2 °C with the URGO system and the assembly is then let to warm up from Tₘᵢₙ₃ of 2 °C to RT (third cycle) (tube 10 to 12, Figure 5(d)),

For the just above 0 °C (Tₘᵢₙ>0°C) cycles, we have carried out 1 and 6 cycles in the following manner:
iiia) 1 cycle during which the temperature of the cells is decreased from RT to Tₘᵢₙ₁ ∼ 2°C and is the cells are then let to warm up from Tₘᵢₙ₁ ∼ 2°C to RT (tube 13 to 15, Figure 8(a));
iiib) 1 cycle during which the temperature of the assembly of cells with 1 mg/mL of M-CMD is decreased from RT to Tₘᵢₙ₁ ∼ 2 °C using the URGO system and the assembly is then let to warm up from Tₘᵢₙ₁ ∼ 2 °C to RT (tube 16 to 18, Figure 8(b));
iva) 6 cycles during which the temperature of the cells is decreased from RT to Tₘᵢₙ₁ of ∼ 4 °C using the URGO system and the cells are then let to warm up from Tₘᵢₙ₁ of ∼ 4 °C to RT (first cycle), the temperature of the cells is decreased from RT to Tₘᵢₙ₂ of ∼ 4 °C with the URGO system and the cells are then let to warm up from Tₘᵢₙ₂ of ∼ 4 °C to RT (second cycle), the temperature of the cells is decreased from RT to Tₘᵢₙ₃ of ∼ 4 °C using the URGO system and the cells are then let to warm up from Tₘᵢₙ₃ of ∼ 4 °C to RT (third cycle), the temperature of the cells is decreased from RT to Tₘᵢₙ₄ of ∼ 5 °C using the URGO system and the cells are then let to warm up from Tₘᵢₙ₄ of ∼ 5 °C to RT (fourth cycle), the temperature of the cells is decreased from RT to Tₘᵢₙ₅ of ∼ 4 °C with the URGO system and the cells are then let to warm up from Tₘᵢₙ₅ of ∼ 4 °C to RT (fifth cycle), the temperature of the cells is decreased from RT to Tₘᵢₙ₆ of ∼ 4 °C using the URGO system and the cells are then let to warm up from Tₘᵢₙ₆ of ∼ 4 °C to RT (sixth cycle) (tube 17 to 19, Figure 8(c)),
ivb) 6 cycles during which the temperature of the cells brought into contact with 1 mg/mL of N-CMD (assembly) is decreased from RT to Tₘᵢₙ₁ of ∼ 3 °C using the URGO system and the assembly is then let to warm up from Tₘᵢₙ₁ of ∼ 3 °C to RT (first cycle), the temperature of the assembly is decreased from RT of ∼ 3 °C to Tₘᵢₙ₂ of ∼3 °C with the URGO system and the assembly are then let to warm up from Tₘᵢₙ₂ of ∼ 3 °C to RT (second cycle), the temperature of the assembly is decreased from RT to Tₘᵢₙ₃ of 4 ∼°C using the URGO system and the assembly is then let to warm up from Tₘᵢₙ₃ of ∼ 4 °C to RT (third cycle), the temperature of the assembly is decreased from RT to Tₘᵢₙ₄ of ∼3 °C using the URGO system and the assembly is then let to warm up from Tₘᵢₙ₄ of ∼ 3 °C to RT (fourth cycle), the temperature of the assembly is decreased from RT to Tₘᵢₙ₅ of ∼3 °C with the URGO system and the assembly is then let to warm up from Tₘᵢₙ₅ of ∼ 3 °C to RT (fifth cycle), the temperature of the assembly is decreased from RT to Tₘᵢₙ₆ of ∼3 °C using the URGO system and the assembly is then let to warm up from Tₘᵢₙ₆ of ∼ 3 °C to RT (sixth cycle) (tube 20 to 22, Figure 8(d)).

For the just below 0 °C (Tₘᵢₙ<0°C) cycles, we have carried out 1 and 3 cycles in the following manner:
va) 1 cycle during which the temperature of the cells is decreased from RT to Tₘᵢₙ₁ of -2 °C and the cells are then let to warm up from Tₘᵢₙ₁ of -2 °C to RT (tube 17 to 19, Figure 6(a));
vb) 1 cycle during which the temperature of the assembly of cells with 1 mg/mL of M-CMD is decreased from RT to Tₘᵢₙ₁ of -2 °C using the URGO system and the assembly is then let to warm up from Tₘᵢₙ₁ of -2 °C to RT (tube 23 to 25, Figure 6(b));
via) 3 cycles during which the temperature of the cells is decreased from RT to Tₘᵢₙ₁ of -1 °C using the URGO system and the cells are then let to warm up from Tₘᵢₙ₁ of - 1 °C to RT (first cycle), the temperature of the cells is decreased from RT to Tₘᵢₙ₂ of -2 °C with the URGO system and the cells are then let to warm up from Tₘᵢₙ₂ of - 2 °C to RT (second cycle), the temperature of the cells is decreased from RT to Tₘᵢₙ₃ of -1 °C using the URGO system and the cells are then let to warm up from Tₘᵢₙ₃ of - 1 °C to RT (third cycle) (tube 26 to 28, Figure 6(c));
vib) 3 cycles during which the temperature of the cells with 1 mg/mL of M-CMD is decreased from RT to Tₘᵢₙ₁ of -1 °C with the URGO system and the assembly are then let to warm up from Tₘᵢₙ₁ of -1 °C to RT (first cycle), the temperature of the assembly is decreased from RT to Tₘᵢₙ₂ of -2 °C with the URGO system and the assembly is then let to warm up from Tₘᵢₙ₂ of -2 °C to RT (second cycle), the temperature of the assembly is decreased from RT to Tₘᵢₙ₃ of -1 °C with the URGO system and the assembly is then let to warm up from Tₘᵢₙ₃ of -1 °C to RT (third cycle) (tube 29 to 31, Figure 6(d)).

For the just below 0 °C (Tₘᵢₙ<0°C) cycles, we have carried out 1 and 6 cycles in the following manner:
viia) 1 cycle during which the temperature of the cells is decreased from RT to Tₘᵢₙ₁ of -2 °C and the cells are then let to warm up from Tₘᵢₙ₁ of -2 °C to RT (tube 32 to 34, Figure 9(a));
viib) 1 cycle during which the temperature of the assembly of cells with 1 mg/mL of M-CMD is decreased from RT to Tₘᵢₙ₁ of -2 °C using the URGO system and the assembly is then let to warm up from Tₘᵢₙ₁ of -2 °C to RT (tube 35 to 36, Figure 9(b));
viiia) 6 cycles during which the temperature of the cells is decreased from RT to Tₘᵢₙ₁ of -2 °C using the URGO system and the cells are then let to warm up from Tₘᵢₙ₁ of -2 °C to RT (first cycle), the temperature of the cells is decreased from RT to Tₘᵢₙ₂ of -1 °C with the URGO system and the cells are then let to warm up from Tₘᵢₙ₂ of -1 °C to RT (second cycle), the temperature of the cells is decreased from RT to Tₘᵢₙ₃ of -3 °C using the URGO system and the cells are then let to warm up from Tₘᵢₙ₃ of -3 °C to RT (third cycle), the temperature of the cells is decreased from RT to Tₘᵢₙ₄ of -1 °C using the URGO system and the cells are then let to warm up from Tₘᵢₙ₄ of -1 °C to RT (fourth cycle), the temperature of the cells is decreased from RT to Tₘᵢₙ₅ of 0 °C with the URGO system and the cells are then let to warm up from Tₘᵢₙ₅ of 0 °C to RT (fifth cycle), the temperature of the cells is decreased from RT to Tₘᵢₙ₆ of -1 °C using the URGO system and the cells are then let to warm up from Tₘᵢₙ₆ of -1 °C to RT (sixth cycle) (tube 37 to 39, Figure 9(c)),
viiib) 6 cycles during which the temperature of the cells brought into contact with 1 mg/mL of N-CMD (assembly) is decreased from RT to Tₘᵢₙ₁ of -3 °C using the URGO system and the assembly is then let to warm up from Tₘᵢₙ₁ of -3 °C to RT (first cycle), the temperature of the assembly is decreased from RT to Tₘᵢₙ₂ of -3 °C with the URGO system and the assembly are then let to warm up from Tₘᵢₙ₂ of -3 °C to RT (second cycle), the temperature of the assembly is decreased from RT to Tₘᵢₙ₃ of -3 °C using the URGO system and the assembly is then let to warm up from Tₘᵢₙ₃ of -3 °C to RT (third cycle), the temperature of the assembly is decreased from RT to Tₘᵢₙ₄ of -2 °C using the URGO system and the assembly is then let to warm up from Tₘᵢₙ₄ of -2 °C to RT (fourth cycle), the temperature of the assembly is decreased from RT to Tₘᵢₙ₅ of -1 °C with the URGO system and the assembly is then let to warm up from Tₘᵢₙ₅ of -1 °C to RT (fifth cycle), the temperature of the assembly is decreased from RT to Tₘᵢₙ₆ of -5 °C using the URGO system and the assembly is then let to warm up from Tₘᵢₙ₆ of -5 °C to RT (sixth cycle) (tube 40 to 42, Figure 9(d)).

As a whole, we used 42 Eppendorf (3 Eppendorf per condition) containing the cells with 1000 µg/mL of N-CMD or the cells without N-CMD.

### Results and discussion:

The duration of the cooling step does not strongly depend on minimum temperature and the presence (or not) of N-CMD. In the different conditions, it is 20-65 sec.

By contrast, the duration of the warming step is longer in the presence of 1 mg/mL of N-CMD (540 sec in average for Tmin>0°C) than in the absence of N-CMD (470 sec in average for Tₘᵢₙ>0 °C).

The cellular destruction is more pronounced for 6 than 1 cycle(s). This is true both for Tₘᵢₙ<0°C and for Tₘᵢₙ>0°C (Figures 7 and 10). It indicates that cellular death can be increased by increasing the number of cycles.

For Tₘᵢₙ>0°C, while for 3 cycles the percentage of living cells is similar in the presence and absence of nanoparticle (Figure 7), for 6 cycles the percentage of living cells is lower in the presence than absence of nanoparticles (Figure 10). It indicates that for Tₘᵢₙ>0°C, cellular toxicity can be induced by the presence of nanoparticles when the number of cycles is increased, preferentially above 3.

The value of B = (%LC (0mg/mL)-%LC (1mg/mL)/%LC (Omg/mL) is larger for minimum temperatures just below 0 °C (Tₘᵢₙ<0°C) than for minimum temperatures just above 0 °C (Tₘᵢₙ>0°C) for the three types of cycles. For 1 cycle, B increases from 3% for Tₘᵢₙ>0°C to 30% for Tₘᵢₙ<0°C. For three cycles, B increases from 10% for Tₘᵢₙ>0°C to 53% for Tₘᵢₙ<0°C. For 6 cycles, B increases from 24% for Tₘᵢₙ>0°C to 65% for Tₘᵢₙ<0° (Figure 11).

The value of B = (%LC (0mg/mL)-%LC (1mg/mL)/%LC (Omg/mL) increases with increasing number of cycles for the two minimum temperatures (Tₘᵢₙ>0°C and Tₘᵢₙ<0°C). B increases from 2% for one cycle to 15% for 6 cycles (for Tₘᵢₙ>0°C), and from 31% for one cycle to 65% for 6 cycles (for Tₘᵢₙ<0°C), Figure 11.

We examine the variations of B between 1 and 3 cycles and between 1 and 6 cycles by estimating C = B_{3cycle}/B_{1cycle} and D = B_{6cycle}/B_{1cycle}, where B_{1cycle}, B_{3cycle}, and B_{6cycle} are the values of B estimated for 1 cycle, 3 cycles, and 6 cycles, respectively. The value of C increases from 1.75 for Tₘᵢₙ<0°C to 3.06 for Tₘᵢₙ>0°C while the value of D increases from 2.1 at Tₘᵢₙ<0°C to 8.1 for Tₘᵢₙ>0°C (Figure 12).

This is counter-intuitive since one may have expected that carrying out cooling cycles below 0°C would lead to a stronger increase in cellular destruction than carrying out cooling cycles above 0 °C. The opposite behavior was observed. Carrying out cycles with Tₘᵢₙ below 0 °C increases less cellular death than carrying out cooling cycles with Tₘᵢₙ above 0 °C..

**Table 1:** PC3-Luc cells alone (0 mg/mL) or PC3-Luc cells brought into contact with 1 mg/mL of N-CMD during 3 hours (1 mg/mL), which are firstly cooled down from Tᵢ to Tₘᵢₙ (> 0°C) during a time t_{d} at a rate of temperature decrease, which are secondly let warming up from Tₘᵢₙ to T_{f} during a time tₘ at a rate of temperature increase.

### Temperature data of PC3-Luc treated by Cryotherapy-1 cycle (>0°C)

**Table 1**

| | | |
|---|---|---|
| **Concentration of Magnetosomes** | 0 mg/mL | 1 mg/mL |
| **Targeted temperature (°C)** | > 0°C | |
| **Initial temperature, Tᵢ (°C) (cooling step)** | 28.9 | 28 |
| **Duration of temperature decrease, t_{d}(s) (cooling step)** | 31 | 26.5 |
| **Minimal temperature, Tₘᵢₙ (°C) (cooling step)** | 1.99 | 1.75 |
| **Rate** of **temperature decrease (°C/sec) (cooling step)** | 0.87 | 0.99 |
| **Duration of temperature increase tₘ(s) (warming step)** | 464 | 617.5 |
| **Final temperature, T_{f} (°C) (warming step)** | 25.29 | 24 |
| **Rate of temperature increase (°C/sec) (warming step)** | 0.05 | 0.036 |
| **Total duration of treatment, Tₜ (sec)** | 495 | 644 |

**Table 2:** PC3-Luc cells alone (0 mg/mL) or PC3-Luc cells brought into contact with 1 mg/mL of N-CMD during 3 hours (1 mg/mL), which are firstly cooled down from Tᵢ to Tₘᵢₙ (< 0°C) during a time t_{d} at a rate of temperature decrease, which are secondly let warming up from Tₘᵢₙ to T_{f} during a time tₘ at a rate of temperature increase.

### Temperature data of PC3-Luc treated by Cryotherapy-1 cycle (< 0°C)

**Table 2**

| | | |
|---|---|---|
| **Concentration of magnetosomes (mg/mL)** | 0 mg/mL | 1 mg/mL |
| **Targeted temperature (°C)** | < 0°C | |
| **Initial temperature, Tᵢ (°C) (cooling step)** | 27.6 | 29.3 |
| **Duration of temperature decrease, t_{d}(s) (cooling step)** | 41.5 | 34 |
| **Minimal temperature, Tₘᵢₙ (°C) (cooling step)** | -2.33 | -2.33 |
| **Rate of temperature decrease (°C/sec) (cooling step)** | 0.72 | 0.93 |
| **Duration of temperature increase tm(s) (warming step)** | 706 | 829.5 |
| **Final temperature, T_{f} (°C) (warming step)** | 25.3 | 24 |
| **Rate of temperature increase (°C/sec) (warming step)** | 0.039 | 0.032 |
| **Total duration of treatment, Tₜ (sec)** | 747.5 | 863.5 |

**Table 3:** PC3-Luc cells alone (0 mg/mL) or PC3-Luc cells brought into contact with 1 mg/mL of N-CMD during 3 hours (1 mg/mL), which are:
- In a first cycle, firstly cooled down from T, to Tₘᵢₙ (> 0°C) during a time t_{d} at a rate of temperature decrease, which are secondly let warming up from Tₘᵢₙ to T_{f} during a time tₘ at a rate of temperature increase,
- In a second cycle, firstly cooled down from T, to Tₘᵢₙ (> 0°C) during a time t_{d} at a rate of temperature decrease, which are secondly let warming up from Tₘᵢₙ to T_{f} during a time tₘ at a rate of temperature increase,
- In a third cycle, firstly cooled down from T, to Tₘᵢₙ (> 0°C) during a time t_{d} at a rate of temperature decrease, which are secondly let warming up from Tₘᵢₙ to T_{f} during a time tₘ at a rate of temperature increase.

### Temperature data of PC3-Luc treated by Cryotherapy-3 cycles (> 0°C)

**Table 3**

| **Concentration of magnetosomes** | **0 mg/mL** | | | | **1 mg/mL** | | |
|---|---|---|---|---|---|---|---|
| **Freezing-thawing cycle** | **1st cycle** | **2nd cycle** | **3rd cycle** | | **1st cycle** | **2nd cycle** | **3rd cycle** |
| **Targeted temperature (°C)** | > 0°C | | | | > 0°C | | |
| **Initial temperature, Tᵢ (°C) (cooling step)** | 29 | 24 | 24 | | 27 | 29.5 | 20 |
| **Duration of temperature decrease, t_{d}(s) (cooling step)** | 26.5 | 20 | 21 | | 38 | 30 | 23 |
| **Minimal temperature, Tₘᵢₙ (°C) (cooling step)** | 0.19 | 1.5 | 1 | | 1.7 | 1.4 | 1.5 |
| **Rate of temperature decrease (°C/sec) (cooling step)** | 1.1 | 1.1 | 1.1 | | 0.7 | 0.9 | 0.8 |
| **Average rate of temperature decrease (°C/sec)** | 1.1 | | | | 0.8 | | |
| **Duration of temperature increase tₘ(s)** | 329 | 357 | 402.5 | | 567.5 | 688.5 | 438 |
| **Temp finale, T_{f} (°C)** | 24 | 24 | 24 | | 24 | 24 | 24 |
| **Rate of temperature increase (°C/sec)** | 0.07 | 0.06 | 0.06 | | 0.04 | 0.03 | 0.05 |
| **Average rate of temperature increase (°C/sec)** | 0.064 | | | | 0.043 | | |

**Table 4:** PC3-Luc cells alone (0 mg/mL) or PC3-Luc cells brought into contact with 1 mg/mL of N-CMD during 3 hours (1 mg/mL), which are:
- In a first cycle, firstly cooled down from T, to Tₘᵢₙ (< 0°C) during a time t_{d} at a rate of temperature decrease, which are secondly let warming up from Tₘᵢₙ to T_{f} during a time tₘ at a rate of temperature increase,
- In a second cycle, firstly cooled down from T, to Tₘᵢₙ (< 0°C) during a time t_{d} at a rate of temperature decrease, which are secondly let warming up from Tₘᵢₙ to T_{f} during a time tₘ at a rate of temperature increase,
- In a third cycle, firstly cooled down from T, to Tₘᵢₙ (< 0°C) during a time t_{d} at a rate of temperature decrease, which are secondly let warming up from Tₘᵢₙ to T_{f} during a time tₘ at a rate of temperature increase.

### Temperature data of PC3-Luc treated by Cryotherapy-3 cycles (< 0°C)

**Table 4**

| **Concentration of maqnetosomes** | **0 mg/mL** | | | | **1 mg/mL** | | |
|---|---|---|---|---|---|---|---|
| **Freezing-thawing cycle** | **1st cycle** | **2nd cycle** | **3rd cycle** | | **1st cycle** | **2nd cycle** | **3rd cycle** |
| **Targeted temperature (°C)** | < 0°C | | | | < 0°C | | |
| **Initial temperature, Tᵢ (°C) (cooling step)** | 27.02 | 25.2 | 25.53 | | 29.02 | 25.97 | 25.88 |
| **Duration of temperature decrease, t_{d}(s) (cooling step)** | 21.5 | 19.5 | 19.5 | | 32.25 | 28 | 28 |
| **Minimal temperature, Tmin (°C) (cooling step)** | -1.24 | -2.40 | -1.21 | | -1.66 | -2.41 | -1.24 |
| **Rate of temperature decrease (°C/sec) (cooling step)** | 1.31 | 1.42 | 1.37 | | 0.95 | 1.01 | 0.97 |
| **Average rate of temperature decrease (°C/sec) (cooling step)** | 1.37 | | | | 0.98 | | |
| **Duration of temperature increase tₘ(s) (warming step)** | 469.5 | 457.5 | 426 | | 589 | 537.5 | 445 |
| **Temp finale, T_{f}(°C) (warming step)** | 25.2 | 25.6 | 24 | | 25.97 | 25.88 | 24.01 |
| **Rate of temperature increase (°C/sec)** | 0.06 | 0.06 | 0.06 | | 0.05 | 0.05 | 0.06 |
| **Average rate of temperature increase (°C/sec)** | 0.059 | | | | 0.052 | | |

**Table 5:** PC3-Luc cells alone (0 mg/mL) or PC3-Luc cells brought into contact with 1 mg/mL of N-CMD during 3 hours (1 mg/mL), which are:
- In a first cycle, firstly cooled down from T, to Tₘᵢₙ (> 0°C) during a time t_{d} at a rate of temperature decrease, which are secondly let warming up from Tₘᵢₙ to T_{f} during a time tₘ at a rate of temperature increase,
- In a second cycle, firstly cooled down from T, to Tₘᵢₙ (> 0°C) during a time t_{d} at a rate of temperature decrease, which are secondly let warming up from Tₘᵢₙ to T_{f} during a time tₘ at a rate of temperature increase,
- In a third cycle, firstly cooled down from T, to Tₘᵢₙ (> 0°C) during a time t_{d} at a rate of temperature decrease, which are secondly let warming up from Tₘᵢₙ to T_{f} during a time tₘ at a rate of temperature increase.
- In a fourth cycle, firstly cooled down from T, to Tₘᵢₙ (> 0°C) during a time t_{d} at a rate of temperature decrease, which are secondly let warming up from Tₘᵢₙ to T_{f} during a time tₘ at a rate of temperature increase,
- In a fifth cycle, firstly cooled down from T, to Tₘᵢₙ (> 0°C) during a time t_{d} at a rate of temperature decrease, which are secondly let warming up from Tₘᵢₙ to T_{f} during a time tₘ at a rate of temperature increase,
- In a sixth cycle, firstly cooled down from T, to Tₘᵢₙ (> 0°C) during a time t_{d} at a rate of temperature decrease, which are secondly let warming up from Tₘᵢₙ to T_{f} during a time tₘ at a rate of temperature increase.

### Temperature data of PC3-Luc treated by Cryotherapy-6 cycles (> 0°C)

**Table 5**

| **Concentration of magnetosomes** | **0 mg/mL** | | | | | | | **1 mg/mL** | | | | | |
|---|---|---|---|---|---|---|---|---|---|---|---|---|---|
| **Freezing-thawing cycle** | **1st cycle** | **2nd cycle** | **3rd cycle** | **4th cycle** | **5th cycle** | **6th cycle** | | **1st cycle** | **2nd cycle** | **3rd cycle** | **4th cycle** | **5th cycle** | **6th cycle** |
| **Targeted temperature (°C)** | >0°C | | | | | | | >0°C | | | | | |
| **Initial temperature, Tᵢ (°C) (cooling step)** | 26.1 | 24.4 | 24.2 | 24.3 | 24.3 | 24.3 | | 26.4 | 25.6 | 24.5 | 23.9 | 24 | 24 |
| **Duration of temperature decrease, t_{d}(s) (cooling step)** | 38 | 76 | 22.5 | 19 | 55 | 46 | | 45 | 24 | 32.5 | 58.5 | 32 | 39 |
| **Minimal temperature, Tₘᵢₙ(°C) (coolinq step)** | 4.2 | 4 | 5.2 | 4.4 | 4.2 | 4 | | 2.9 | 3.4 | 4.3 | 3.2 | 3.4 | 3.3 |
| **Rate of temperature decrease (°C/sec) (cooling step)** | 0.58 | 0.27 | 0.85 | 1.05 | 0.37 | 0.44 | | 0.52 | 0.93 | 0.62 | 0.35 | 0.64 | 0.53 |
| **Average rate of temperature decrease (°C/sec) (cooling step)** | 0.59 | | | | | | | 0.6 | | | | | |
| **Duration of temperature increase tₘ(s) (warming step)** | 352.5 | 358 | 338 | 298 | 251 | 269 | | 432 | 370 | 312.5 | 309 | 354 | 340.5 |
| **Final temperature, T_{f}(°C) (warming step)** | 24.4 | 24.2 | 24.3 | 24.3 | 24.35 | 24 | | 25.6 | 24.5 | 23.9 | 24 | 24.1 | 24 |
| **Rate of temperature increase (°C/sec) (warming step)** | 0.06 | 0.06 | 0.06 | 0.07 | 0.08 | 0.07 | | 0.05 | 0.06 | 0.06 | 0.07 | 0.06 | 0.06 |
| **Average rate of temperature increase (°C/sec) (warming step)** | 0.065 | | | | | | | 0.06 | | | | | |

**Table 6:** PC3-Luc cells alone (0 mg/mL) or PC3-Luc cells brought into contact with 1 mg/mL of N-CMD during 3 hours (1 mg/mL), which are:
- In a first cycle, firstly cooled down from Tᵢ to Tₘᵢₙ (< 0°C) during a time t_{d} at a rate of temperature decrease, which are secondly let warming up from Tₘᵢₙ to T_{f} during a time tₘ at a rate of temperature increase,
- In a second cycle, firstly cooled down from T, to Tₘᵢₙ (< 0°C) during a time t_{d} at a rate of temperature decrease, which are secondly let warming up from Tₘᵢₙ to T_{f} during a time tₘ at a rate of temperature increase,
- In a third cycle, firstly cooled down from Tᵢ to Tₘᵢₙ (< 0°C) during a time t_{d} at a rate of temperature decrease, which are secondly let warming up from Tₘᵢₙ to T_{f} during a time tₘ at a rate of temperature increase.
- In a fourth cycle, firstly cooled down from Tᵢ to Tₘᵢₙ (< 0°C) during a time t_{d} at a rate of temperature decrease, which are secondly let warming up from Tₘᵢₙ to T_{f} during a time tₘ at a rate of temperature increase,
- In a fifth cycle, firstly cooled down from Tᵢ to Tₘᵢₙ (< 0°C) during a time t_{d} at a rate of temperature decrease, which are secondly let warming up from Tₘᵢₙ to T_{f} during a time tₘ at a rate of temperature increase,
- In a sixth cycle, firstly cooled down from Tᵢ to Tₘᵢₙ (< 0°C) during a time t_{d} at a rate of temperature decrease, which are secondly let warming up from Tₘᵢₙ to T_{f} during a time tₘ at a rate of temperature increase.

### Temperature data of PC3-Luc treated by Cryotherapy-6 cycles (< 0°C)

**Table 6**

| **Concentration of magnetosomes** | 0 mg/mL | | | | | | | 1 mg/mL | | | | | |
|---|---|---|---|---|---|---|---|---|---|---|---|---|---|
| **Freezing thawing cycle** | 1st cycle | 2nd cycle | 3rd cycle | 4th cycle | 5th cycle | 6th cycle | | 1st cycle | 2nd cycle | 3rd cycle | 4th cycle | 5th cycle | 6th cycle |
| **Temperature (°C)** | <0°C | | | | | | | < 0°C | | | | | |
| **Initial temperature, Tᵢ(°C) (cooling step)** | 27.51 | 24.9 | 25 | 24.76 | 24.4 | 24.86 | | 27.2 | 24.5 | 24.7 | 24 | 23.8 | 24.1 |
| **Duration of temperature decrease, t_{d}(s) (cooling step)** | 23.5 | 27 | 27 | 21 | 35 | 42 | | 47 | 47 | 43 | 37 | 47 | 33 |
| **Minimal temperature, Tₘᵢₙ(°C) (cooling step)** | -2.58 | -1.06 | -3.75 | -1.98 | -0.67 | -1.48 | | -3.84 | -3.12 | -3.45 | -2.06 | -1.62 | -5.5 |
| **Rate of temperature decrease (°C/sec) (cooling step)** | 1.28 | 0.96 | 1.07 | 1.27 | 0.72 | 0.63 | | 0.66 | 0.59 | 0.65 | 0.7 | 0.54 | 0.9 |
| **Average rate of temperature decrease (°C/sec) (cooling step)** | 0.99 | | | | | | | 0.67 | | | | | |
| **Duration of temperature increase tₘ(s) (warming step)** | 640.5 | 844 | 761 | 837 | 603 | 349 | | 939 | 959 | 1027 | 868 | 631 | 860 |
| **Final temperature, T_{f} (°C) (warming step)** | 24.95 | 24.8 | 24.28 | 24.37 | 24.9 | 24 | | 24.5 | 24.7 | 24.01 | 23.8 | 24.1 | 23.9 |
| **Rate of temperature increase (°C/sec) (warming step)** | 0.04 | 0.03 | 0.04 | 0.03 | 0.04 | 0.07 | | 0.03 | 0.03 | 0.03 | 0.03 | 0.04 | 0.03 |
| **Average** rate of **temperature increase (°C/sec) (warming step)** | 0.043 | | | | | | | 0.032 | | | | | |

## Claims

1. Nanoparticle for use in a method of treating a body part of an individual by cryotherapy comprising the following steps:
a) administering nanoparticles to a body part of an individual;
b) cooling the body part comprising the nanoparticles by decreasing the temperature of body part from an initial temperature to a cooling temperature of said body part, which is lower than the initial temperature;
c) optionally not maintaining the body part comprising the nanoparticles at the cooling temperature for a duration of time of more than 100 seconds; and
d) warming the body part comprising the nanoparticles by increasing the temperature of the body part from the cooling temperature of said body part to a final temperature of said body part that is above the cooling temperature, **characterized in that** said cooling temperature of the body part is:
- above the freezing temperature and/or above 5, 0 or -5°C, preferably above -5 or 0°C, more preferably above 0°C;
and/or
- above a threshold temperature, where the threshold temperature has at least one property selected from the group consisting of:
i) above the threshold temperature, the body part comprises a quantity of ice-balls smaller than the quantity of nanoparticles;
ii) above the threshold temperature, the volume occupied by ice-balls in the body part is smaller than the volume occupied by the nanoparticles in the body part;
iii) below the threshold temperature, the body part comprises a quantity of ice-balls larger than the quantity of nanoparticles;
iv) below the threshold temperature, the volume occupied by ice-ball in the body part is larger than the volume occupied by the nanoparticles in the body part; and
v) below the threshold temperature, the size or diameter of at least one ice-ball is larger than the size or diameter of at least one nanoparticle.

2. Nanoparticle for use according to claim 1, wherein the succession of steps of at least the cooling step and the warming step, preferably the cooling step, the maintaining step and the warming step is repeated more than once, preferably more than 3 times.

3. Nanoparticle for use according to any of the preceding claims, wherein the initial temperature and/or the final temperature is/are physiological temperature(s).

4. Nanoparticle for use according to any of the preceding claims, wherein the cooling temperature has at least one characteristic selected from the group consisting of:
i) a difference ΔT₁ between the initial and the cooling temperature lower than 57 °C, and/or
ii) a difference ΔT₂ between the final and the cooling temperature lower than 57 °C, and preferably wherein the difference(s) ΔT₁ and/or ΔT₂ decrease(s) when the nanoparticle concentration increases in the body part.

5. Nanoparticle for use according to any of the preceding claims, wherein:
in step d) the warming time to reach the final temperature from the cooling temperature is increased with increasing nanoparticle concentration in the body part, and/or
in step b) the cooling time to reach the cooling temperature from the initial temperature is not predominantly dependent on nanoparticle concentration in the body part.

6. Nanoparticle for use according to any of the preceding claims, wherein in step b) the temperature decreases from the initial temperature to the cooling temperature in the cooling step according to at least one of:
i) a cooling rate in the range from 10⁻⁶ °C/sec to 10⁶ °C/sec, preferably 10⁻³ °C/sec to 10³ °C/sec,
ii) a cooling rate with the nanoparticles that differs by less than 10 °C/sec from the cooling rate without the nanoparticles,
iii) a cooling rate that does vary with varying nanoparticle concentration or vary by a factor in the range of less than 10⁻⁶ °C/sec to 10⁶ °C/sec, preferably less than 10⁻³ °C/sec to 10³ °C/sec, when the nanoparticle concentration increases, preferably either by a factor of at least 1.1 or from a concentration lower than 100 µg of nanoparticles per cm³ of body part to a concentration larger than 100 µg of nanoparticles per cm³ of body part,
iv) a cooling time of between 10⁻⁶ and 10⁶ seconds, preferably of between 10⁻³ and 10³ seconds,
v) a cooling rate that is smaller than the rate of increasing the temperature in the warming step d) by a factor in the range from 10⁻¹⁰ to 10⁵, preferably 10⁻⁵ to 10³, and
vi) a cooling time shorter than the warming time in the warming step d) by a factor in the range of 10⁻⁶ to 10⁶, preferably 10⁻³ to 10³.

7. Nanoparticle for use according to any of the preceding claims, wherein the maintaining step is carried out according to at least one of:
i) a maintaining time that is shorter than the cooling time and/or warming time, preferably by a factor of at least 1.5, more preferably by a factor of at least 10, and
ii) fluctuation of the temperature during the maintaining step c) that is smaller, preferably by a factor of at least 1.5, than ΔT₁ and/or ΔT₂ according to claim **3.**

8. Nanoparticle for use according to any of the preceding claims, wherein in step d) the temperature increases from the cooling temperature to the final temperature according to at least one of:
i) a warming rate in the range of 10⁻⁶ °C/sec to 10⁶ °C/sec, preferably in the range of 10⁻³ °C/sec to 10³ °C/sec,
ii) a warming rate with the nanoparticle(s) that differs by a factor of more than 1.1 from the warming rate without the nanoparticles,
iii) a warming rate that increases with increasing nanoparticle concentration by a factor in the range of 10⁻⁶ °C/sec to 10⁶ °C/sec, preferably 10⁻³ °C/sec to 103 °C/sec,
iv) a warming rate that is smaller than the cooling rate by a factor in the range from 10⁻¹⁰ to 10⁵, preferably 10⁻⁵ to 10³,
v) a warming time of between 10⁻⁶ and 10⁶ seconds, preferably of between 10⁻³ and 10³ seconds,
and
vi) a warming time longer than the cooling time by a factor in the range of 10⁻⁶ to 10⁶, preferably a factor in the range 10⁻³ to 10³.

9. Nanoparticle for use according to any of the preceding claims, wherein the temperature is controlled during the cooling step b) and/or the maintaining step c), whereas the temperature is not controlled during the warming step d), preferably wherein the temperature control is carried out using an equipment or a substance, preferably a cryoprobe.

10. Nanoparticle for use according to any of the preceding claims, wherein at least one of the cooling step, the maintaining step, and the warming step is carried out in the presence of:
i) a percentage of nanoparticles internalized in cells of the body part is in a range from 10⁻³% to 90%, and/or
ii) a concentration of nanoparticles in the body part is in the range from 10⁻⁹ to 10⁹ mg of nanoparticles per cm³ of body part or from 10⁻⁵ to 10⁵ mg of nanoparticles per cell.

11. Nanoparticle for use according to any of the preceding claims, wherein at least one of the cooling step, the maintaining step, and the warming step is carried out when nanoparticles occupy a portion of the body part, which is between 10⁻⁵% and 90% by mass or volume of the body part as a whole.

12. Nanoparticle for use according to any of the preceding claims, wherein the individual in need thereof is suffering from an infectious disease, and the body part comprises cells affected by the infectious disease.

13. Nanoparticles for use according to any of the preceding claims, wherein the nanoparticles have a heating capacity with at least one property selected from the group consisting of:
i) the heating capacity is comprised between 10⁻¹⁰ and 10¹⁰ °C per second per mg of nanoparticle,
ii) the heating capacity is the difference between the heating rate of the body part comprising the nanoparticles and the heating rate of the body part without the nanoparticles,
iii) the heating capacity is measured during the heating step,
iv) the heating capacity can be increased by applying an external source of radiation such as an acoustic wave, laser, or magnetic field on the body part or nanoparticle, and
v) when the heating capacity is increased, the nanoparticles more efficiently destroy cells.

14. Nanoparticles for use according to any of the preceding claims, wherein the nanoparticles are magnetosomes.

15. Method comprising the following steps:
a) administering nanoparticles to a medium;
b) cooling the medium comprising the nanoparticles by decreasing the temperature of the medium from an initial temperature of said medium, to a cooling temperature of the medium, which is lower than the initial temperature;
c) optionally not maintaining the medium comprising the nanoparticles at the cooling temperature for a duration of time of more than 100 seconds; and
d) warming the medium comprising the nanoparticles by increasing the temperature of the medium from the cooling temperature to a final temperature above the cooling temperature,
**characterized in that** said cooling temperature of the medium is:
- above the freezing temperature or above 5, 0 or -5°C, preferably above -5 or 0°C, more preferably above 0°C;
and/or
- above a threshold temperature, where the threshold temperature has at least one property selected from the group consisting of:
i) above the threshold temperature, the body part comprises a quantity of ice-balls smaller than the quantity of nanoparticles;
ii) above the threshold temperature, the volume occupied by ice-balls in the medium is smaller than the volume occupied by the nanoparticles in the medium;
iii) below the threshold temperature, the medium comprises a quantity of ice-balls larger than the quantity of nanoparticles;
iv) below the threshold temperature, the volume occupied by ice-ball in the medium is larger than the volume occupied by the nanoparticles in the medium;
and
v) below the threshold temperature, the size or diameter of at least one ice-ball is larger than the size or diameter of at least one nanoparticle.
